(19) Europäisches Patentamt
European Patent Office
Office européen des brevets

(11) EP 4 183 780 A1

(12) EUROPEAN PATENT APPLICATION
published in accordance with Art. 153(4) EPC

(43) Date of publication:
**24.05.2023 Bulletin 2023/21**

(21) Application number: **21841293.0**

(22) Date of filing: **19.07.2021**

(51) International Patent Classification (IPC):
***C07D 401/04*** (2006.01) ***C07D 401/10*** (2006.01)
***C07D 417/04*** (2006.01) ***C07D 491/048*** (2006.01)
***C07D 513/04*** (2006.01) ***C07F 5/02*** (2006.01)
***H01L 51/42*** (2006.01) ***C07D 209/12*** (2006.01)
***C07D 487/04*** (2006.01)

(52) Cooperative Patent Classification (CPC):
**C07D 209/12; C07D 401/04; C07D 401/10; C07D 417/04; C07D 487/04; C07D 491/048; C07D 513/04; C07F 5/02; H10K 30/00;**
Y02E 10/549

(86) International application number:
**PCT/JP2021/027028**

(87) International publication number:
**WO 2022/014721 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.07.2020 JP 2020123172**

(71) Applicant: **FUJIFILM Corporation**
**Tokyo 106-8620 (JP)**

(72) Inventors:
- **KANEKO Kazuhei**
**Ashigarakami-gun, Kanagawa 258-8577 (JP)**
- **YAMAMOTO Yosuke**
**Ashigarakami-gun, Kanagawa 258-8577 (JP)**

(74) Representative: **HGF**
**HGF Limited**
**1 City Walk**
**Leeds LS11 9DX (GB)**

# (54) PHOTOELECTRIC CONVERSION ELEMENT, IMAGING ELEMENT, OPTICAL SENSOR, AND COMPOUND

(57) An object of the present invention is to provide a photoelectric conversion element that includes a photoelectric conversion film excellent in the vapor deposition suitability, and that exhibits excellent external quantum efficiency to light at all wavelengths in a red wavelength range, a green wavelength range, and a blue wavelength range. Another object of the present invention is to provide an imaging element, an optical sensor, and a compound related to the photoelectric conversion element.

The photoelectric conversion element includes, in the following order, a conductive film, a photoelectric conversion film, and a transparent conductive film, in which the photoelectric conversion film contains a compound represented by Formula (1).

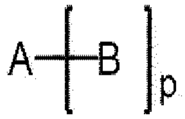 (1)

EP 4 183 780 A1

## Description

## BACKGROUND OF THE INVENTION

1. Field of the Invention

The present invention relates to a photoelectric conversion element, an imaging element, an optical sensor, and a compound.

2. Description of the Related Art

As a known solid-state imaging element, a planar solid-state imaging element in which photodiodes (PDs) are arranged two-dimensionally, and signal charges generated in each PD are read out through a circuit is widely used.

In order to achieve a color solid-state imaging element, a structure in which a color filter that transmits light at a specific wavelength is disposed on a light incident surface side of the planar solid-state imaging element is commonly adopted. Currently, a single plate-type solid-state imaging element in which color filters that transmit blue (B) light, green (G) light, and red (R) light are regularly arranged on each PD arranged two-dimensionally is well known. However, in this single plate-type solid-state imaging element, the light that has not passed through the color filters is not used, which causes poor light utilization efficiency.

In order to solve this disadvantage, a photoelectric conversion element having a structure in which an organic photoelectric conversion film is disposed on a substrate for reading signals has been developed in recent years.

For example, it is disclosed in JP2009-167348A that a photoelectric conversion element has a photoelectric conversion film containing a compound as described below (a compound 17 described in paragraph 0030).

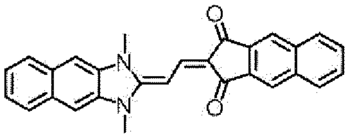

In addition, it is disclosed in JP2004-211096A that a photoelectric conversion element has a photoelectric conversion film containing a compound as described below (see claim 3).

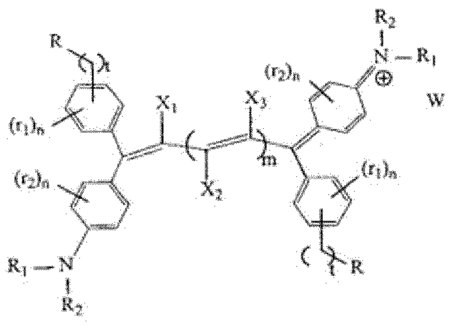

II

## SUMMARY OF THE INVENTION

In recent years, along with the demand for improving the performance of imaging elements, optical sensors, and the like, further improvements are required for various characteristics required for photoelectric conversion elements used therein.

For example, there is a demand for performance exhibiting an excellent external quantum efficiency to light at all wavelengths in a red wavelength range, a green wavelength range, and a blue wavelength range.

In addition, it is also required that a photoelectric conversion film can be manufactured by using a vapor deposition method.

As a result of examining the photoelectric conversion element described in JP2009-167348A, the present inventors have clarified that performance requirements for exhibiting an external quantum efficiency even excellent in light at all wavelengths in a red wavelength range, a green wavelength range, and a blue wavelength range are not satisfied, and there is a room for improving this situation. In addition, as a result of examining the photoelectric conversion

element described in JP2004-211096A, the present inventor has clarified that there may be challenges in manufacturing the photoelectric conversion film by using the vapor deposition method. Hereinafter, the manufacturing suitability of the photoelectric conversion film in the use of the vapor deposition method is also referred to as "vapor deposition suitability".

In view of the above circumstances, an object of the present invention is to provide a photoelectric conversion element that includes a photoelectric conversion film excellent in the vapor deposition suitability, and that exhibits the excellent external quantum efficiency to light at all wavelengths in a red wavelength range, a green wavelength range, and a blue wavelength range.

Another object of the present invention is to provide an imaging element, an optical sensor, and a compound related to the photoelectric conversion element.

The present inventors have conducted extensive studies on the above-described problems, and as a result, the inventors have found that it is possible to solve the above-described problems by configurations described below and have completed the present invention.

[1] A photoelectric conversion element comprising, in the following order, a conductive film, a photoelectric conversion film, and a transparent conductive film, the photoelectric conversion film containing a compound represented by Formula (1) described below.

[2] The photoelectric conversion element according to [1], in which in Formula (1), A represents an aromatic ring-containing methine coloring agent residue.

[3] The photoelectric conversion element according to [1] or [2], in which in Formula (1), an atom at a bonding position with B of the first monocyclic structure is a nitrogen atom.

[4] The photoelectric conversion element according to any one of [1] to [3], in which in Formula (1), A represents a residue derived from a compound represented by Formula (3) described later, which is formed by removing p hydrogen atom bonded to ring-constituting atoms forming a ring structure in the compound represented by Formula (3) described above, a residue derived from a compound represented by Formula (4) described later, which is formed by removing p hydrogen atom bonded to ring-constituting atoms forming a ring structure in the compound represented by Formula (4) described above, or a residue derived from a compound represented by Formula (5) described later, which is formed by removing p hydrogen atom bonded to ring-constituting atoms forming a ring structure in the compound represented by Formula (5) described above.

[5] The photoelectric conversion element according to any one of [1] to [4], in which in Formula (1), B represents an aromatic ring-containing methine coloring agent residue or a pyrromethene coloring agent residue.

[6] The photoelectric conversion element according to any one of [1] to [5], in which in Formula (1), B represents a residue derived from a compound represented by Formula (6) described later, which is formed by removing one hydrogen atom bonded to ring-constituting atoms forming a ring structure in the compound represented by Formula (6) described above.

[7] The photoelectric conversion element according to any one of [1] to [6], in which the photoelectric conversion film further includes a n-type organic semiconductor, and has a bulk hetero structure formed in a state where the compound represented by Formula (1) and the n-type organic semiconductor are mixed with each other.

[8] The photoelectric conversion element according to any one of [1] to [7], in which the photoelectric conversion film further includes a p-type organic semiconductor.

[9] The photoelectric conversion element according to any one of [1] to [8], further comprising one or more interlayers between the conductive film and the transparent conductive film, in addition to the photoelectric conversion film.

[10] An imaging element comprising the photoelectric conversion element according to any one of [1] to [9].

[11] An optical sensor comprising the photoelectric conversion element according to any one of [1] to [9].

[12] A compound represented by Formula (1) described below.

[13] The compound according to [12], in which in Formula (1), A represents an aromatic ring-containing methine coloring agent residue.

[14] The compound according to [12] or [13], in which in Formula (1), an atom at a bonding position with B of the first monocyclic structure is a nitrogen atom.

[15] The compound according to any one of [12] to [14], in which in Formula (1), A represents a residue derived from a compound represented by Formula (3) described later, which is formed by removing p hydrogen atom bonded to ring-constituting atoms forming a ring structure in the compound represented by Formula (3) described above, a residue derived from a compound represented by Formula (4) described later, which is formed by removing p hydrogen atom bonded to ring-constituting atoms forming a ring structure in the compound represented by Formula (4) described above, or a residue derived from a compound represented by Formula (5) described later, which is formed by removing p hydrogen atom bonded to ring-constituting atoms forming a ring structure in the compound represented by Formula (5) described above.

[16] The compound according to any one of [12] to [15], in which in Formula (1), B represents an aromatic ring-containing methine coloring agent residue or a pyrromethene coloring agent residue.

[17] The compound according to any one of [12] to [16], in which in Formula (1), B represents a residue derived from a compound represented by Formula (6) described later, which is formed by removing one hydrogen atom bonded to ring-constituting atoms forming a ring structure in the compound represented by Formula (6) described above.

According to the present invention, it is possible to provide the photoelectric conversion element that includes the photoelectric conversion film excellent in the vapor deposition suitability, and that exhibits the excellent external quantum efficiency to light at all wavelengths in a red wavelength range, a green wavelength range, and a blue wavelength range.

In addition, according to the present invention, it is possible to provide the imaging element, the optical sensor, and the compound related to the photoelectric conversion element.

## BRIEF DESCRIPTION OF THE DRAWINGS

Fig. 1 is a schematic cross-sectional view illustrating a configuration example of a photoelectric conversion element.
Fig. 2 is a schematic cross-sectional view illustrating a configuration example of the photoelectric conversion element.

## DESCRIPTION OF THE PREFERRED EMBODIMENTS

Hereinafter, the present invention will be described in detail.

Configuration requirements will be described below based on the representative embodiments of the present invention, but the present invention is not limited to such embodiments.

In the present specification, a substituent for which whether it is substituted or unsubstituted is not specified may be further substituted with a substituent (for example, a substituent W described below) within the scope not impairing an intended effect. For example, the term "alkyl group" means an alkyl group, which may be substituted with a substituent (for example, a substituent W described below).

In addition, in the present specification, the numerical range represented by "to" means a range including numerical values denoted before and after "to" as a lower limit value and an upper limit value.

The bonding direction of a divalent group described in the present specification is not particularly limited, and for example, in a case of -CO-O-, both -CO-O- and -O-CO- may be adopted.

In the present specification, the term (hetero)aryl means aryl and heteroaryl.

[Photoelectric Conversion Element]

As a feature of the present invention, compared to the related art, there is a point that a compound represented by Formula (1) described below (hereinafter, also referred to as a "specific compound") is used in a photoelectric conversion film.

The above-described configuration enables the photoelectric conversion element according to an embodiment of the present invention to include the photoelectric conversion film excellent in the vapor deposition suitability, and to exhibit the excellent external quantum efficiency to light at all wavelengths in a red wavelength range, a green wavelength range, and a blue wavelength range.

The action mechanism of the photoelectric conversion element according to the embodiment of the present invention to exhibit the above effects is not clear. However, it is presumed that the photoelectric conversion element has photosensitivity and exhibits the predetermined external quantum efficiency to light at all wavelengths in a red wavelength range, a green wavelength range, and a blue wavelength range because the specific compound has a coloring agent residue satisfying predetermined characteristics represented by "A" and "B". In addition, as shown in Example columns, it has been also confirmed that the photoelectric conversion element exhibits further excellent external quantum efficiency to light at all wavelengths in a red wavelength range, a green wavelength range, and a blue wavelength range, in a case where the coloring agent residue has a specific structure. In addition, since the specific compound has a structure in which individual coloring agent residues are directly bonded to each other without a linker, and a dihedral angle $\alpha$ described later is in a predetermined numerical range, the structure is in a state in which the leveling is broken, resulting in which the intermolecular interaction between molecules is difficult to work. As a result, it is presumed that the photoelectric conversion film containing the specific compound has excellent vapor deposition suitability.

Hereinafter, the fact that the external quantum efficiency to light at each of wavelengths in a red wavelength range, a green wavelength range, and a blue wavelength range is more excellent, and/or the vapor deposition suitability is more excellent is also simply referred to as the "effect of the present invention is more excellent".

Hereinafter, suitable embodiments of the photoelectric conversion element of the present invention will be described with reference to the drawings.

Fig. 1 is a schematic cross-sectional view of one embodiment of a photoelectric conversion element of the present invention. A photoelectric conversion element 10a illustrated in Fig. 1 has a configuration in which a conductive film (hereinafter, also referred to as a lower electrode) 11 functioning as a lower electrode, an electron blocking film 16A, a photoelectric conversion film 12 containing the specific compound described later, and a transparent conductive film (hereinafter, also referred to as an upper electrode) 15 functioning as an upper electrode are laminated in this order. Fig. 2 is a schematic cross-sectional view of another embodiment of the photoelectric conversion element of the present invention. A photoelectric conversion element 10b illustrated in Fig. 2 has a configuration in which the electron blocking film 16A, the photoelectric conversion film 12, a positive hole blocking film 16B, and the upper electrode 15 are laminated on the lower electrode 11 in this order. The lamination order of the electron blocking film 16A, the photoelectric conversion film 12, and the positive hole blocking film 16B in Figs. 1 and 2 may be appropriately changed according to the application and the characteristics.

In the photoelectric conversion element 10a (or 10b), it is preferable that light is incident on the photoelectric conversion film 12 through the upper electrode 15.

In a case where the photoelectric conversion element 10a (or 10b) is used, a voltage can be applied. In this case, it is preferable that the lower electrode 11 and the upper electrode 15 form a pair of electrodes, and a voltage of $1 \times 10^{-5}$ to $1 \times 10^{7}$ V/cm is applied between the pair of electrodes. From the viewpoint of the performance and power consumption, the applied voltage is more preferably $1 \times 10^{-4}$ to $1 \times 10^{7}$ V/cm, and still more preferably $1 \times 10^{-3}$ to $5 \times 10^{6}$ V/cm.

Regarding a voltage application method, in Figs. 1 and 2, it is preferable that the voltage is applied such that the electron blocking film 16A side is a cathode and the photoelectric conversion film 12 side is an anode. In a case where the photoelectric conversion element 10a (or 10b) is used as an optical sensor, or also in a case where the photoelectric conversion element 10a (or 10b) is incorporated in an imaging element, the voltage can be applied by the same method.

As described in detail below, the photoelectric conversion element 10a (or 10b) can be suitably applied to applications of the optical sensor and the imaging element.

Hereinafter, the form of each layer constituting the photoelectric conversion element according to the embodiment of the present invention will be described in detail.

[Photoelectric Conversion Film]

<Compound (Specific Compound) Represented by Formula (1)>

Hereinbelow, the compound (specific compound) represented by Formula (1) will be described in detail.

In the present specification, in a case where an aromatic ring-containing coloring agent residue represented by "A" or "B" in the specific compound is an aromatic ring-containing methine coloring agent residue, the specific compound includes both cis and trans isomers, which can be distinguished based on a C=C double bond contained in the aromatic ring-containing coloring agent residue. In other words, in the specific compound, a geometric isomer structure, which can be distinguished based on a C=C double bond contained in the aromatic ring-containing coloring agent residue, is not limited.

In the present specification, an "aromatic ring-containing methine coloring agent" is a coloring agent containing a chromophore having one or more aromatic rings and a non-aromatic methine moiety in a molecular structure (the non-aromatic methine moiety is a moiety represented by $*=C(R^T)-*$ ($R^T$ represents a hydrogen atom or a substituent, and * represents a bonding position) and a moiety represented by *=C-*, and $*=C(R^T)-*$ is preferable), and for example, the "aromatic ring-containing methine coloring agent" corresponds to a compound represented by Formula (3) described later. In addition, the aromatic ring-containing methine coloring agent residue is intended as a group in which a hydrogen atom at a predetermined position in an aromatic ring-containing methine coloring agent is removed to be a bonding site, and an example thereof includes a residue derived from the compound represented by Formula (3) described later.

That is, for example, in a case where "A" represents the residue derived from the compound represented by Formula (3) described later, Formula (3) includes cis and trans isomers of geometric isomers, which can be distinguished based on a C=C double bond composed of a carbon atom to which $R^{31}$ is bonded and a carbon atom adjacent to the carbon atom to which $R^{31}$ is bonded in Formula (3) described later. Formula (3) includes both cis and trans isomers of geometric isomers, which can be distinguished based on a C=C double bond composed of a carbon atom to which $R^{32}$ is bonded and a carbon atom adjacent to the carbon atom to which $R^{32}$ is bonded in Formula (3). In other words, in the residue derived from the compound represented by Formula (3), a geometric isomer structure, which can be distinguished based on a C=C double bond contained in the residue, is not limited.

The aromatic ring-containing methine coloring agent corresponds to compounds represented by Formulae (4)

to (6) described later in addition to the compound represented by Formula (3) described above. In a case where the above-described "A" represents a residue derived from the compound represented by Formula (4) described later or a residue derived from the compound represented by Formula (5) described later, the same applies to geometric isomers, which can be distinguished based on a C=C double bond contained in each residue. In addition, in a case where "B" represents a residue derived from the compound represented by Formula (6) described later, the same applies to a geometric isomer, which can be distinguished based on a C=C double bond contained in the above-described residue.

The specific compound is exemplified below.

$$A \left[ B \right]_p \quad (1)$$

In Formula (1),

A has $\Delta E_A$ of 2.00 to 3.20 eV and represents a first aromatic ring-containing coloring agent residue having a first monocyclic structure directly bonded to B.

B has $\Delta E_B$ of 2.80 to 4.00 eV and represents a second aromatic ring-containing coloring agent residue having a second monocyclic structure directly bonded to A.

p represents 1 or 2.

In the present specification, an aromatic ring-containing coloring agent is intended as a coloring agent having a chromophore containing one or more aromatic rings in a molecular structure. In addition, the aromatic ring-containing coloring agent residue is intended as a group formed by removing a hydrogen atom from the above-described aromatic ring-containing coloring agent.

The above-described aromatic ring may be any one of a monocyclic ring or a polycyclic ring. In addition, the aromatic ring may be any one of an aromatic hydrocarbon ring or an aromatic heterocyclic ring.

Furthermore, in a case where the above-described chromophore corresponds to a fused ring, at least one monocyclic ring contained in the fused ring may be an aromatic ring.

Here, examples of the chromophore containing one or more aromatic rings include quinacridone-based coloring agents, fluorescein-based coloring agents, phenazine-based coloring agents, phthalocyanine-based coloring agents, naphthalocyanine-based coloring agents, coumarin-based coloring agents, porphyrin-based coloring agents, oxazine-based coloring agents containing an aromatic ring in the molecule, pyrrolopyrrole-based coloring agents containing an aromatic ring in the molecule, diketopyrrolopyrrole-based coloring agents containing an aromatic ring in the molecule, furofuran-based coloring agents containing an aromatic ring in the molecule coloring agents, squarylium-based coloring agents containing an aromatic ring in the molecule, methine-based coloring agents containing an aromatic ring in the molecule (for example, thiophene-based coloring agents, thiazole-based coloring agents, pyrrole-based coloring agents, furan-based coloring agents, oxazole-based coloring agents, imidazole-based coloring agents, triarylamine-based coloring agents, and other methine-based coloring agents), and azo-based coloring agents containing an aromatic ring in the molecule (for example, azobenzene-based coloring agents, azonaphthalene-based coloring agents, and other azo-based coloring agents), and other coloring agents.

In addition, in the present specification, $\Delta E_A$ and $\Delta E_B$ are intended as numerical values obtained by the following method. $\Delta E_A$ and $\Delta E_B$ satisfy a relationship of $AE_A < \Delta E_B$.

$\Delta E_A$: in a case where a structure optimization calculation is performed on a compound UA obtained by replacing B with a hydrogen atom in the compound represented by Formula (1), by density functional calculation B3LYP/6-31G (d) with quantum chemical calculation software Gaussian09 (manufactured by Gaussian, Inc.) to obtain HOMO energy and LUMO energy, the obtained HOMO energy being represented by $E_{HOMO-A}$ (eV) and the obtained LUMO energy being represented by $E_{LUMO-A}$ (eV), $\Delta E_A$ represents a numeric value (eV) obtained by Formula (EA).

$$\text{Formula (EA): } \Delta E_A = E_{LUMO-A} - E_{HOMO-A}$$

$\Delta E_B$: in a case where a structure optimization calculation is performed on a compound UB obtained by replacing A with a hydrogen atom in the compound represented by Formula (1), by density functional calculation B3LYP/6-31G (d) with quantum chemical calculation software Gaussian09 (manufactured by Gaussian, Inc.) to obtain HOMO energy and LUMO energy, the obtained HOMO energy being represented by $E_{HOMO-B}$ (eV) and the obtained LUMO energy being represented by $E_{LUMO-B}$ (eV), $\Delta E_B$ represents a numeric value (eV) obtained by Formula (EB).

$$\text{Formula (EB): } \Delta E_B = E_{LUMO\text{-}B} - E_{HOMO\text{-}B}$$

$\Delta E_A$ is 2.00 to 3.20 eV, and $\Delta E_B$ is 2.80 to 4.00 eV. $\Delta E_A$ is preferably 2.30 to 2.80 eV from the viewpoint of further expanding the band of a photosensitive wavelength of the specific compound. In addition, $\Delta E_B$ is preferably 2.80 to 3.30 eV

The first aromatic ring-containing coloring agent residue represented by A contains, in the molecule, a first monocyclic structure that is directly bonded to B. That is, the first aromatic ring-containing coloring agent residue represented by A corresponds to a group formed by removing p hydrogen atom bonded to ring-constituting atoms forming a ring structure in the aromatic ring-containing coloring agent. Here, the first monocyclic structure may form a fused ring with one or more other monocyclic rings. That is, the first monocyclic structure included in the first aromatic ring-containing coloring agent residue represented by A means a monocyclic ring that is directly bonded to B, and this monocyclic ring may be configured to form a part of the fused ring.

The first monocyclic structure is preferably a heterocyclic ring in which an atom at a bonding position with B is a nitrogen atom, from the viewpoint that the effect of the present invention is more excellent.

In addition, from the viewpoint that the effect of the present invention is more excellent, in the first aromatic ring-containing coloring agent residue, the chromophore containing one or more aromatic rings is preferably a fused ring, and the first monocyclic structure is also preferably configured to form a part of the fused ring.

In addition, in a case where p in Formula (1) represents 2, the first monocyclic structure directly bonded to B may have the same monocyclic rings or different monocyclic rings. That is, two B's may be directly bonded to one monocyclic ring in the first aromatic ring-containing coloring agent residue represented by A, or two B's may be directly respectively bonded to two monocyclic rings different from each other in the first aromatic ring-containing coloring agent residue represented by A.

In addition, the first monocyclic structure may have a substituent. A substituent is not particularly limited, and examples thereof include groups exemplified by the substituent W described later.

The second aromatic ring-containing coloring agent residue represented by B contains, in the molecule, a second monocyclic structure that is directly bonded to A. That is, the second aromatic ring-containing coloring agent residue represented by B corresponds to a group formed by removing one hydrogen atom bonded to ring-constituting atoms forming a ring structure in the aromatic ring-containing coloring agent. Here, the second monocyclic structure may form a fused ring with one or more other monocyclic rings. That is, the second monocyclic structure included in the second aromatic ring-containing coloring agent residue represented by B means a monocyclic ring that is directly bonded to A, and this monocyclic ring may be configured to form a part of the fused ring.

In addition, from the viewpoint that the effect of the present invention is more excellent, in the second aromatic ring-containing coloring agent residue, the chromophore containing one or more aromatic rings is preferably a fused ring, and the second monocyclic structure is also preferably configured to form a part of the fused ring.

In addition, the second monocyclic structure may have a substituent. A substituent is not particularly limited, and examples thereof include groups exemplified by the substituent W described later.

In the compound represented by Formula (1), a dihedral angle $\alpha$ formed of the first monocyclic structure and the second monocyclic structure defined below is 30° to 90°. The dihedral angle $\alpha$ is defined by the following method.

dihedral angle $\alpha$: in a structure obtained by performing a structure optimization calculation on the compound represented by Formula (1), by density functional calculation B3LYP/6-31G (d) with quantum chemical calculation software Gaussian09 (manufactured by Gaussian, Inc.), the dihedral angle $\alpha$ is a dihedral angle having a smallest absolute value among four kinds of dihedral angles (X1) to (X4), which are able to be formed with four adjacent atoms represented by a to f in a partial structure corresponding to Formula (1-A) in Formula (1).

(X1) a-b-e-d

(X2) a-b-e-f

(X3) c-b-e-d

(X4) c-b-e-f

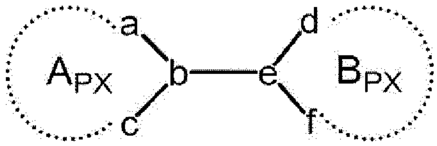

(1-A)

In Formula (1-A),

$A_{PX}$ corresponds to the first monocyclic structure contained in A in Formula (1), and at least contains three atoms represented by a, b, and c as ring-constituting atoms;
$B_{PX}$ corresponds to the second monocyclic structure contained in B in Formula (1), and at least contains three atoms represented by d, e, and f as ring-constituting atoms.

In Formula (1-A), all of bonds represented by a-b, c-b, d-e, e-f, and b-e are covalent bonds.
As described above, the first monocyclic structure is preferably a heterocyclic ring in which an atom at a bonding position with B is a nitrogen atom, from the viewpoint that the effect of the present invention is more excellent. That is, b in Formula (1-A) is preferably a nitrogen atom.
The dihedral angle $\alpha$ is 30° to 90°, preferably 45° to 90°, and more preferably 60° to 90° from the viewpoint that the effect of the present invention is more excellent.
p represents 1 or 2. p is preferably 1 from the viewpoint that the effect of the present invention is more excellent.
The above-described first aromatic ring-containing coloring agent residue represented by A and the above-described second aromatic ring-containing coloring agent residue represented by B are each preferably an aromatic ring-containing methine coloring agent residue, from the viewpoint that a molar absorption coefficient is high, and the external quantum efficiency is more improved, and a structure in which the chromophore containing one or more aromatic rings described above and a structure usually used in a merocyanine coloring agent as an acidic nucleus are bonded via a linking group represented by $*=C(R^A) - C(R^B)=*$ or $*=C(R^C)-*$ is more preferable.
$R^A$ to $R^C$ each independently represent a hydrogen atom or a substituent. * represents a bonding position.
A substituent is not particularly limited, and examples thereof include groups exemplified by the substituent W described later.
Among these, the above-described first aromatic ring-containing coloring agent residue represented by A still more preferably has a structure in which the chromophore containing one or more aromatic rings described above and a structure usually used in a merocyanine coloring agent as an acidic nucleus are bonded via a linking group represented by $*=C(R^A) - C(R^B)=*$. In addition, among these, the above-described second aromatic ring-containing coloring agent residue represented by B still more preferably has a structure in which the chromophore containing one or more aromatic rings described above and a structure usually used in a merocyanine coloring agent as an acidic nucleus are bonded via a linking group represented by $*=C(R^C)-*$.
Specific examples of the acidic nuclei are as follows:

(a) 1,3-dicarbonyl nuclei: for example, a 1,3-indandione nucleus, 1,3-cyclohexanedione, 5,5-dimethyl-1,3-cyclohexanedione, 1,3-dioxane-4,6-dione, and the like;
(b) pyrazolinone nuclei: for example, 1-phenyl-2-pyrazolin-5-one, 3-methyl-1-phenyl-2-pyrazolin-5-one, 1-(2-benzothiazolyl)-3-methyl-2-pyrazolin-5-one, and the like;
(c) isoxazolinone nuclei: for example, 3-phenyl-2-isoxazolin-5-one, 3-methyl-2-isoxazolin-5-one, and the like;
(d) oxindole nuclei: for example, 1-alkyl-2,3-hydro-2-oxindole, and the like;
(e) 2,4,6-trioxohexahydropyrimidine nuclei: for example, barbituric acid or 2-thibarbituric acid and derivatives thereof, and the like, and examples of the derivatives include 1-alkyl compounds such as 1-methyl and 1-ethyl, 1,3-dialkyl compounds such as 1,3-dimethyl, 1,3-diethyl, 1,3-dibutyl, 1,3-diaryl compounds such as 1,3-diphenyl, 1,3-di(p-chlorophenyl), 1,3-di(p-ethoxycarbonylphenyl), 1-alkyl-1-aryl compounds such as 1-ethyl-3-phenyl, 1,3-diheteroaryl compounds such as 1,3-di(2-pyridyl), and the like;
(f) 2-thio-2,4-thiazolidinedione nuclei: for example, rhodanine and derivatives thereof, and the like, and examples of the derivatives include 3-aklylrhodanine such as 3-methylrhodanine, 3-ethylrhodanine, 3-allylrhodanine, 3-arylrhodanine such as 3-phenylrhodanine, 3-heteroarylrhodanine such as 3-(2-pyridyl)rhodanine, and the like;
(g) 2-thio-2,4-oxazolidinedione (2-thio-2,4-(3H,5H)-oxazoledione nuclei: for example, 3-ethyl-2-thio-2,4-oxazolidinedione, and the like;
(h) thianaphthenone nuclei: for example, 3(2H)-thianaphthenone-1,1-dioxide, and the like;
(i) 2-thio-2,5-thiazolidinedione nuclei: for example, 3-ethyl-2-thio-2,5-thiazolidinedione, and the like;
(j) 2,4-thiazolidinedione nuclei: for example, 2,4-thiazolidinedione, 3-ethyl-2,4-thiazolidinedione, 3-phenyl-2,4-thiazolidinedione, and the like;

(k) thiazoliin-4-one nuclei: for example, 4-thiazolinone, 2-ethyl-4-thiazolinone, and the like;
(l) 2,4-imidazolidinedione (hydantoin) nuclei: for example, 2,4-imidazolidinedione, 3-ethyl-2,4-imidazolidinedione, and the like;
(m) 2-thio-2,4-imidazolidinedione (2-thiohydantoin) nuclei: for example, 2-thio-2,4-imidazolidinedione, 3-ethyl-2-thio-2,4-imidazolidinedione, and the like;
(n) imidazolin-5-one nuclei: for example, 2-propylmercapto-2-imidazolin-5-one, and the like;
(o) 3,5-pyrazolidinedione nuclei: for example, 1,2-diphenyl-3,5-pyrazolidinedione, 1,2-dimethyl-3,5-pyrazolidinedione, and the like;
(p) benzothiophen-3(2H)-one nuclei: for example, benzothiophen-3(2H)-one, oxobenzothiophen-3(2H)-one, dioxobenzothiophen-3(2H)-one, and the like;
(q) indanone nuclei: for example, 1-indanone, 3-phenyl-1-indanone, 3-methyl-1-indanone, 3,3-diphenyl-1-indanone, 3,3-dimethyl-1-indanone, and the like;
(r) benzofuran-3-(2H)-one nucleus: for example, benzofuran-3-(2H)-one, and the like;
(s) 2,2-dihydrophenalene-1,3-dione nucleus, and the like.

From the viewpoint that the effect of the present invention is more excellent, the first aromatic ring-containing coloring agent residue represented by A is preferably an aromatic ring-containing methine coloring agent residue, and preferably represents a residue derived from a compound represented by Formula (3) described later, which is formed by removing p hydrogen atom bonded to ring-constituting atoms forming a ring structure in the compound represented by Formula (3), a residue derived from a compound represented by Formula (4) described later, which is formed by removing p hydrogen atom bonded to ring-constituting atoms forming a ring structure in the compound represented by Formula (4), or a residue derived from a compound represented by Formula (5) described later, which is formed by removing p hydrogen atom bonded to ring-constituting atoms forming a ring structure in the compound represented by Formula (5).

The first aromatic ring-containing coloring agent residue represented by A is more preferably a residue derived from the compound represented by Formula (3), from the viewpoint that the external quantum efficiency to light at each of wavelengths in a red wavelength range, a green wavelength range, and a blue wavelength range is more excellent.

The term "ring-constituting atom forming a ring structure" is intended as an atom constituting a ring skeleton included in the molecule. The ring structure may be either aromatic or non-aromatic. In a case where the compound represented by Formula (3) is exemplified, examples of the ring structure include a ring represented by $A^3$, an aromatic ring represented by $B^3$, and a nitrogen-containing heterocyclic ring obtained by $B^3$ being fused. In addition, in a case where $R^{31}$ to $R^{34}$ in Formula (3) each independently represent a substituent having a ring structure (for example, an aryl group, a heteroaryl group, or other groups), the ring structure included in the above-described substituent is also applicable. In addition, for example, a ring-constituting atom in the nitrogen-containing heterocyclic ring obtained by $B^3$ being fused corresponds to three carbon atoms or two nitrogen atoms.

Hereinafter, each of the compounds represented by Formulae (3) to (5) will be described in detail.

(Compound Represented by Formula (3))

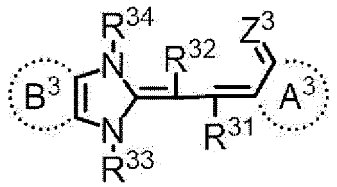

(3)

$A^3$ in Formula (3) represents a ring containing at least two carbon atoms. The two carbon atoms are intended as a carbon atom to which $Z^3$ in Formula (3) is bonded and a carbon atom adjacent to the carbon atom to which $Z^3$ is bonded, and the two carbon atoms are atoms that constitute $A^3$.

$A^3$ preferably has 3 to 30 carbon atoms, more preferably has 3 to 20 carbon atoms, and still more preferably has 3 to 15 carbon atoms. The number of carbon atoms described above includes two carbon atoms specified in Formula (3).

$A^3$ may have a heteroatom, and examples of the heteroatom include a nitrogen atom, a sulfur atom, an oxygen atom, a selenium atom, a tellurium atom, a phosphorus atom, a silicon atom, and a boron atom. Among these, the nitrogen atom, the sulfur atom, or the oxygen atom is preferable, and the oxygen atom is more preferable.

$A^3$ may have a substituent, and a halogen atom is preferable as the substituent.

$A^3$ preferably has 0 to 10 heteroatoms, more preferably has 0 to 5 heteroatoms, and still more preferably has 0 to 2 heteroatoms. The number of heteroatoms described above does not include the number of heteroatoms that the

group represented by $Z^3$ in Formula (3) contains and the number of halogen atoms that $A^3$ can have as a substituent.

$A^3$ may or may not exhibit aromaticity.

$A^3$ may have a monocyclic structure or a fused ring structure, but is preferably a 5-membered ring, a 6-membered ring, or a fused ring containing at least any one of a 5-membered ring or a 6-membered ring. The number of rings forming the above-described fused ring is preferably 2 to 4, and more preferably 2 to 3.

The ring represented by $A^3$ preferably has a group represented by Formula (A1). $*^1$ represents a bonding position with a carbon atom to which $Z^3$ specified in Formula (1) is bonded, and $*^2$ represents a bonding position with a carbon atom adjacent to the carbon atom to which $Z^3$ specified in Formula (3) is bonded.

$$*^1\text{-L-Y-Z-}*^2 \ldots \qquad \text{(A1)}$$

In Formula (A1), L represents a single bond or $-NR^L-$.

$R^L$ represents a hydrogen atom or a substituent. Among these, $R^L$ is preferably an alkyl group, an aryl group, or a heteroaryl group, which may have a substituent, and more preferably an alkyl group or an aryl group, which may have a substituent.

L is preferably a single bond.

Y represents $-CR^{Y1}=CR^{Y2}-$, $-CS-NR^{Y3}-$, -CS-, $-NR^{Y4}-$, or $-N=CR^{Y5}-$, and among these, $-CR^{Y1}=CR^{Y2}-$ is preferable.

$R^{Y1}$ to $R^{Y5}$ each independently represent a hydrogen atom or a substituent. Among these, $R^{Y1}$ to $R^{Y5}$ are each independently preferably an alkyl group, an aryl group, or a heteroaryl group, which may have a substituent, and more preferably an alkyl group or an aryl group, which may have a substituent.

In a case where Y represents $-CR^{Y1}=CR^{Y2}-$, $R^{Y1}$ and $R^{Y2}$ are preferably bonded to each other to form a ring, and $R^{Y1}$ and $R^{Y2}$ are more preferably bonded to each other to form a benzene ring.

Z represents a single bond, -CO-, -CS-, $-C(=NR^{Z1})-$, or $-C(=CR^{Z2}R^{Z3})-$, and among these, Z more preferably represents -CO- or $-C(=CR^{Z2}R^{Z3})-$, and still more preferably represents -CO-.

$R^{Z1}$ represents a hydrogen atom or a substituent.

The type of a substituent represented by $R^{Z1}$ is not particularly limited, and examples thereof include groups exemplified by the substituent W described later.

From the viewpoint that the effect of the present invention is more excellent, $R^{Z1}$ is preferably a hydrogen atom, or an alkyl group, an aryl group, or a heteroaryl group, which may have a substituent, and more preferably a hydrogen atom.

$R^{Z2}$ and $R^{Z3}$ each independently represent a cyano group or $-COOR^{Z4}$. $R^{Z4}$ represents an alkyl group, an aryl group, or a heteroaryl group, which may have a substituent.

Among these, $R^{Z2}$ and $R^{Z3}$ are each independently preferably a cyano group.

The combination of L, Y, and Z described above is preferably a combination of -L-Y-Z-, which is bonded to two carbon atoms specified in Formula (3) to form a ring that is a 5-membered ring or a 6-membered ring. However, as described above, the 5-membered ring or the 6-membered ring may be fused with a different ring (preferably a benzene ring) to form a fused ring structure.

Among these, the group represented by Formula (A1) is more preferably a group represented by Formula (A2).

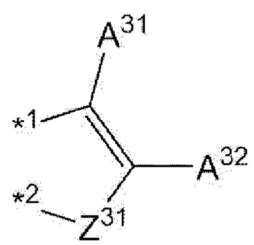

(A2)

In Formula (A2), $A^{31}$ and $A^{32}$ each independently represent a hydrogen atom or a substituent.

$A^{31}$ and $A^{32}$ are preferably bonded to each other to form a ring, and $A^{31}$ and $A^{32}$ are more preferably bonded to each other to form a benzene ring.

The above-described benzene ring formed by $A^{31}$ and $A^{32}$ further preferably has a substituent. As the substituent, a halogen atom is preferable, and a chlorine atom or a fluorine atom is more preferable.

Substituents that the above-described benzene ring formed by $A^{31}$ and $A^{32}$ has may be further bonded to each other to form a ring. For example, substituents that the above-described benzene ring formed by $A^{31}$ and $A^{32}$ has may be further bonded to each other to form a benzene ring.

*', $*^{2}$, and $Z^{31}$ in Formula (A2) each have the same definitions as $*^{1}$, $*^{2}$, and Z in Formula (A1) described above, and the suitable embodiments thereof are also the same.

Among these, the group represented by Formula (A1) is still more preferably a group represented by Formula (A3).

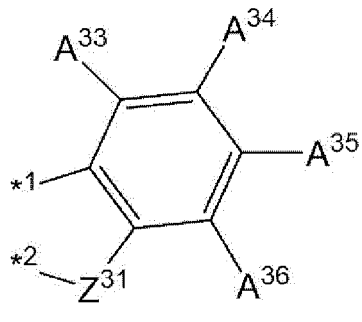

(A3)

In Formula (A3), $A^{33}$ to $A^{36}$ each independently represent a hydrogen atom or a substituent. Among these, $A^{33}$ to $A^{36}$ are each independently preferably a hydrogen atom or a halogen atom, and more preferably a hydrogen atom, a chlorine atom, or a fluorine atom, and still more preferably a hydrogen atom.

$A^{33}$ and $A^{34}$ may be bonded to each other to form a ring, $A^{34}$ and $A^{35}$ may be bonded to each other to form a ring, and $A^{35}$ and $A^{36}$ may be bonded to each other to form a ring. Rings formed by respectively bonding $A^{33}$ and $A^{34}$, $A^{34}$ and $A^{35}$, and $A^{35}$ and $A^{36}$ are each independently preferably a benzene ring. Among these, $A^{34}$ and $A^{35}$ are preferably bonded to each other to form a ring, and the ring formed by bonding $A^{34}$ and $A^{35}$ to each other is preferably a benzene ring. The ring formed by bonding $A^{34}$ and $A^{35}$ to each other may be further substituted with a substituent.

$*^{1}$, $*^{2}$, and $Z^{31}$ in Formula (A3) each have the same definitions as $*^{1}$, $*^{2}$, and Z in Formula (A1) described above, and the suitable embodiments thereof are also the same.

Such a ring represented by $A^{3}$ preferably has a structure usually used in a merocyanine coloring agent as an acidic nucleus, and specific examples thereof include nuclei of (a) to (s) described above.

$Z^{3}$ represents an oxygen atom, a sulfur atom, $=NR^{Z31}$, or $=CR^{Z32}R^{Z33}$, and among these, $Z^{3}$ is preferably an oxygen atom or $=CR^{Z32}R^{Z33}$, and more preferably an oxygen atom, from the viewpoint that the effect of the present invention is more excellent.

$R^{Z31}$ represents a hydrogen atom or a substituent.

The type of a substituent represented by $R^{Z31}$ is not particularly limited, and examples thereof include groups exemplified by the substituent W described later.

From the viewpoint that the effect of the present invention is more excellent, $R^{Z31}$ is preferably a hydrogen atom, or an alkyl group, an aryl group, or a heteroaryl group, which may have a substituent, and more preferably a hydrogen atom.

$R^{Z32}$ and $R^{Z33}$ each independently represent a cyano group or $-COOR^{Z34}$. $R^{Z34}$ represents an alkyl group, an aryl group, or a heteroaryl group, which may have a substituent.

Among these, $R^{Z32}$ and $R^{Z33}$ are each preferably a cyano group, from the viewpoint that the effect of the present invention is more excellent.

$R^{31}$ and $R^{32}$ each independently represent a hydrogen atom or a substituent.

The type of a substituent represented by each of $R^{31}$ and $R^{32}$ is not particularly limited, and examples thereof include groups exemplified by the substituent W described later.

$R^{33}$ and $R^{34}$ each independently represent an aryl group, a heteroaryl group, or an alkyl group, which may have a substituent, or a hydrogen atom.

From the viewpoint that the effect of the present invention is more excellent, among them, one of $R^{33}$ and $R^{34}$ represents a hydrogen atom, and a position where the hydrogen atom is removed is preferably a bonding position with the second aromatic ring-containing coloring agent residue represented by B described above. Furthermore, in the above aspect, it is more preferable that the other one of $R^{33}$ and $R^{34}$ represents an aryl group, a heteroaryl group, or an alkyl group, which may have a substituent.

$B^{3}$ represents an aromatic ring containing at least two carbon atoms. The two carbon atoms are intended as two carbon atoms that are ring-constituting atoms of a nitrogen-containing ring specified in Formula (3) and that are bonded through a double bond. Each carbon atom is an atom constituting $B^{3}$.

The aromatic ring represented by $B^{3}$ may have a substituent. The substituent is not particularly limited, and examples thereof include groups exemplified by the substituent W described later.

The aromatic ring may be a monocyclic ring or a polycyclic ring.

Examples of the aromatic ring include an aromatic hydrocarbon ring and an aromatic heterocyclic ring. Examples of the aromatic hydrocarbon ring include a benzene ring, a naphthalene ring, an anthracene ring, and a phenanthrene ring. Examples of the aromatic heterocyclic ring include a quinoxaline ring, a pyrazine ring, a pyrrole ring, a furan ring, a thiophene ring, an imidazole ring, and an oxazole ring. These rings may be further condensed with another ring (which may be a non-aromatic ring).

Among these, $B^3$ is preferably an aromatic heterocyclic ring, and more preferably a quinoxaline ring or a pyrazine ring.

(Compound Represented by Formula (4))

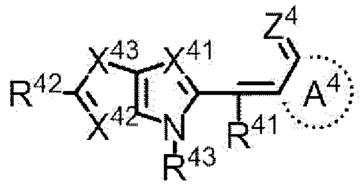

(4)

In Formula (4), $A^4$ represents a ring which contains at least two carbon atoms and may have a substituent. $A^4$ has the same definition as $A^3$ in Formula (3), and the suitable embodiment thereof is also the same.

$Z^4$ represents an oxygen atom, a sulfur atom, $=NR^{Z41}$, or $=CR^{Z42}R^{Z43}$, and among these, $Z^4$ is preferably an oxygen atom or $=CR^{Z42}R^{Z43}$, and more preferably an oxygen atom, from the viewpoint that the effect of the present invention is more excellent.

$R^{Z41}$ represents a hydrogen atom or a substituent.

The type of a substituent represented by $R^{Z41}$ is not particularly limited, and examples thereof include groups exemplified by the substituent W described later.

From the viewpoint that the effect of the present invention is more excellent, $R^{Z41}$ is preferably a hydrogen atom, or an alkyl group, an aryl group, or a heteroaryl group, which may have a substituent, and more preferably a hydrogen atom.

$R^{Z42}$ and $R^{Z43}$ each independently represent a cyano group or $-COOR^{Z44}$. $R^{Z44}$ represents an alkyl group, an aryl group, or a heteroaryl group, which may have a substituent.

Among these, $R^{Z42}$ and $R^{Z43}$ are each preferably a cyano group, from the viewpoint that the effect of the present invention is more excellent.

$R^{41}$ and $R^{42}$ each independently represent a hydrogen atom or a substituent.

The type of a substituent represented by each of $R^{41}$ and $R^{42}$ is not particularly limited, and examples thereof include groups exemplified by the substituent W described later.

Among these, $R^{42}$ is preferably an alkyl group, an aryl group, or a heteroaryl group, which may have a substituent.

$R^{43}$ represents an aryl group, a heteroaryl group, or an alkyl group, which may have a substituent, or a hydrogen atom.

From the viewpoint that the effect of the present invention is more excellent, among these, $R^{43}$ represents a hydrogen atom, and a position where the hydrogen atom is removed is preferably a bonding position with the second aromatic ring-containing coloring agent residue represented by B described above.

$X^{41}$ and $X^{42}$ each independently represent a nitrogen atom or $CR^{X4}$. $R^{x4}$ represents a hydrogen atom or a substituent.

$X^{43}$ each independently represents an oxygen atom, a sulfur atom, or $NR^{X4}$. $R^{X4}$ represents a hydrogen atom or a substituent.

The type of a substituent represented by $R^{X4}$ is not particularly limited, and examples thereof include groups exemplified by the substituent W described later.

An oxygen atom or a sulfur atom is preferable as $X^{43}$.

(Compound Represented by Formula (5))

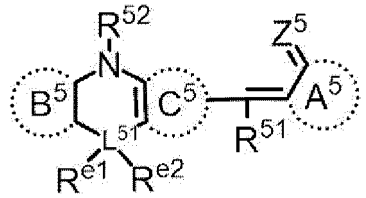

(5)

In Formula (5), $A^5$ represents a ring which contains at least two carbon atoms and may have a substituent. $A^5$ has the same definition as $A^3$ in Formula (3), and the suitable embodiment thereof is also the same.

$Z^5$ represents an oxygen atom, a sulfur atom, $=NR^{Z51}$, or $=CR^{Z52}R^{Z53}$.

$R^{Z51}$ represents a hydrogen atom or a substituent.

The type of a substituent represented by $R^{Z51}$ is not particularly limited, and examples thereof include groups exemplified by the substituent W described later.

From the viewpoint that the effect of the present invention is more excellent, $R^{Z51}$ is preferably a hydrogen atom, or an alkyl group, an aryl group, or a heteroaryl group, which may have a substituent, and more preferably a hydrogen atom.

$R^{Z52}$ and $R^{Z53}$ each independently represent a cyano group or $-COOR^{Z54}$. $R^{Z54}$ represents an alkyl group, an aryl group, or a heteroaryl group, which may have a substituent.

Among these, $R^{Z52}$ and $R^{Z53}$ are each preferably a cyano group, from the viewpoint that the effect of the present invention is more excellent.

$R^{51}$ represents a hydrogen atom or a substituent.

The type of a substituent represented by $R^{51}$ is not particularly limited, and examples thereof include groups exemplified by the substituent W described later.

$R^{52}$ represents an aryl group, a heteroaryl group, or an alkyl group, which may have a substituent, or a hydrogen atom.

From the viewpoint that the effect of the present invention is more excellent, among these, $R^{52}$ represents a hydrogen atom, and a position where the hydrogen atom is removed is preferably a bonding position with the second aromatic ring-containing coloring agent residue represented by B described above.

$R^{e1}$ and $R^{e2}$ each independently represent a substituent.

A substituent represented by each of $R^{e1}$ and $R^{e2}$ is an aryl group, a heteroaryl group, or an alkyl group, which may have a substituent. $R^{e1}$ and $R^{e2}$ may be bonded to each other to form a ring.

$L^{51}$ represents a carbon atom, a silicon atom, or a germanium atom.

$B^5$ represents an aromatic ring containing at least two carbon atoms. The two carbon atoms are ring-constituting atoms of a nitrogen-containing ring specified in Formula (5), and are intended as a carbon atom bonded to a nitrogen atom and a carbon atom adjacent to the carbon atom bonded to a nitrogen atom. Each carbon atom is an atom constituting $B^5$.

The aromatic ring represented by $B^5$ may have a substituent. The substituent is not particularly limited, and examples thereof include groups exemplified by the substituent W described later.

The aromatic ring may be a monocyclic ring or a polycyclic ring.

Examples of the aromatic ring include an aromatic hydrocarbon ring and an aromatic heterocyclic ring. Specific examples of the aromatic hydrocarbon ring and the aromatic heterocyclic ring include the same as the aromatic ring represented by $B^3$ in Formula (3) described above.

$C^5$ represents an aromatic ring containing at least three carbon atoms. The three carbon atoms are ring-constituting atoms of a nitrogen-containing ring specified in Formula (5), and are intended as two carbon atoms bonded through a double bond and a carbon atom adjacent to the carbon atom to which $R^{51}$ is bonded. Each carbon atom is an atom constituting $C^5$.

The aromatic ring represented by $C^5$ may have a substituent. The substituent is not particularly limited, and examples thereof include groups exemplified by the substituent W described later.

The aromatic ring may be a monocyclic ring or a polycyclic ring.

Examples of the aromatic ring include an aromatic hydrocarbon ring and an aromatic heterocyclic ring. Specific examples of the aromatic hydrocarbon ring and the aromatic heterocyclic ring include the same as the aromatic ring represented by $B^3$ in Formula (3) described above.

The second aromatic ring-containing coloring agent residue represented by B is preferably selected from the group consisting of an aromatic ring-containing methine coloring agent residue and a pyrromethene coloring agent residue, more preferably an aromatic ring-containing methine coloring agent residue, and still more preferably a residue

derived from the compound represented by Formula (6) described later, which is formed by removing one hydrogen atom bonded to ring-constituting atoms forming a ring structure in the compound represented by Formula (6).

Hereinafter, the compound represented by Formula (6) will be described in detail.

(Compound Represented by Formula (6))

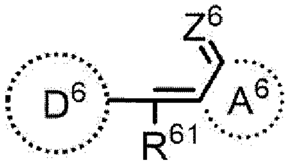

(6)

In Formula (6), $A^6$ represents a ring which contains at least two carbon atoms and may have a substituent. $A^6$ has the same definition as $A^3$ in Formula (3), and the suitable embodiment thereof is also the same.

$Z^6$ represents an oxygen atom, a sulfur atom, $=NR^{Z61}$, or $=CR^{Z62}R^{Z63}$, and among these, $Z^6$ is preferably an oxygen atom or $=CR^{Z62}R^{Z63}$, and more preferably an oxygen atom, from the viewpoint that the effect of the present invention is more excellent.

$R^{Z61}$ represents a hydrogen atom or a substituent.

The type of a substituent represented by $R^{Z61}$ is not particularly limited, and examples thereof include groups exemplified by the substituent W described later.

From the viewpoint that the effect of the present invention is more excellent, $R^{Z61}$ is preferably a hydrogen atom, or an alkyl group, an aryl group, or a heteroaryl group, which may have a substituent, and more preferably a hydrogen atom.

$R^{Z62}$ and $R^{Z63}$ each independently represent a cyano group or $-COOR^{Z64}$. $R^{Z64}$ represents an alkyl group, an aryl group, or a heteroaryl group, which may have a substituent.

Among these, $R^{Z62}$ and $R^{Z63}$ are each preferably a cyano group, from the viewpoint that the effect of the present invention is more excellent.

$D^6$ represents a heteroaryl group which may have a substituent. The kinds of an atom that adjacent to a carbon atom to which $R^{61}$ is bonded, which is specified in Formula (6), and that is bonded to the heteroaryl group represented by $D^6$ are not limited, and examples thereof include a carbon atom, a nitrogen atom, and other kinds of atoms.

A heteroaryl ring represented by $D^6$ may have a substituent. The substituent is not particularly limited, and examples thereof include groups exemplified by the substituent W described later.

The heteroaryl group represented by $D^6$ may be a monocyclic ring or a polycyclic ring.

From the viewpoint that the effect of the present invention is more excellent, among these, $D^6$ is preferably a heteroaryl group having a polycyclic structure, or a heteroaryl group having, as a substituent, an aryl group or a heteroaryl group. The aryl group or the heteroaryl group may have a substituent. The substituent is not particularly limited, and examples thereof include groups exemplified by the substituent W described later.

The aromatic heterocyclic ring that constitutes the heteroaryl group having a polycyclic structure is not particularly limited, and examples thereof include a quinoxaline ring, an indole ring, a benzofuran ring, a benzothiophene ring, a benzoimidazole ring, and a benzoxazole ring.

In addition, from the viewpoint that the effect of the present invention is more excellent, the residue derived from the compound represented by Formula (6) is preferably a residue formed by removing one hydrogen atom bonded to ring-constituting atoms forming the above-described heteroaryl group having a polycyclic structure or a residue formed by removing one hydrogen atom bonded to ring-constituting atoms forming any ring structure in the heteroaryl group having the above aryl group or the heteroaryl group as a substituent.

$R^{61}$ represents a hydrogen atom or a substituent.

The type of a substituent represented by $R^{61}$ is not particularly limited, and examples thereof include groups exemplified by the substituent W described later.

As the residue derived from the compound represented by Formula (6), for example, Formula (6A) or Formula (6B) described below is preferable.

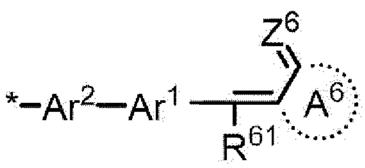

(6A)

$A^6$, $Z^6$, and $R^{61}$ in Formula (6A) each have the same definitions as $A^6$, $Z^6$, and $R^{61}$ in Formula (6), and the suitable embodiments thereof are also the same.

$Ar^1$ in Formula (6A) represents a heteroarylene group which may have a substituent.

The substituent is not particularly limited, and examples thereof include groups exemplified by the substituent W described later.

The heteroarylene group represented by $Ar^1$ may be a monocyclic ring or a polycyclic ring. The aromatic heterocyclic ring constituting the heteroarylene group of a monocyclic ring is not particularly limited, and examples thereof include a thiophene ring, a pyrrole ring, a furan ring, a pyridine ring, a pyridazine ring, a pyrimidine ring, a pyrazine ring, a triazine ring, a thiazole ring, an oxazole ring, and an imidazole ring. The aromatic heterocyclic ring that constitutes the heteroarylene group having a polycyclic structure is not particularly limited, and examples thereof include a quinoxaline ring, an indole ring, a benzofuran ring, a benzothiophene ring, a benzoimidazole ring, and a benzoxazole ring.

$Ar^2$ in Formula (6A) represents an arylene group or a heteroarylene group, which may have a substituent. The substituent is not particularly limited, and examples thereof include groups exemplified by the substituent W described later. As $Ar^2$, an arylene group which may have a substituent is preferable, and a phenylene group which may have a substituent is more preferable.

In Formula (6A), * represents a bonding position.

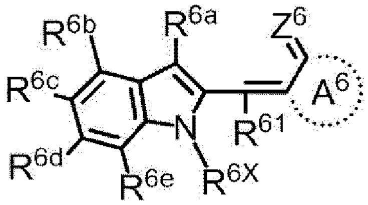

(6B)

$A^6$, $Z^6$, and $R^{61}$ in Formula (6B) each have the same definitions as $A^6$, $Z^6$, and $R^{61}$ in Formula (6), and the suitable embodiments thereof are also the same.

In Formula (6B), $R^{6a}$ to $R^{6e}$, and $R^{6X}$ each independently represent a hydrogen atom or a substituent. Substituents represented by $R^{6a}$ to $R^{6e}$, and $R^{6X}$ are not particularly limited, and examples thereof include groups exemplified by the substituent W described later.

At least one of Formula (6B), $R^{6a}$ to $R^{6e}$, and $R^{6X}$ represents a hydrogen atom, and a position where this hydrogen atom is removed represents a bonding position (*).

In a case where the second aromatic ring-containing coloring agent residue represented by B is a pyrromethene coloring agent residue, the pyrromethene coloring agent residue preferably has a structure represented by Formula (X1) described below.

Formula (X1)

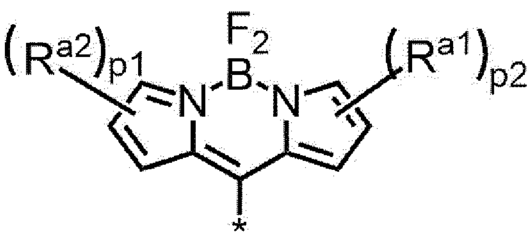

In Formula, $R^{a1}$ and $R^{a2}$ each represent a substituent.

The type of substituents represented by $R^{a1}$ and $R^{a2}$ is not particularly limited, and examples thereof include groups exemplified by the substituent W described later.

p1 and p2 each independently represent an integer of 0 to 3.
* represents a bonding position.

The specific compound preferably does not contain any of a carboxy group and a salt of the carboxy group, a phosphoric acid group and a salt of the phosphoric acid group, and a sulfonic acid group and a salt of the sulfonic acid group, from the viewpoint that the vapor deposition suitability of the photoelectric conversion film is more excellent.

(Substituent W)

A substituent W in the present specification will be described below.

Examples of the substituent W include a halogen atom, an alkyl group (including a cycloalkyl group, a bicycloalkyl group, and a tricycloalkyl group), an alkenyl group (including a cycloalkenyl group and a bicycloalkenyl group), an alkynyl group, an aryl group, a heterocyclic group (may also be referred to as a heterocyclic group), a cyano group, a hydroxy group, a nitro group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group (including an anilino group), an ammonio group, an acylamino group, an aminocarbonylamino group, an alkoxycarbonylamino group, an aryloxycarbonylamino group, a sulfamoylamino group, an alkyl or an arylsulfonylamino group, a mercapto group, an alkylthio group, an arylthio group, a heterocyclic thio group, a sulfamoyl group, an alkyl or an arylsulfinyl group, an alkyl or an arylsulfonyl group, an acyl group, an aryloxycarbonyl group, an alkoxycarbonyl group, a carbamoyl group, an aryl or a heterocyclic azo group, an imide group, a phosphino group, a phosphinyl group, a phosphinyloxy group, a phosphinylamino group, a phosphono group, a silyl group, a hydrazino group, a ureido group, a boronate group ($-B(OH)_2$), and other known substituents.

In addition, the substituent W may be further substituted with another substituent W. For example, an alkyl group may be substituted with a halogen atom.

The details of the substituent W are described in paragraph [0023] of JP2007-234651A.

However, as described above, the specific compound preferably does not contain any of a carboxy group and a salt of the carboxy group, a phosphoric acid group and a salt of the phosphoric acid group, and a sulfonic acid group and a salt of the sulfonic acid group, from the viewpoint of avoiding deterioration of the vapor deposition suitability.

(Alkyl Group, Aryl group, Heteroaryl Group, Arylene Group, and Heteroarylene Group that Specific Compound May Have)

The number of carbon atoms of an alkyl group contained in the specific compound (the residue derived from each of the compounds represented by Formulae (3) to (6) in the specific compound) is not particularly limited, but the alkyl group preferably has 1 to 10 carbon atoms, more preferably has 1 to 6 carbon atoms, and still more preferably has 1 to 4 carbon atoms. The alkyl group may be any of linear, branched, or cyclic. In addition, the alkyl group may be substituted with a substituent (for example, a substituent W).

Examples of the alkyl group include a methyl group, an ethyl group, a n-propyl group, an i-propyl group, a n-butyl group, a t-butyl group, a n-hexyl group, a cyclohexyl group, and the like.

The number of carbon atoms of an aryl group contained in the specific compound (the residue derived from each of the compounds represented by Formulae (3) to (6) in the specific compound) is not particularly limited, but the aryl group preferably has 6 to 30 carbon atoms, more preferably has 6 to 18 carbon atoms, and still more preferably has 6 carbon atoms. The aryl group may have a monocyclic structure or a fused ring structure (fused ring structure) in which two or more rings are fused to form a ring. In addition, the aryl group may be substituted with a substituent (for example, a substituent W).

Examples of the aryl group include a phenyl group, a naphthyl group, an anthryl group, a pyrenyl group, a phenanthrenyl group, a methylphenyl group, a dimethylphenyl group, a biphenyl group, a fluorenyl group, and the like, and a phenyl group, a naphthyl group, or an anthryl group is preferable.

An example of the arylene group (divalent aromatic hydrocarbon ring group) contained in the specific compound (the residue derived from each of the compounds represented by Formulae (3) to (6) in the specific compound) includes a group formed by further removing one hydrogen atom from an aryl group (monovalent aromatic hydrocarbon ring group) contained in the specific compound (the residue derived from each of the compounds represented by Formulae (3) to (6) in the specific compound).

The number of carbon atoms in a heteroaryl group (monovalent aromatic heterocyclic group) contained in the specific compound (residue derived from each of the compounds represented by Formulae (3) to (6)) is not particularly limited, but the heteroaryl group preferably has 3 to 30 carbon atoms, and more preferably has 3 to 18 carbon atoms. The heteroaryl group may be substituted with a substituent (for example, a substituent W).

The heteroaryl group contains a heteroatom in addition to a carbon atom and a hydrogen atom. Examples of the heteroatom include a sulfur atom, an oxygen atom, a nitrogen atom, a selenium atom, a tellurium atom, a phosphorus atom, a silicon atom, and a boron atom, and a sulfur atom, an oxygen atom, or a nitrogen atom is preferable.

The number of heteroatoms of the heteroaryl group is not particularly limited, but is usually about 1 to 10, preferably 1 to 4, and more preferably 1 and 2.

The number of ring members of the heteroaryl group is not particularly limited, but is preferably 3 to 8, more preferably 5 to 7, and still more preferably 5 to 6. The heteroaryl group may have a monocyclic structure or a fused ring structure (fused ring structure) in which two or more rings are fused to form a ring. In a case of a fused ring structure, an aromatic hydrocarbon ring having no heteroatom (for example, a benzene ring) may be contained.

Examples of the heteroaryl group include a pyridyl group, a quinolyl group, an isoquinolyl group, an acridinyl group, a phenanthridinyl group, a pteridinyl group, a pyrazinyl group, a quinoxalinyl group, a pyrimidinyl group, a quinazolyl group, a pyridazinyl group, a cinnolinyl group, a phthalazinyl group, a triazinyl group, an oxazolyl group, a benzoxazolyl group, a thiazolyl group, a benzothiazolyl group, an imidazolyl group, a benzimidazolyl group, a pyrazolyl group, an indazolyl group, an isoxazolyl group, a benzisoxazolyl group, an isothiazolyl group, a benzisothiazolyl group, an oxadiazolyl group, a thiadiazolyl group, a triazolyl group, a tetrazolyl group, a furyl group, a benzofuryl group, a thienyl group, a benzothienyl group, a dibenzofuryl group, a dibenzothienyl group, a pyrrolyl group, an indolyl group, an imidazopyridinyl group, a carbazolyl group, and the like.

An example of the heteroarylene group (divalent aromatic heterocyclic group) contained in the specific compound (the residue derived from each of the compounds represented by Formulae (3) to (6) in the specific compound) includes a group formed by further removing one hydrogen atom from an heteroaryl group (monovalent aromatic heterocyclic group) contained in the specific compound (the residue derived from each of the compounds represented by Formulae (3) to (6) in the specific compound).

Hereinafter, specific examples of the compound represented by Formula (1) will be described, but the present embodiment is not limited thereto.

In a case where the following compound includes a C=C double bond, the geometric isomer structure that can be distinguished based on a C=C double bond is not limited.

In each compound illustrated in «Specific Examples of Compound Represented by Formula (1)» below, "$B^1$" represents one coloring agent residue selected from «B Residue Group 1» described later, and "$B^2$" represents one coloring agent residue selected from «B Residue Group 2» described later. In addition, * in the coloring agent residues listed in «B Residue Group 1>> and «B Residue Group 2» described later represents a bonding position.

In addition, in <<Specific Examples of Compound Represented by Formula (1)», moieties excluding "$B^1$" and "$B^2$" each correspond to the first aromatic ring-containing coloring agent residue represented by "A" described above. In addition, "$B^1$" and "$B^2$" each correspond to the second aromatic ring-containing coloring agent residue described above.

A more specific example is as follows. For example, in a case of the leftmost compound in the first column of the compounds represented by «Specific Examples of Compound Represented by Formula (1)» below (hereinafter, also referred to as a "compound (P1)"), "$B^1$" in the compound may be any of coloring agent residues exemplified in «B Residue Group 1» described below. For example, in a case where "$B^1$" is the leftmost coloring agent residue in the first column shown in «B Residue Group 1>> (hereinafter, also referred to as a "coloring agent residue (P2)"), the compound (P1) corresponds to a compound represented by Formula (P3) described below.

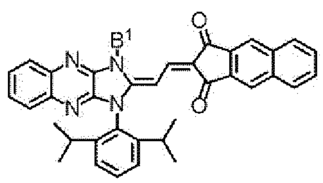
(P1)

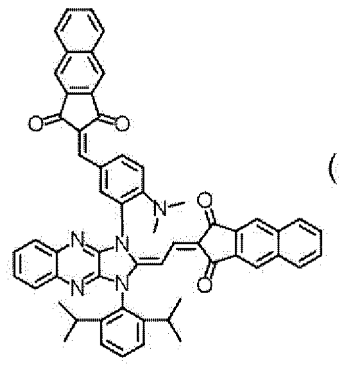
(P3)

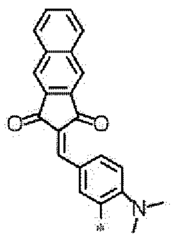
(P2)

«Specific Examples of Compound Represented by Formula (1)»

In a case where the following compound includes a C=C double bond, the geometric isomer structure that can be distinguished based on a C=C double bond is not limited.

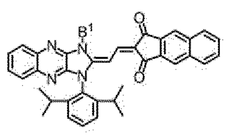 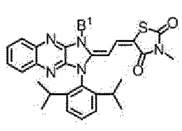 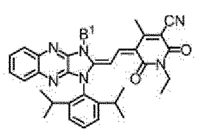 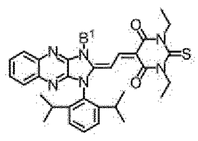

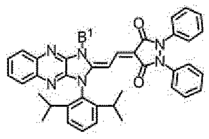
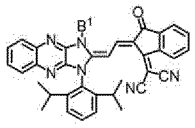
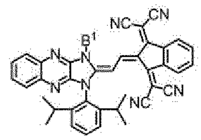
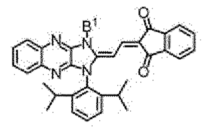

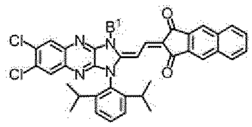
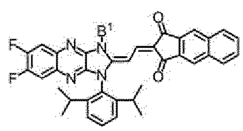
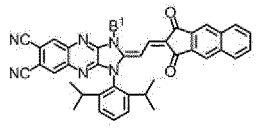
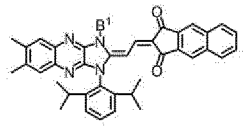

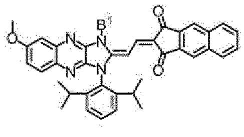
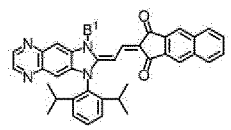
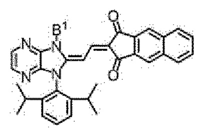
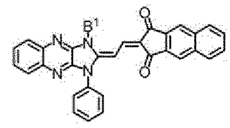

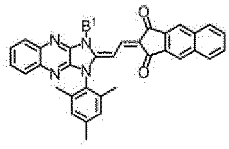
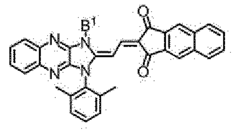
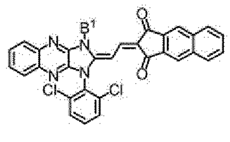
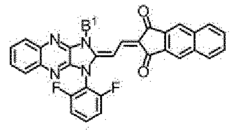

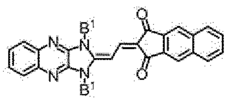
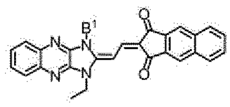
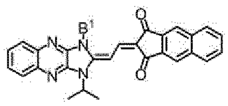
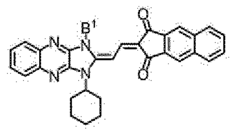

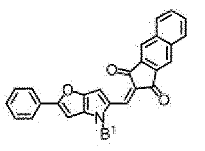
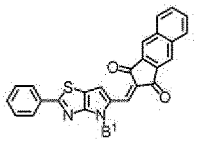
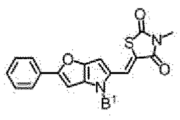
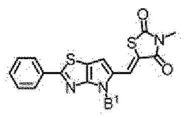

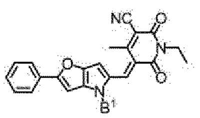
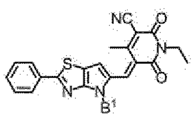
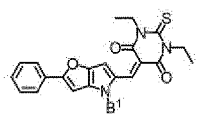
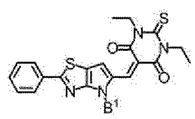

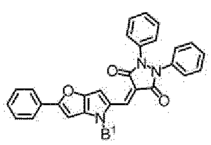
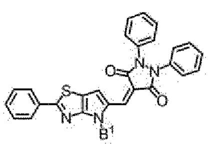
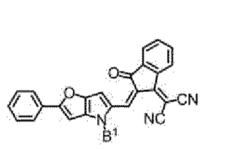
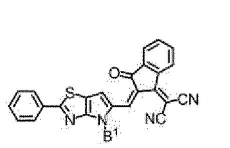

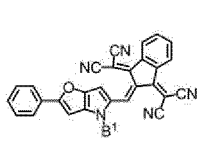
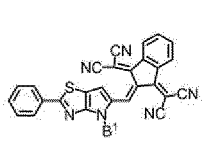
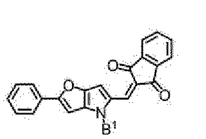
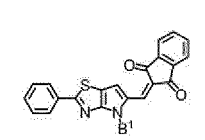

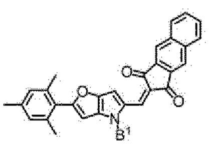
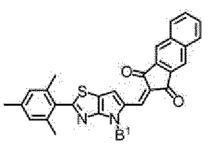
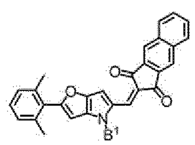
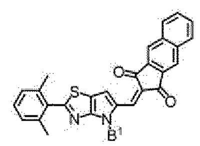

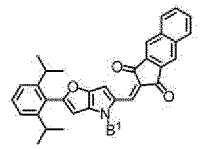
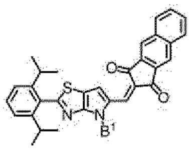
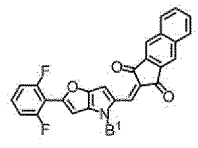
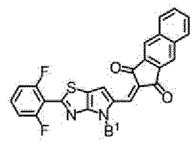
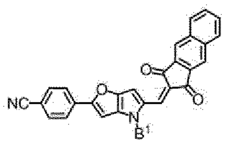
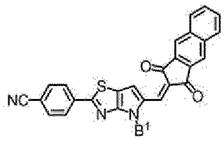
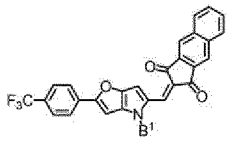
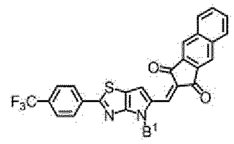
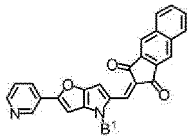
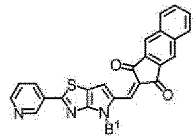
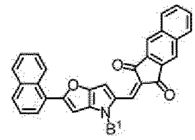
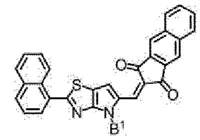
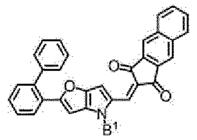
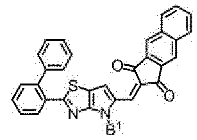
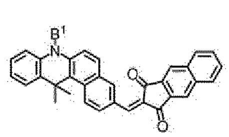
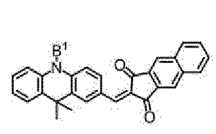
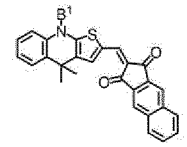
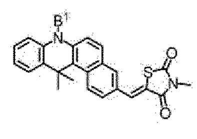
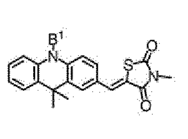
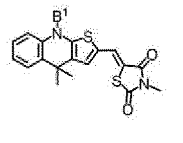
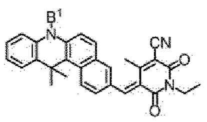
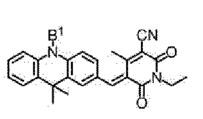
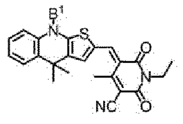
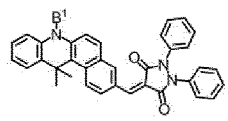
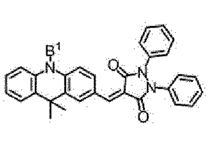
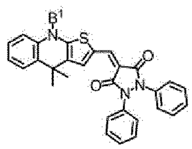
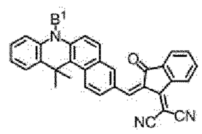
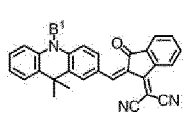
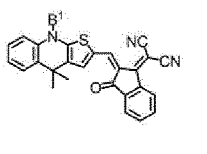
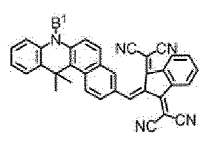
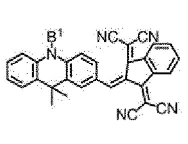
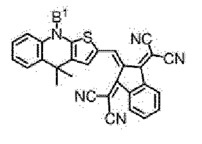
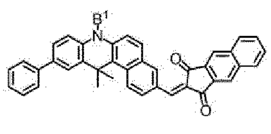
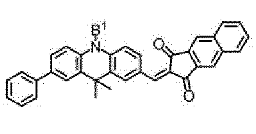
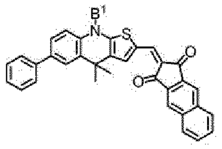
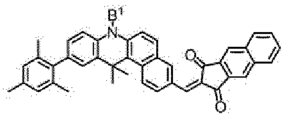

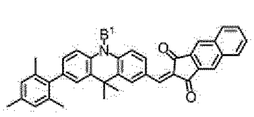
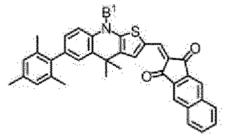
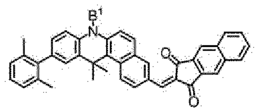
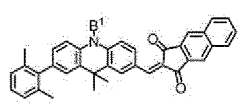

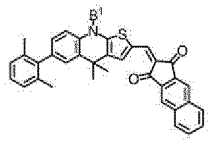
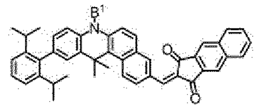
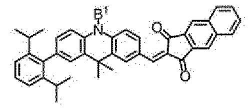
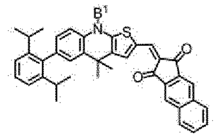

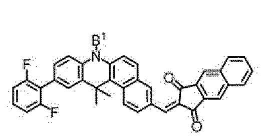
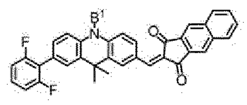
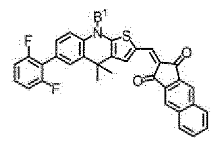
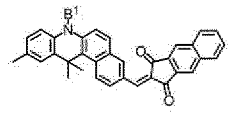

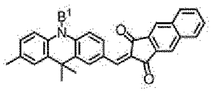
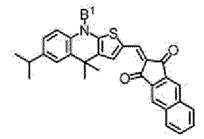
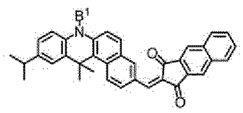
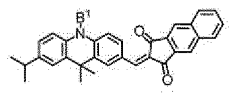

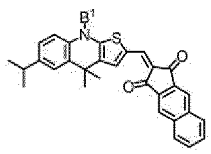
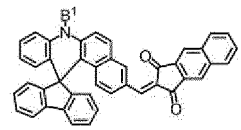
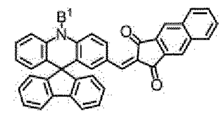
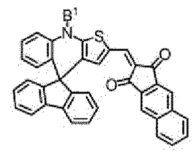

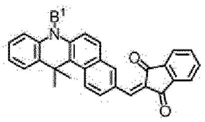
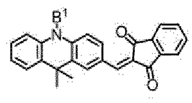
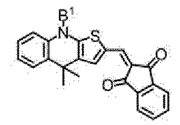
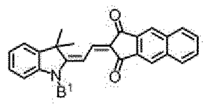

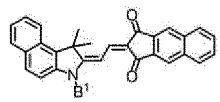
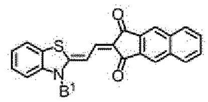
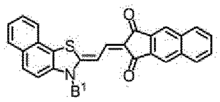
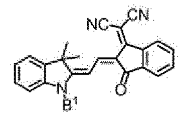

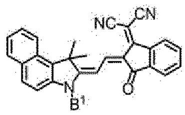
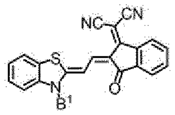
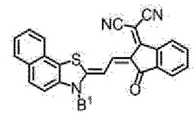
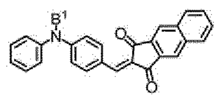

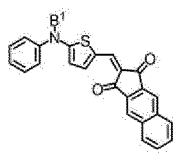
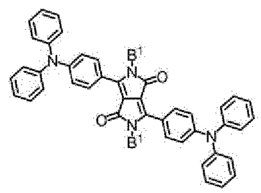
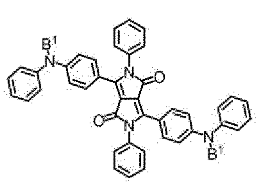
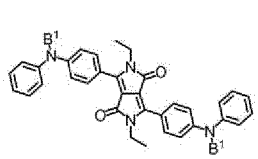

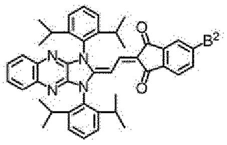 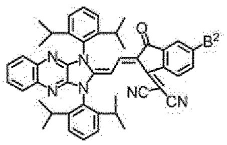 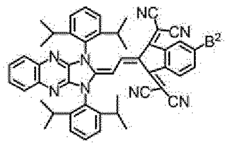 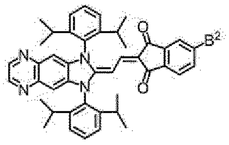

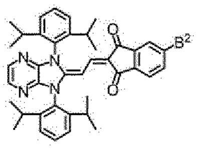 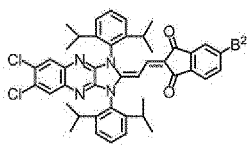 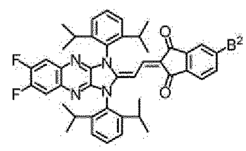 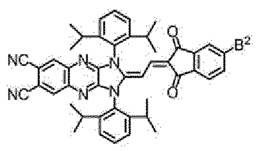

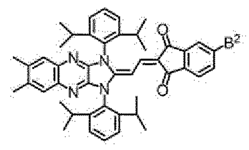 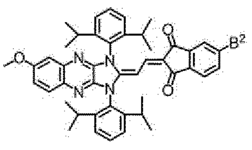 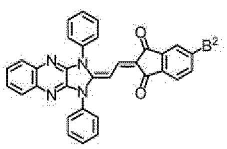 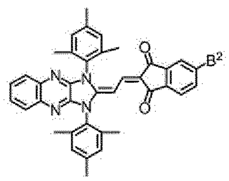

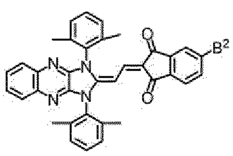 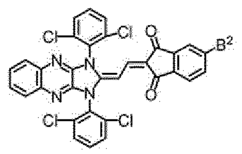 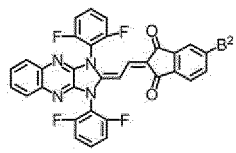 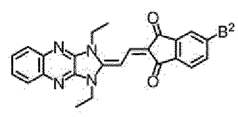

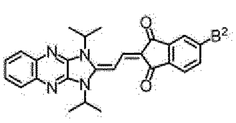 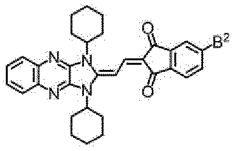 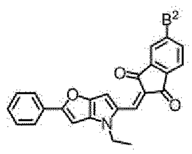 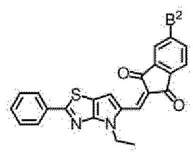

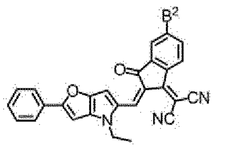 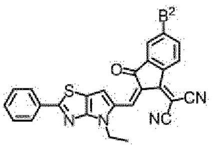 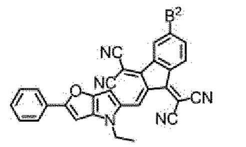 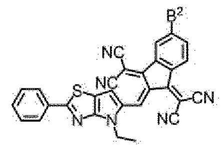

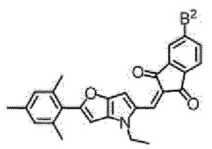 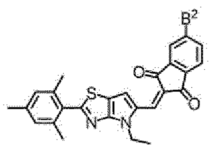 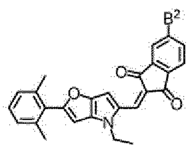 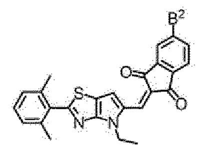

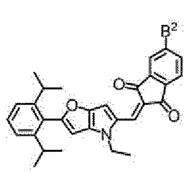 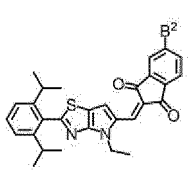 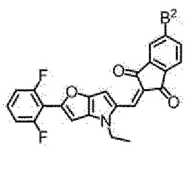 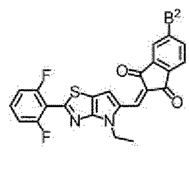

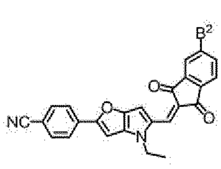 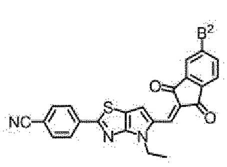 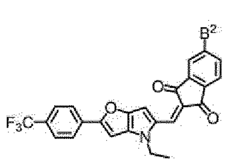 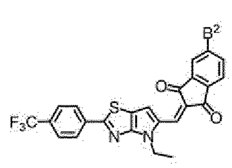

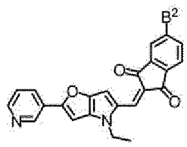 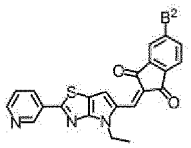 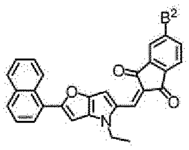 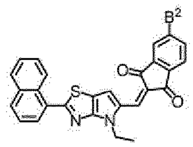

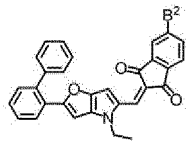 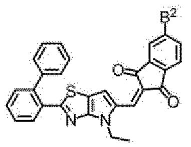 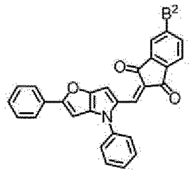 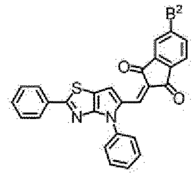

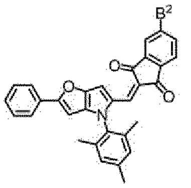 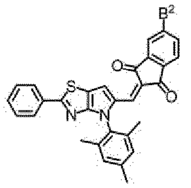 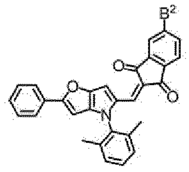 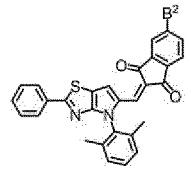

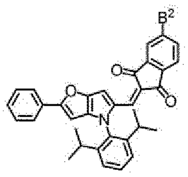 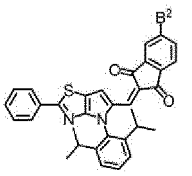 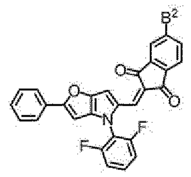 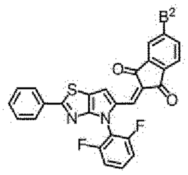

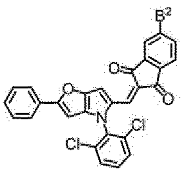 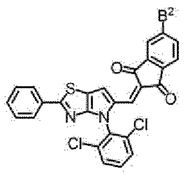 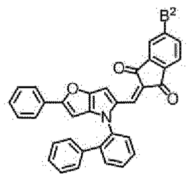 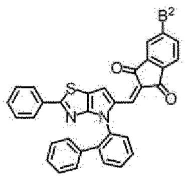

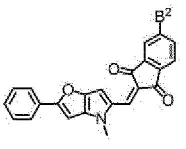 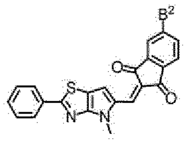 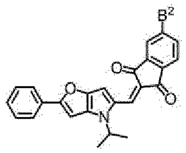 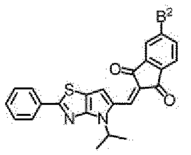

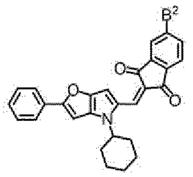 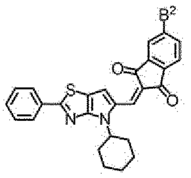 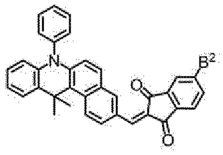 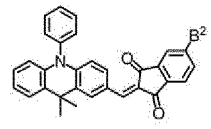

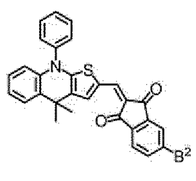 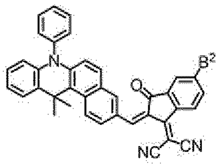 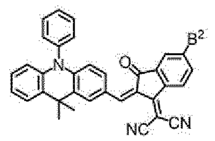 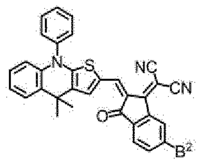

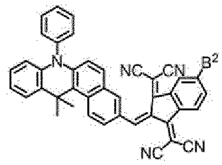
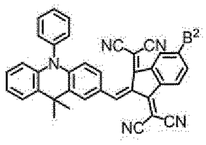
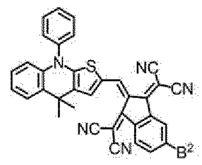
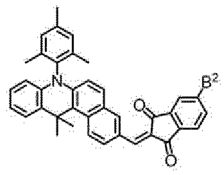
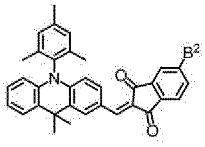
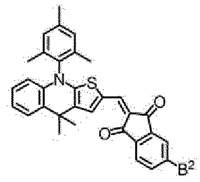
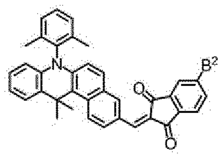
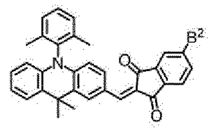
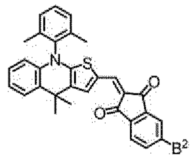
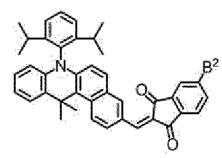
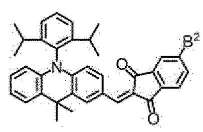
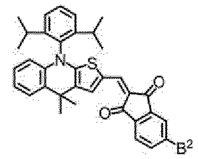
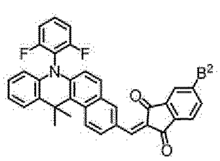
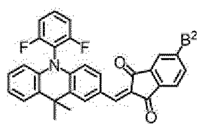
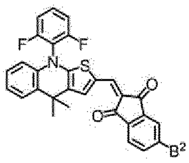
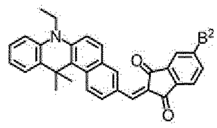
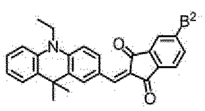
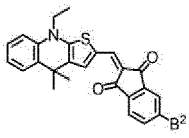
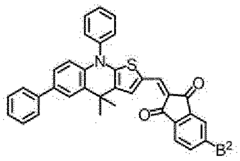
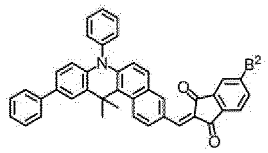
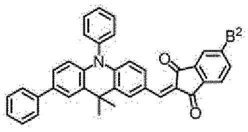
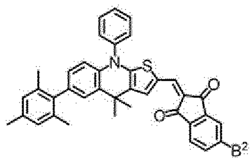
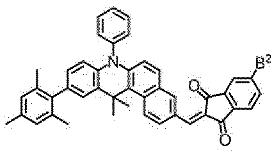
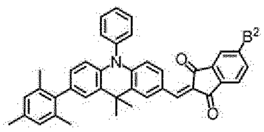
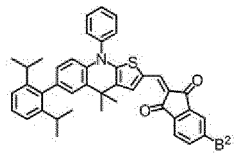
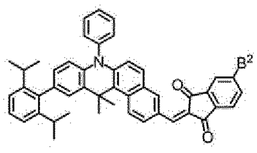
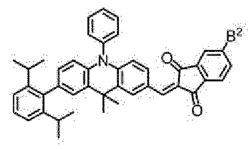
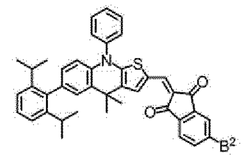
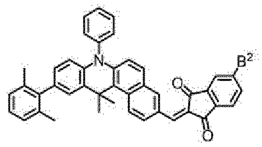
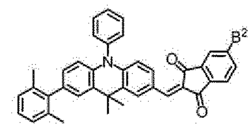
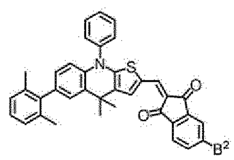
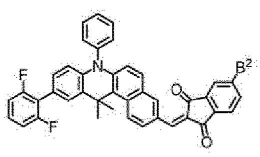

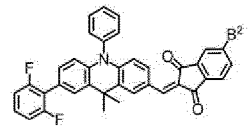
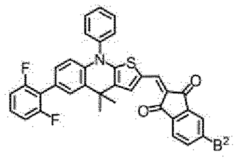
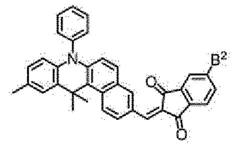
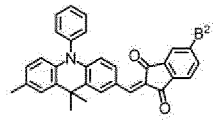
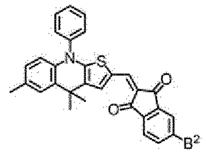
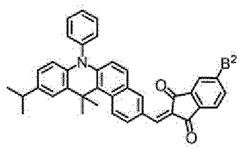
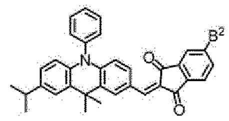
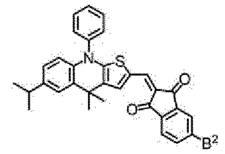
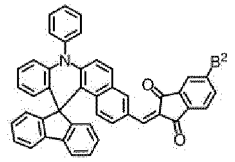
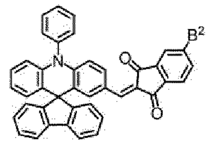
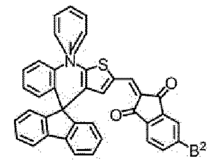
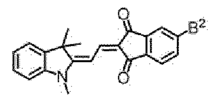
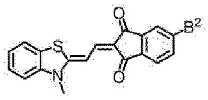
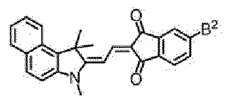
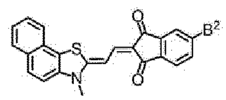
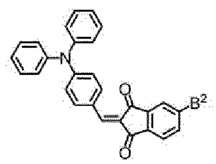
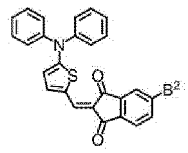
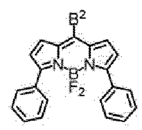

Next, «B residue group 1>> and >>B residue group 2» will be exemplified.

In addition, in a case where each coloring agent residue in «B Residue Group 1>> and <<B Residue Group 2» contains a C=C double bond, the geometric isomer structure that can be distinguished based on this C=C double bond is not limited.

In addition, in each coloring agent residue, * indicates a bonding position.

«B Residue Group 1»

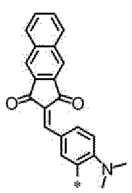
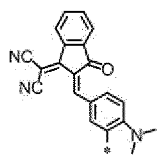
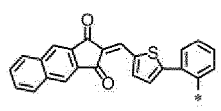
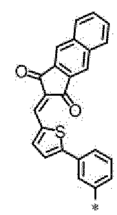
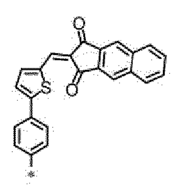
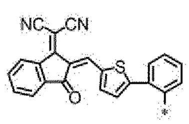
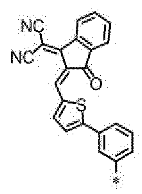
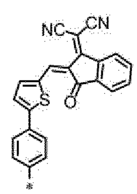

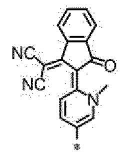 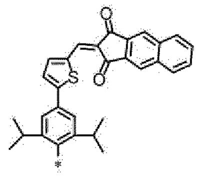 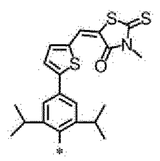 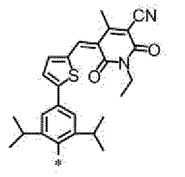

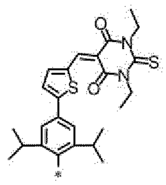 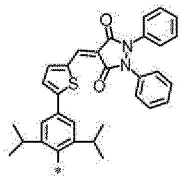 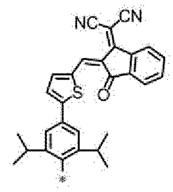 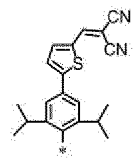

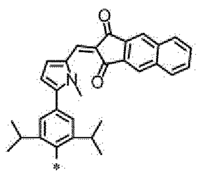 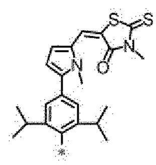 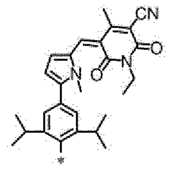 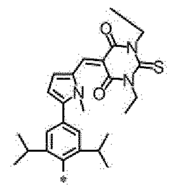

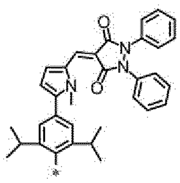 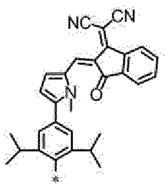 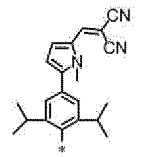 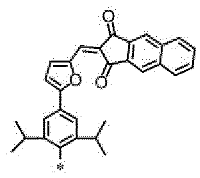

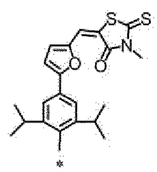 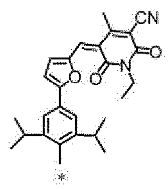 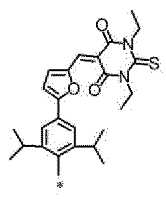 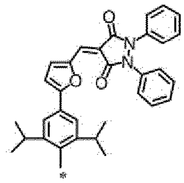

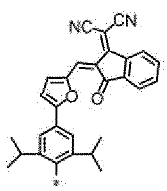 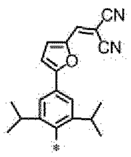 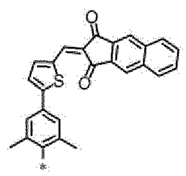 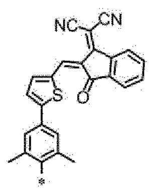

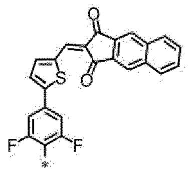 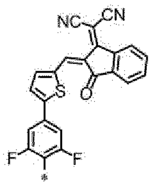 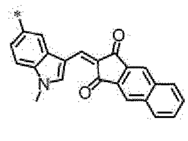 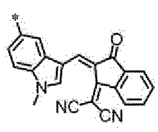

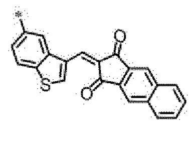 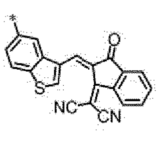 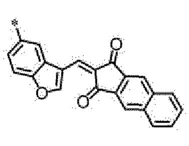 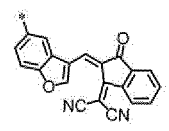

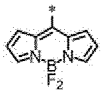 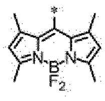 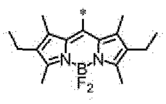 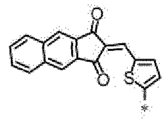

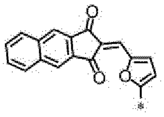 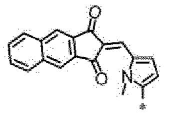 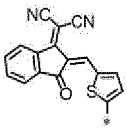 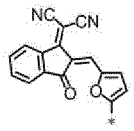

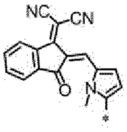 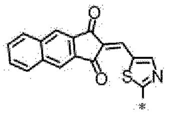 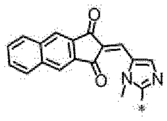 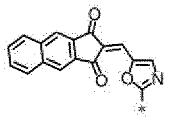

«B Residue Group 2»

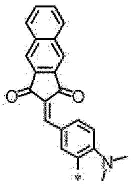 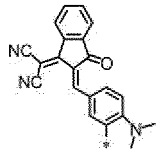 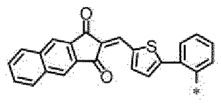 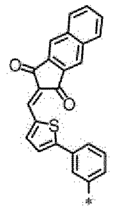

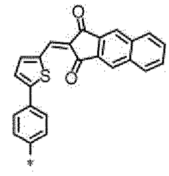 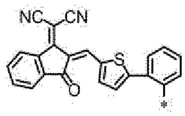 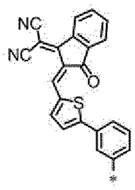 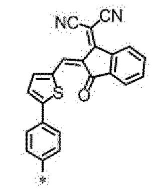

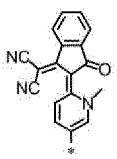 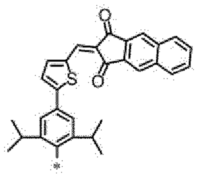 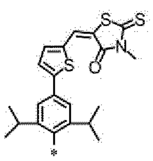 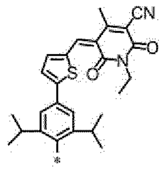

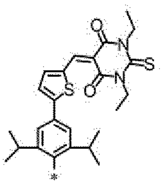 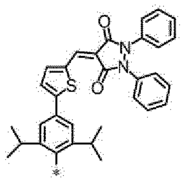 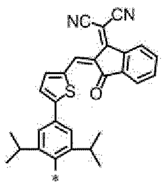 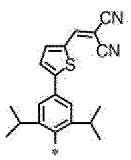

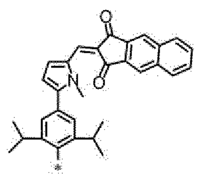 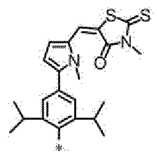 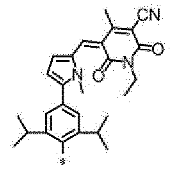 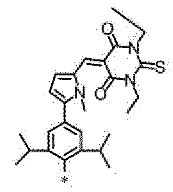

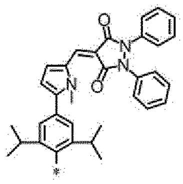 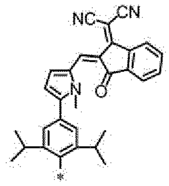 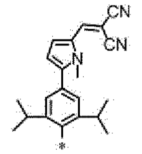 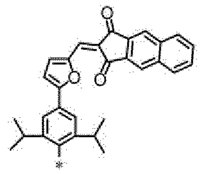

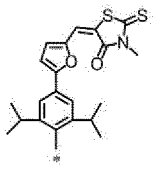 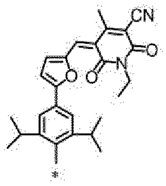 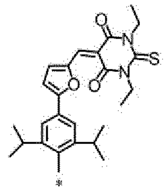 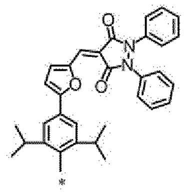

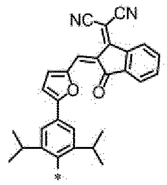 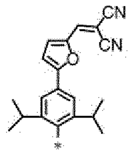 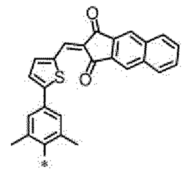 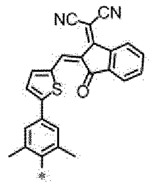

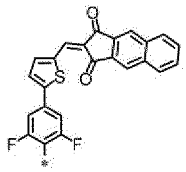 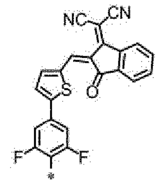 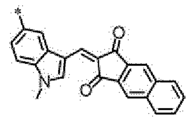 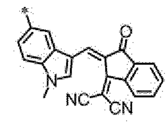

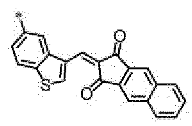 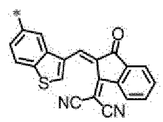 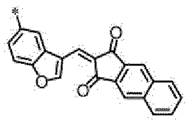 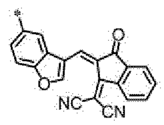

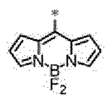 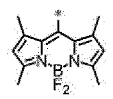 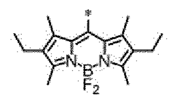 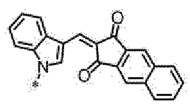

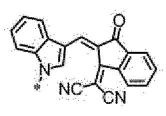

A molecular weight of the specific compound is not particularly limited, but is preferably 300 to 1200, and more preferably 300 to 1000. In a case where the molecular weight is 1200 or less (preferably, 1000 or less), a vapor deposition temperature is not increased, and the compound is not easily decomposed. In a case where the molecular weight is 300 or more, a glass transition point of a vapor deposition film is not lowered, and the heat resistance of the photoelectric

conversion element is improved.

The specific compound is preferably a compound having an ionization potential of 5.2 to 6.2 eV in a single film, more preferably a compound having an ionization potential of 5.2 to 6.1 eV, and still more preferably a compound having an ionization potential of 5.4 to 6.0 eV from the viewpoint of matching the p-type semiconductor material described later with the energy level.

The specific compound contained in the photoelectric conversion film may be used alone, or two or more thereof may be used in combination.

The photoelectric conversion film preferably further contains the n-type semiconductor material described later, or the n-type semiconductor material described later and the p-type semiconductor material described later, in addition to the specific compound described above.

In a case where the photoelectric conversion film includes the n-type semiconductor material described later, a content of the specific compound with respect to a total content of the specific compound and the n-type semiconductor material in the entire photoelectric conversion film (=sum of film thicknesses of specific compounds in terms of single layer/(sum of film thicknesses of specific compounds in terms of single layer + film thickness of n-type semiconductor material in terms of single layer) × 100) is preferably 20% to 80% by volume, and more preferably 40% to 80% by volume, from the viewpoint of responsiveness of the photoelectric conversion element.

In addition, in a case where the photoelectric conversion film includes the n-type semiconductor material described later and the p-type semiconductor material described later, a content of the specific compounds in the entire photoelectric conversion film (= sum of film thicknesses of specific compounds in terms of single layer/(sum of film thicknesses of specific compounds in terms of single layer + film thickness of n-type semiconductor material in terms of single layer + film thickness of p-type semiconductor material in terms of single layer) × 100) is preferably 15% to 75% by volume, and more preferably 25% to 75% by volume, from the viewpoint of responsiveness of the photoelectric conversion element.

It is preferable that the photoelectric conversion film is substantially composed of the specific compound and the n-type semiconductor material, or is substantially composed of the specific compound, the n-type semiconductor material, and the p-type semiconductor material. The term "substantially" is intended that the total content of the specific compound and the n-type semiconductor material is 95% by mass or more with respect to the total mass of the photoelectric conversion film in a case where the photoelectric conversion film is composed of the specific compound and the n-type semiconductor material, and that the total content of the specific compound, the n-type semiconductor material, and the p-type semiconductor material is 95% by mass or more with respect to the total mass of the photoelectric conversion film in a case where the photoelectric conversion film is composed of the specific compound, the n-type semiconductor material, and the p-type semiconductor material.

<n-type Semiconductor Material>

The photoelectric conversion film preferably includes the n-type semiconductor material as another component in addition to the specific compound. The n-type semiconductor material is an acceptor-property organic semiconductor material (a compound), and refers to an organic compound having a property of easily accepting an electron.

Further specifically, the n-type semiconductor material is an organic compound having further excellent electron transport properties than the specific compound. The n-type semiconductor material preferably has a high electron affinity for the specific compound.

In the present specification, the electron transport properties (electron carrier mobility) of a compound can be evaluated by, for example, a time-of-flight method (a TOF method) or by using a field effect transistor element.

The electron carrier mobility of the n-type semiconductor material is preferably $10^{-4}$ $cm^2/V{\cdot}s$ or more, more preferably $10^{-3}$ $cm^2/V{\cdot}s$ or more, and still more preferably $10^{-2}$ $cm^2/V{\cdot}s$ or more. The upper limit of the electron carrier mobility described above is not particularly limited, but is preferably 10 $cm^2/V{\cdot}s$ or less, for example, from the viewpoint of suppressing the flow of a small amount of current without light irradiation.

In the present specification, a value (value multiplied by -1) of a reciprocal number of the LUMO value obtained by the calculation of B3LYP/6-31G (d) using quantum chemical calculation software Gaussian 09 (manufactured by Gaussian, Inc.) as a value of the electron affinity.

The electron affinity of the n-type semiconductor material is preferably 3.0 to 5.0 eV.

Examples of the n-type semiconductor material include fullerenes selected from the group consisting of a fullerene and derivatives thereof, fused aromatic carbocyclic compounds (for example, a naphthalene derivative, an anthracene derivative, a phenanthrene derivative, a tetracene derivative, a pyrene derivative, a perylene derivative, and a fluoranthene derivative); a heterocyclic compound having a 5- to 7-membered ring having at least one of a nitrogen atom, an oxygen atom, or a sulfur atom (for example, pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, and thiazole); polyarylene compounds; fluorene compounds; cyclopentadiene compounds; silyl

compounds; 1,4,5,8-naphthalenetetracarboxylic acid anhydride; 1,4,5,8-naphthalenetetracarboxylic acid anhydride imide derivative; oxadiazole derivative; anthraquinodimethane derivatives; diphenylquinone derivatives; bathocuproine, bathophenanthroline, and derivatives thereof; triazole compounds; a distyrylarylene derivative; a metal complex having a nitrogen-containing heterocyclic compound as a ligand; a silole compound; and compounds disclosed in paragraphs [0056] to [0057] of JP2006-100767A.

Among these, it is preferable that examples of the n-type semiconductor material include fullerenes selected from the group consisting of a fullerene and derivatives thereof.

Examples of the fullerenes include a fullerene C60, a fullerene C70, a fullerene C76, a fullerene C78, a fullerene C80, a fullerene C82, a fullerene C84, a fullerene C90, a fullerene C96, a fullerene C240, a fullerene C540, and a mixed fullerene.

Examples of the fullerene derivatives include compounds in which a substituent is added to the above fullerenes. The substituent is preferably an alkyl group, an aryl group, or a heterocyclic group. The fullerene derivative is preferably compounds described in JP2007-123707A.

In a case where the n-type semiconductor material includes fullerenes, a content of the fullerenes to a total content of the n-type semiconductor materials in the photoelectric conversion film (=(film thickness of fullerenes in terms of single layer/film thickness of total n-type semiconductor material in terms of single layer) × 100) is preferably 15% to 100% by volume, more preferably 35% to 100% by volume.

An organic coloring agent may be used as the n-type semiconductor material in place of the n-type semiconductor material described in the upper row or together with the n-type semiconductor material described in the upper row.

By using an organic coloring agent as the n-type semiconductor material, it is easy to control an absorption wavelength (maximum absorption wavelength) of the photoelectric conversion element to be within any wavelength range.

Examples of the organic coloring agent include a cyanine coloring agent, a styryl coloring agent, a hemicyanine coloring agent, a merocyanine coloring agent (including zeromethine merocyanine (simple merocyanine)), a rhodacyanine coloring agent, an allopolar coloring agent, an oxonol coloring agent, a hemioxonol coloring agent, a squarylium coloring agent, a croconium coloring agent, an azamethine coloring agent, a coumarin coloring agent, an arylidene coloring agent, an anthraquinone coloring agent, a triphenylmethane coloring agent, an azo coloring agent, an azomethine coloring agent, a metallocene coloring agent, a fluorenone coloring agent, a flugide coloring agent, a perylene coloring agent, a phenazine coloring agent, a phenothiazine coloring agent, a quinone coloring agent, a diphenylmethane coloring agent, a polyene coloring agent, an acridine coloring agent, an acridinone coloring agent, a diphenylamine coloring agent, a quinophthalone coloring agent, a phenoxazine coloring agent, a phthaloperylene coloring agent, a dioxane coloring agent, a porphyrin coloring agent, a chlorophyll coloring agent, a phthalocyanine coloring agent, a subphthalocyanine coloring agent, a metal complex coloring agent, compounds disclosed in paragraphs [0083] to [0089] of JP2014-082483A, compounds disclosed in paragraphs [0029] to [0033] of JP2009-167348A, compounds disclosed in paragraphs [0197] to [0227] of JP2012-077064A, compounds disclosed in paragraphs [0035] to [0038] of WO2018-105269A, compounds disclosed in paragraphs [0041] to [0043] of WO2018-186389A, compounds disclosed in paragraphs [0059] to [0062] of WO2018-186397A, compounds disclosed in paragraphs [0078] to [0083] of WO2019-009249A, compounds disclosed in paragraphs [0054] to [0056] of WO2019-049946A, compounds disclosed in paragraphs [0059] to [0063] of WO2019-054327A, and compounds disclosed in paragraphs [0086] to [0087] of WO2019-098161A.

In a case where the n-type semiconductor material includes an organic coloring agent, a content of the organic coloring agent described above to a total content of the n-type semiconductor material in the photoelectric conversion film (=(film thickness of organic coloring agent in terms of single layer/film thickness of total n-type semiconductor material in terms of single layer) × 100) is preferably 15% to 100% by volume, more preferably 35% to 100% by volume.

The molecular weight of the n-type semiconductor material is preferably 200 to 1200, and more preferably 200 to 1000.

It is preferable that the photoelectric conversion film has a bulk hetero structure formed in a state where the specific compound and the n-type semiconductor material are mixed. The bulk hetero structure refers to a layer in which the specific compound and the n-type semiconductor material are mixed and dispersed in the photoelectric conversion film. The photoelectric conversion film having the bulk hetero structure can be formed by either a wet method or a dry method. The bulk hetero structure is described in detail in, for example, paragraphs [0013] and [0014] of JP2005-303266A and the like.

A content of the n-type semiconductor material in the photoelectric conversion film (=(film thickness of n-type semiconductor material in terms of single layer/film thickness of entire photoelectric conversion film) × 100) is preferably 5% to 70% by volume, more preferably 10% to 60% by volume, and still more preferably 15% to 60% by volume.

The n-type semiconductor material contained in the photoelectric conversion film may be used alone, or two or more thereof may be used in combination.

<p-type Semiconductor Material>

The photoelectric conversion film also further preferably includes the p-type semiconductor material as another component other than the specific compound in addition to the specific compound and the n-type semiconductor material. In a case where the specific compound is used as the p-type semiconductor material, the above described p-type semiconductor material is intended to include a p-type semiconductor material other than the specific compound.

The p-type semiconductor material is a donor organic semiconductor material (a compound), and refers to an organic compound having a property of easily donating an electron.

Further specifically, the p-type semiconductor material is an organic compound having more excellent hole transport properties than the specific compound.

In the present specification, the hole transport properties (hole carrier mobility) of a compound can be evaluated by, for example, a time-of-flight method (a TOF method) or by using a field effect transistor element.

The hole carrier mobility of the p-type semiconductor material is preferably $10^{-4}$ $cm^2/V{\cdot}s$ or more, more preferably $10^{-3}$ $cm^2/V{\cdot}s$ or more, and still more preferably $10^{-2}$ $cm^2/V{\cdot}s$ or more. The upper limit of the hole carrier mobility described above is not particularly limited, but is preferably 10 $cm^2/V{\cdot}s$ or less, for example, from the viewpoint of suppressing the flow of a small amount of current without light irradiation.

In addition, the p-type semiconductor material also preferably has a small ionization potential with respect to both the specific compound.

In a case where the photoelectric conversion film includes the p-type semiconductor material, the photoelectric conversion film preferably has a bulk hetero structure formed in a state where the specific compound, the p-type semiconductor material, and the above-described n-type semiconductor material are mixed.

Examples of the p-type semiconductor material include triarylamine compounds (for example, N, N'-bis (3-methylphenyl)-(1,1'-biphenyl)-4,4'-diamine (TPD), 4,4'-bis [N-(naphthyl)-N-Phenyl-amino] biphenyl (α-NPD), compounds disclosed in paragraphs [0128] to [0148] of JP2011-228614A, compounds disclosed in paragraphs [0052] to [0063] of JP2011-176259A, compounds disclosed in paragraphs [0119] to [0158] of JP2011-225544A, compounds disclosed in paragraphs [0044] to [0051] of JP2015-153910A, and compounds disclosed in paragraphs [0086] to [0090] of JP2012-094660A, pyrazoline compounds, styrylamine compounds, hydrazone compounds, polysilane compounds, thiophene compounds (for example, a thienothiophene derivative, a dibenzothiophene derivative, a benzodithiophene derivative, a dithienothiophene derivative, a [1] benzothieno [3,2-b] thiophene (BTBT) derivative, a thieno [3,2-f: 4,5-f] bis [1] benzothiophene (TBBT) derivative, compounds disclosed in paragraphs [0031] to [0036] of JP2018-014474A, compounds disclosed in paragraphs [0043] to [0045] of WO2016-194630A, compounds disclosed in paragraphs [0025] to [0037], and [0099] to [0109] of WO2017-159684A, compounds disclosed in paragraphs [0029] to [0034] of JP2017-076766A, compounds disclosed in paragraphs [0015] to [0025] of WO2018-207722A, compounds disclosed in paragraphs [0045] to [0053] of JP2019-054228A, compounds disclosed in paragraphs [0045] to [0055] of WO2019-058995A, compounds disclosed in paragraphs [0063] to [0089] of WO2019-081416A, compounds disclosed in paragraphs [0033] to [0036] of JP2019-080052A, compounds disclosed in paragraphs [0044] to [0054] of WO2019-054125A, compounds disclosed in paragraphs [0041] to [0046] of WO2019-093188A, and the like), a cyanine compound, an oxonol compound, a polyamine compound, an indole compound, a pyrrole compound, a pyrazole compound, a polyarylene compound, a fused aromatic carbocyclic compound (for example, a naphthalene derivative, an anthracene derivative, a phenanthrene derivative, a tetracene derivative, a pentacene derivative, a pyrene derivative, a perylene derivative, and a fluoranthene derivative), a porphyrin compound, a phthalocyanine compound, a triazole compound, an oxadiazole compound, an imidazole compound, a polyarylalkane compound, a pyrazolone compound, an amino-substituted chalcone compound, an oxazole compound, a fluorenone compound, a silazane compound, and a metal complex having nitrogen-containing heterocyclic compounds as ligands.

The p-type semiconductor material is preferably a compound represented by Formula (p1), a compound represented by Formula (p2), a compound represented by Formula (p3), and a compound represented by Formula (p4), or is also preferably a compound represented by Formula (p5).

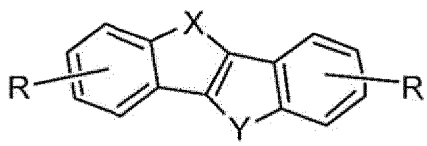
(p1)

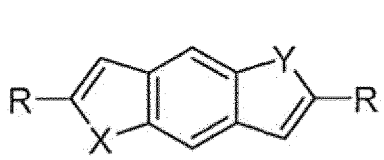
(p2)

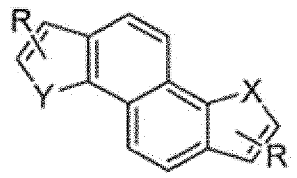

(p3)

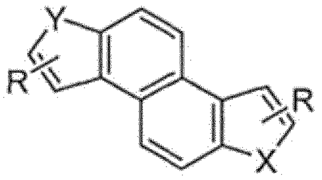

(p4)

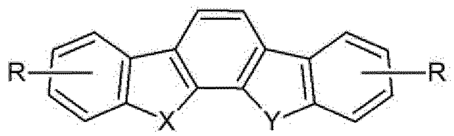

(p5)

Two R's present in Formulae (p1) to (p5) each independently represent a hydrogen atom or a substituent. Examples of the substituent represented by R include an alkyl group, an alkoxy group, a halogen atom, an alkylthio group, a (hetero)arylthio group, an alkylamino group, a (hetero)arylamino group, and a (hetero)aryl group. These groups each may further have a substituent as much as possible. For example, the (hetero)aryl group may be an arylaryl group (that is, a biaryl group; at least one of aryl groups constituting this group may be a heteroaryl group), which may further have a substituent.

As substituents represented by R, groups represented by R in Formula (IX) of WO2019-081416A are also preferable.

X and Y each independently represent $-CR^2{}_2-$, a sulfur atom, an oxygen atom, $-NR^2-$, or $-SiR^2{}_2-$.

$R^2$ represents a hydrogen atom, an alkyl group (preferably a methyl group or a trifluoromethyl group), an aryl group, or a heteroaryl group, which may have a substituent. Two or more $R^2$'s may be the same or different from each other.

The compounds that can be used as the p-type semiconductor materials are exemplified below.

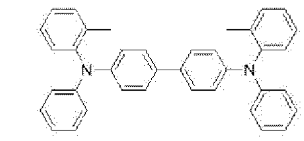

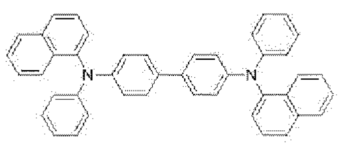

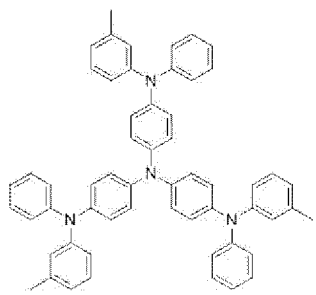

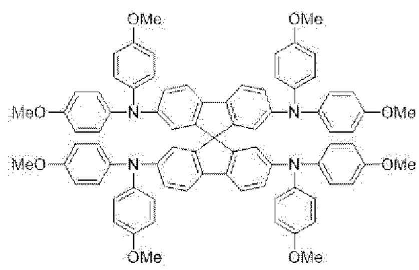

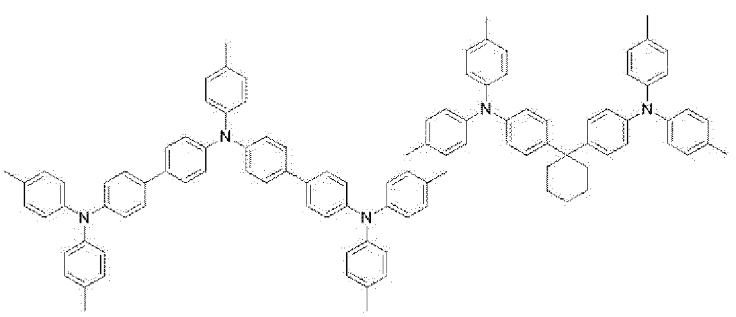

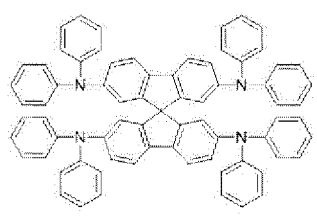

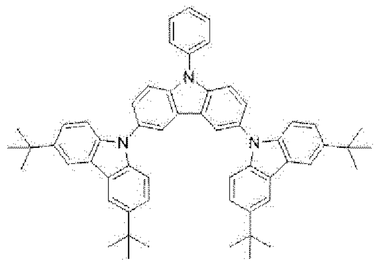
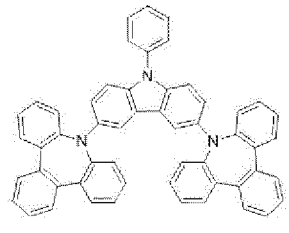
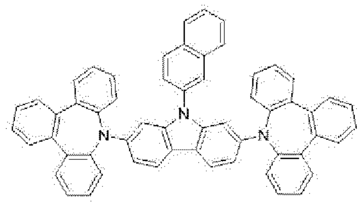
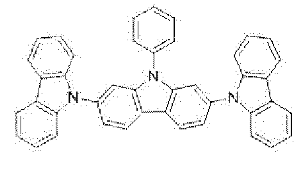
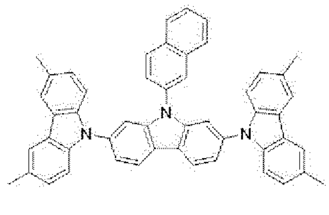
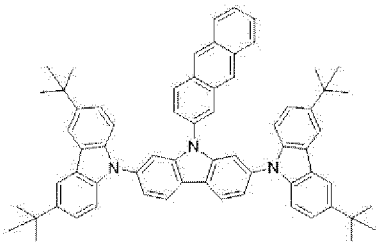
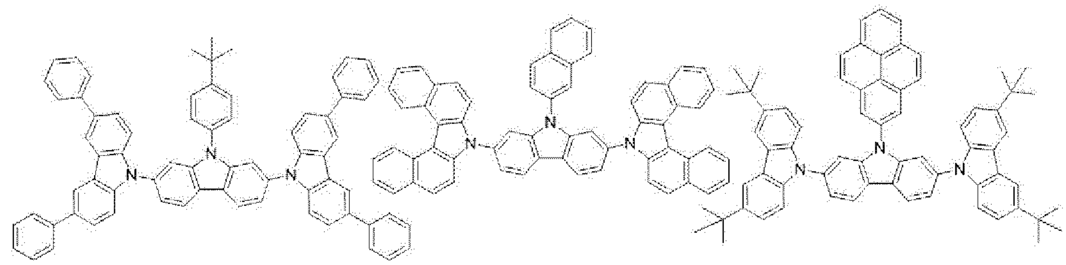
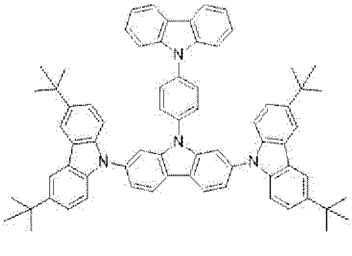
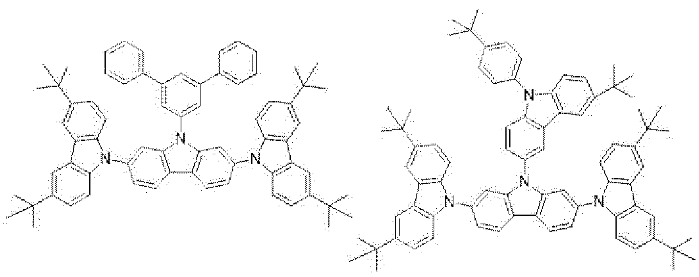
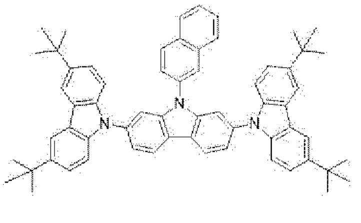
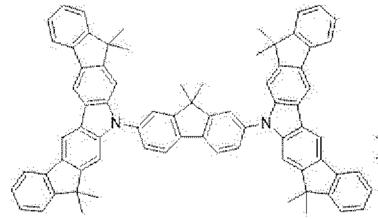
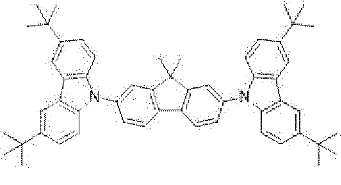
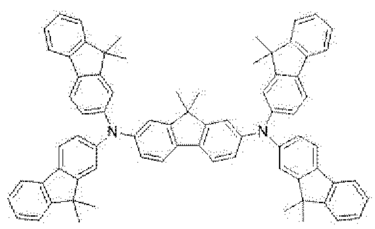
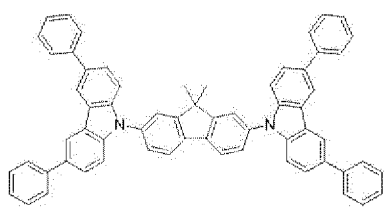
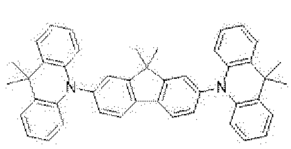

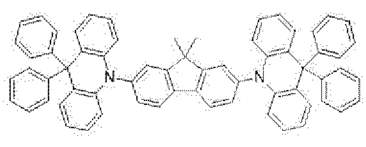
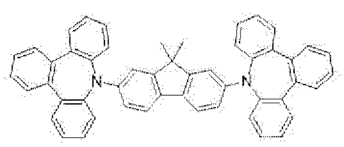
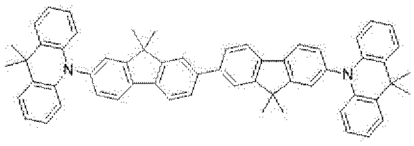

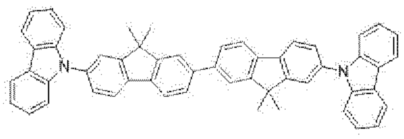
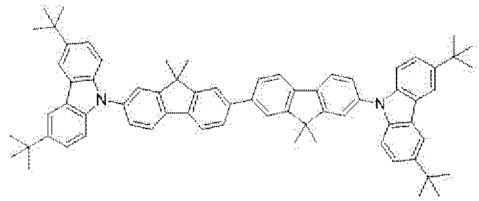

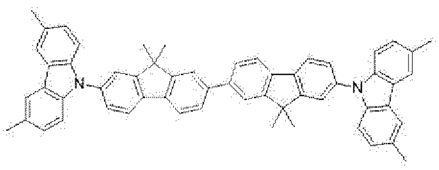
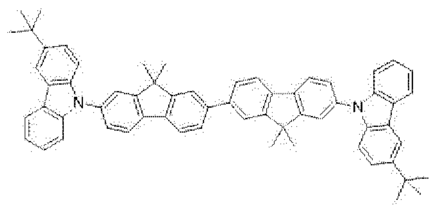

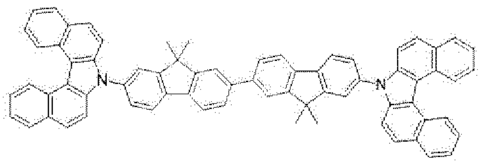
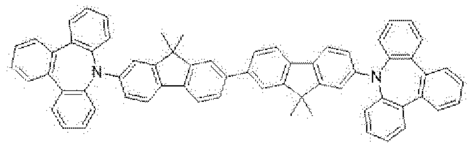

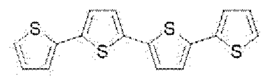
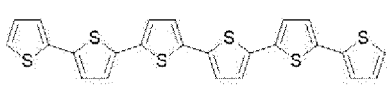
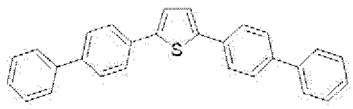

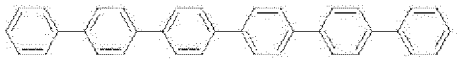
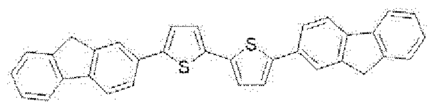

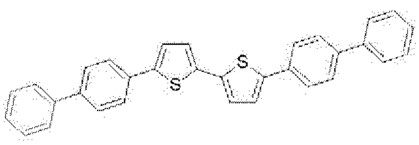
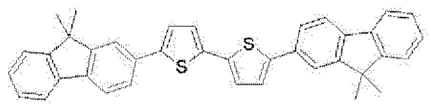

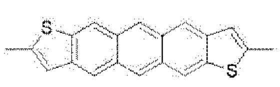
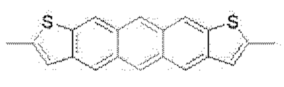
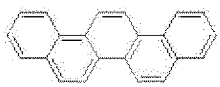
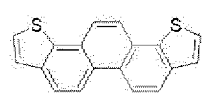

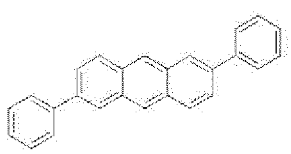
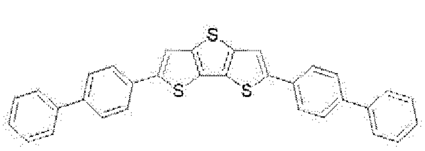
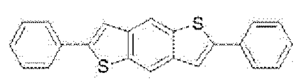

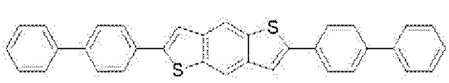
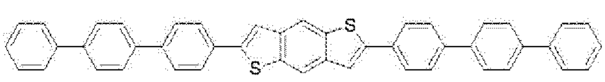

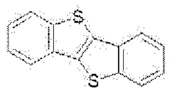

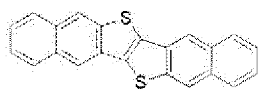

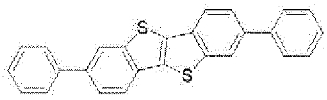

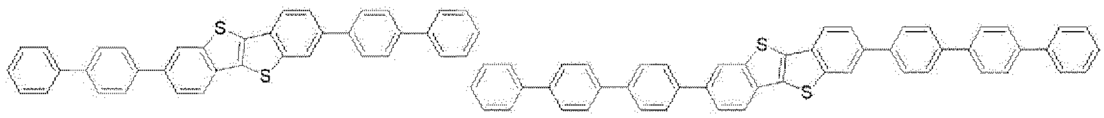

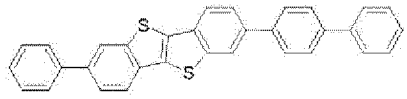

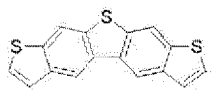

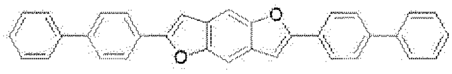

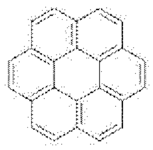

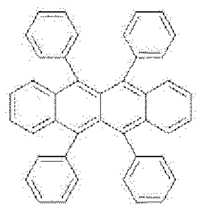

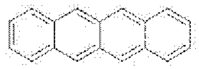

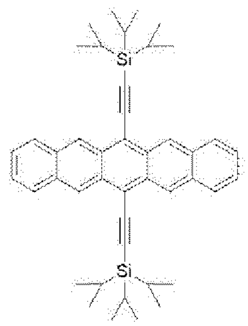

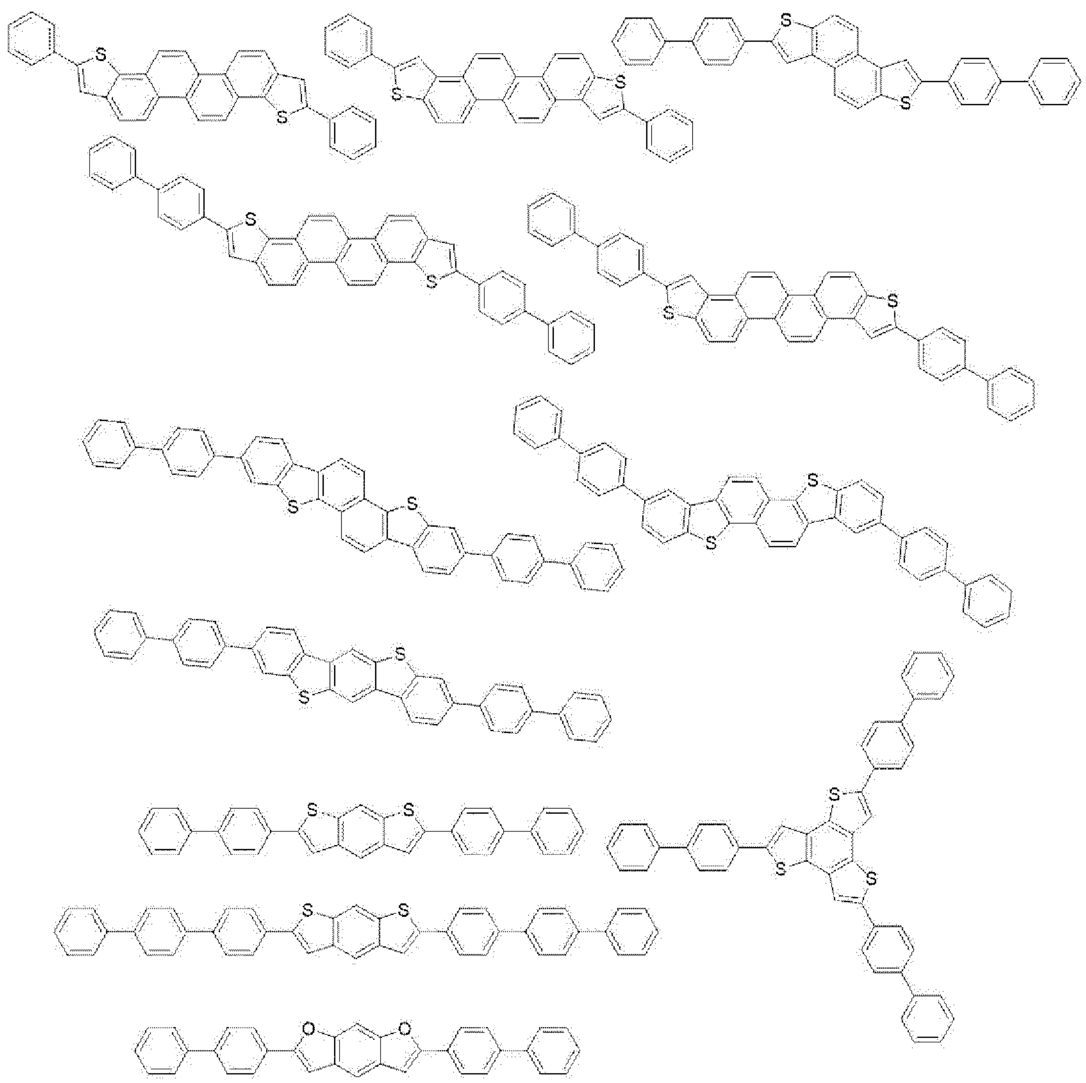

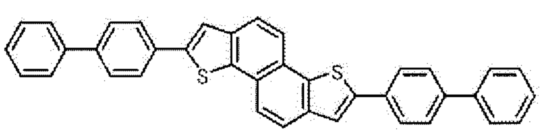

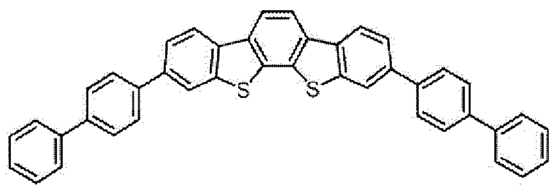

A content of the p-type semiconductor material in the photoelectric conversion film (=(film thickness of p-type semiconductor material in terms of single layer/film thickness of entire photoelectric conversion film) × 100) is preferably 5% to 70% by volume, more preferably 10% to 50% by volume, and still more preferably 15% to 40% by volume.

The p-type semiconductor material contained in the photoelectric conversion film may be used alone, or two or more thereof may be used in combination.

The photoelectric conversion film according to the embodiment of the present invention is a non-light emitting film, and has a feature different from an organic light emitting diode (OLED). The non-light emitting film is intended for a film having a light emission quantum efficiency of 1% or less, and the light emission quantum efficiency is preferably 0.5% or less, and more preferably 0.1% or less.

<Film Formation Method>

The photoelectric conversion film can be formed mostly by a coating film formation method and a dry film formation method.

Examples of the coating film formation method include a coating methods such as a drop casting method, a

casting method, a dip coating method, a die coater method, a roll coater method, a bar coater method, and a spin coating method, various printing methods such as an ink jet method, a screen printing method, a gravure printing method, a flexographic printing method, an offset printing method, and a microcontact printing method, and a Langmuir-Blodgett (LB) method.

Examples of the dry film formation method include a physical vapor deposition method such as a vapor deposition method (in particular, a vacuum vapor deposition method), a sputtering method, and an ion plating method, a molecular beam epitaxy (MBE) method, and a chemical vapor deposition (CVD) method such as plasma polymerization.

Among these, the dry film formation method is preferable, and the vacuum vapor deposition method is more preferable. In a case where the photoelectric conversion film is formed by the vacuum vapor deposition method, manufacturing conditions such as a degree of vacuum and a vapor deposition temperature can be set according to the normal method.

The thickness of the photoelectric conversion film is preferably 10 to 1000 nm, more preferably 50 to 800 nm, and still more preferably 50 to 500 nm.

<Electrode>

Electrodes (the upper electrode (the transparent conductive film) 15 and the lower electrode (the conductive film) 11) are formed of conductive materials. Examples of the conductive material include metals, alloys, metal oxides, electrically conductive compounds, and mixtures thereof.

Since light is incident through the upper electrode 15, the upper electrode 15 is preferably transparent to light to be detected. Examples of the materials constituting the upper electrode 15 include conductive metal oxides such as tin oxide (antimony tin oxide (ATO), fluorine doped tin oxide (FTO)) doped with antimony, fluorine, or the like, tin oxide, zinc oxide, indium oxide, indium tin oxide (ITO), and indium zinc oxide (IZO); metal thin films such as gold, silver, chromium, and nickel; mixtures or laminates of these metals and the conductive metal oxides; and organic conductive materials such as polyaniline, polythiophene, and polypyrrole. Among these, conductive metal oxides are preferable from the viewpoints of high conductivity, transparency, and the like.

In general, in a case where the conductive film is made to be thinner than a certain range, a resistance value is rapidly increased. However, in the solid-state imaging element into which the photoelectric conversion element according to the present embodiment is incorporated, the sheet resistance is preferably 100 to 10000 Ω/□, and a degree of freedom of a range of the film thickness that can be thinned is large. In addition, as the thickness of the upper electrode (the transparent conductive film) 15 is thinner, the amount of light that the upper electrode absorbs is smaller, and the light transmittance usually increases. The increase in the light transmittance causes an increase in light absorbance in the photoelectric conversion film and an increase in the photoelectric conversion ability, which is preferable. Considering the suppression of leakage current, an increase in the resistance value of the thin film, and an increase in transmittance accompanied by the thinning, the film thickness of the upper electrode 15 is preferably 5 to 100 nm, and more preferably 5 to 20 nm.

There is a case where the lower electrode 11 has transparency or an opposite case where the lower electrode 11 does not have transparency and reflects light, depending on the application. Examples of a material constituting the lower electrode 11 include conductive metal oxides such as tin oxide (ATO, FTO) doped with antimony, fluorine, or the like, tin oxide, zinc oxide, indium oxide, indium tin oxide (ITO), and indium zinc oxide (IZO); metals such as gold, silver, chromium, nickel, titanium, tungsten, and aluminum, conductive compounds (for example, titanium nitride (TiN)) such as oxides or nitrides of these metals; mixtures or laminates of these metals and conductive metal oxides; and organic conductive materials such as polyaniline, polythiophene, and polypyrrole.

The method of forming electrodes is not particularly limited, and can be appropriately selected in accordance with the electrode material. Specific examples thereof include a wet method such as a printing method and a coating method; a physical method such as a vacuum vapor deposition method, a sputtering method, and an ion plating method; and a chemical method such as a CVD method and a plasma CVD method.

In a case where the material of the electrode is ITO, examples thereof include an electron beam method, a sputtering method, a resistance heating vapor deposition method, a chemical reaction method (such as a sol-gel method), and a coating method with a dispersion of indium tin oxide.

<Charge Blocking Film: Electron Blocking Film and Positive Hole Blocking Film>

It is also preferable that the photoelectric conversion element according to the embodiment of the present invention has one or more interlayers between the conductive film and the transparent conductive film, in addition to the photoelectric conversion film. An example of the interlayer includes a charge blocking film. In a case where the photoelectric conversion element has this film, the characteristics (such as photoelectric conversion efficiency and responsiveness) of the obtained photoelectric conversion element are more excellent. Examples of the charge blocking film

include an electron blocking film and a positive hole blocking film. The photoelectric conversion element preferably includes at least an electron blocking film as an interlayer.

Hereinafter, each of the films will be described in detail.

(Electron Blocking Film)

The electron blocking film is a donor organic semiconductor material (compound).

The electron blocking film preferably has an ionization potential of 4.8 to 5.8 eV.

An ionization potential Ip(B) of the electron blocking film, an ionization potential Ip (1) of the first compound, and an ionization potential Ip (2) of the second compound preferably satisfy a relationship of Ip(B) < Ip (1) and Ip(B) < Ip (2).

As the electron blocking film, for example, a p-type semiconductor material can be used. The p-type semiconductor material may be used alone, or two or more thereof may be used in combination.

Examples of the p-type semiconductor material include a p-type organic semiconductor material, and specific examples thereof include triarylamine compounds (for example, N,N'-bis(3-methylphenyl)-(1,1'-biphenyl)-4,4'-diamine (TPD), 4,4'-bis[N-(naphthyl)-N-Phenyl-amino] biphenyl (α-NPD), compounds disclosed in paragraphs [0128] to [0148] of JP2011-228614A, compounds disclosed in paragraphs [0052] to [0063] of JP2011-176259A, compounds disclosed in paragraphs [0119] to [0158] of JP2011-225544A, compounds disclosed in paragraphs [0044] to [0051] of JP2015-153910A, and compounds disclosed in paragraphs [0086] to [0090] of JP2012-094660A, pyrazoline compounds, styrylamine compounds, hydrazone compounds, polysilane compounds, thiophene compounds (for example, a thienothiophene derivative, a dibenzothiophene derivative, a benzodithiophene derivative, a dithienothiophene derivative, a [1]benzothieno[3,2-b]thiophene(BTBT)derivative, a thieno[3,2-f:4,5-f']bis[1]benzothiophene (TBBT) derivative, compounds disclosed in paragraphs [0031] to [0036] of JP2018-014474A, compounds disclosed in paragraphs [0043] to [0045] of WO2016-194630A, compounds disclosed in paragraphs [0025] to [0037], and [0099] to [0109] of WO2017-159684A, compounds disclosed in paragraphs [0029] to [0034] of JP2017-076766A, compounds disclosed in paragraphs [0015] to [0025] of WO2018-207722A, compounds disclosed in paragraphs [0045] to [0053] of JP2019-054228A, compounds disclosed in paragraphs [0045] to [0055] of WO2019-058995A, compounds disclosed in paragraphs [0063] to [0089] of WO2019-081416A, compounds disclosed in paragraphs [0033] to [0036] of JP2019-80052A, compounds disclosed in paragraphs [0044] to [0054] of WO2019-054125A, compounds disclosed in paragraphs [0041] to [0046] of WO2019-093188A, and the like), a cyanine compound, an oxonol compound, a polyamine compound, an indole compound, a pyrrole compound, a pyrazole compound, a polyarylene compound, a fused aromatic carbocyclic compound (for example, a naphthalene derivative, an anthracene derivative, a phenanthrene derivative, a tetracene derivative, a pentacene derivative, a pyrene derivative, a perylene derivative, and a fluoranthene derivative), a porphyrin compound, a phthalocyanine compound, a triazole compound, an oxadiazole compound, an imidazole compound, a polyarylalkane compound, a pyrazolone compound, an amino-substituted chalcone compound, an oxazole compound, a fluorenone compound, a silazane compound, and a metal complex having nitrogen-containing heterocyclic compounds as ligands.

Examples of the p-type semiconductor material include compounds having an ionization potential smaller than that of the n-type semiconductor material, and in a case where this condition is satisfied, the organic coloring agents exemplified as the n-type semiconductor material can be used.

A polymer material can also be used as the electron blocking film.

Examples of the polymer material include a polymer such as phenylenevinylene, fluorene, carbazole, indole, pyrene, pyrrole, picoline, thiophene, acetylene, and diacetylene, and a derivative thereof.

The electron blocking film may be formed of a plurality of films.

The electron blocking film may be formed of an inorganic material. In general, since an inorganic material has a dielectric constant larger than that of an organic material, in a case where the inorganic material is used in the electron blocking film, a large voltage is applied to the photoelectric conversion film. Therefore, the photoelectric conversion efficiency increases. Examples of the inorganic material that can be used for the electron blocking film include calcium oxide, chromium oxide, copper chromium oxide, manganese oxide, cobalt oxide, nickel oxide, copper oxide, copper gallium oxide, copper strontium oxide, niobium oxide, molybdenum oxide, copper indium oxide, silver indium oxide, and indium oxide.

(Positive Hole Blocking Film)

A positive hole blocking film is an acceptor-property organic semiconductor material (compound), and the n-type semiconductor material described above can be used.

The method of manufacturing the charge blocking film is not particularly limited, and examples thereof include a dry film formation method and a wet film formation method. Examples of the dry film formation method include a vapor deposition method and a sputtering method. The vapor deposition method may be any of a physical vapor deposition

(PVD) method and a chemical vapor deposition (CVD) method, and the physical vapor deposition method such as a vacuum vapor deposition method is preferable. Examples of the wet film formation method include an ink jet method, a spray method, a nozzle printing method, a spin coating method, a dip coating method, a casting method, a die coating method, a roll coating method, a bar coating method, and a gravure coating method, and an ink jet method is preferable from the viewpoint of high accuracy patterning.

Each thickness of the charge blocking films (the electron blocking film and the positive hole blocking film) is preferably 3 to 200 nm, more preferably 5 to 100 nm, and still more preferably 5 to 30 nm.

<Substrate>

The photoelectric conversion element may further include a substrate. Types of the substrate to be used are not particularly limited, and examples of the substrate include a semiconductor substrate, a glass substrate, and a plastic substrate.

A position of the substrate is not particularly limited, and in general, the conductive film, the photoelectric conversion film, and the transparent conductive film are laminated on the substrate in this order.

<Sealing Layer>

The photoelectric conversion element may further include a sealing layer. The performance of a photoelectric conversion material may deteriorate noticeably due to the presence of deterioration factors such as water molecules. The deterioration can be prevented by coating and sealing the entirety of the photoelectric conversion film with the sealing layer such as diamond-like carbon (DLC) or ceramics such as metal oxide, or metal nitride, and metal nitride oxide which are dense and into which water molecules do not permeate.

The material of the sealing layer may be selected and the sealing layer may be manufactured according to the description in paragraphs [0210] to [0215] of JP2011-082508A.

In the photoelectric conversion element according to the embodiment of the present invention, the photoelectric conversion film may have a configuration of only one layer or a multilayer structure with two or more layers. In a case where the photoelectric conversion film in the photoelectric conversion element according to the embodiment of the present invention has a multilayer structure with two or more layers, at least one layer may contain the specific compound.

In a case where the photoelectric conversion element according to the embodiment of the present invention is applied to an imaging element and an optical sensor described later, the photoelectric conversion film in the photoelectric conversion element is preferably composed as a laminate including, for example, a layer containing the specific compound and a layer having photosensitivity in the near-infrared region and infrared region. Configurations of photoelectric conversion elements disclosed in JP2019-208026A, JP2018-125850A, JP2018-125848A, and other related arts can apply to such a configuration of the photoelectric conversion element, for example.

[Imaging Element]

An example of the application of the photoelectric conversion element includes an imaging element having a photoelectric conversion element. The imaging element is an element that converts optical information of an image into an electric signal. In general, a plurality of the photoelectric conversion elements are arranged in a matrix on the same plane, and an optical signal is converted into an electric signal in each photoelectric conversion element (pixel) to sequentially output the electric signal to the outside of the imaging element for each pixel. Therefore, each pixel is formed of one or more photoelectric conversion elements and one or more transistors.

The imaging element is mounted on an imaging element such as a digital camera and a digital video camera, an electronic endoscope, and imaging modules such as a cellular phone.

The photoelectric conversion element according to the embodiment of the present invention is also preferably used for an optical sensor including the photoelectric conversion element of the present invention. The photoelectric conversion element may be used alone as the optical sensor, and the photoelectric conversion element may be used as a line sensor in which the photoelectric conversion elements are linearly arranged or as a two-dimensional sensor in which the photoelectric conversion elements are arranged in plane.

[Compound]

The present invention also relates to a compound.

The compound according to an embodiment of the present invention is a compound (specific compound) represented by the above-described Formula (1), and the suitable embodiments thereof are also the same.

The specific compound is particularly useful as a material of the photoelectric conversion film used for the

optical sensor, the imaging element, or a photoelectric cell. In addition, the specific compound usually functions as the p-type organic semiconductor in the photoelectric conversion film in many cases. The specific compound can also be used as a coloring material, a liquid crystal material, an organic semiconductor material, a charge transport material, a pharmaceutical material, and a fluorescent diagnostic material.

## Examples

The present invention will be described in more detail based on Examples below. Materials, used amounts, ratios, treatment contents, treatment procedures, and the like described in the following Examples can be appropriately changed within the range that does not depart from the gist of the present invention. Therefore, the range of the present invention should not be limitatively interpreted by the following Examples.

[Synthesis Examples]

[Synthesis of Compounds (D-1) to (D-16) and Comparative Compounds (R-1) to (R-3)]

<Synthesis of Compound (D-1)>

A compound (D-1) was synthesized according to the following scheme.

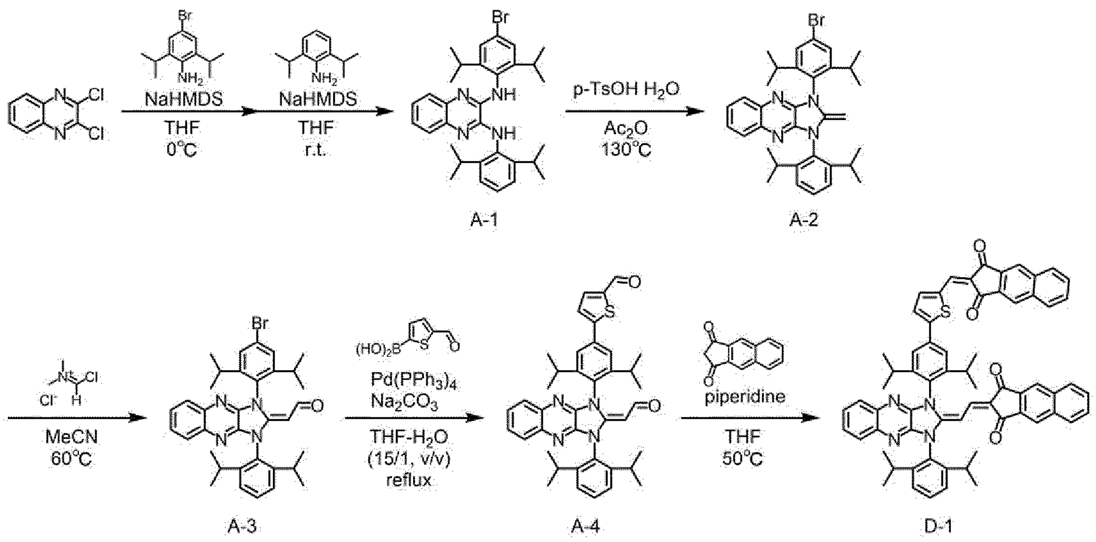


(Synthesis of Compound (A-1))

2,3-dichloroquinoxaline (5.00 g, 25.1 mmol) and 4-bromo-2,6-diisopropylaniline (6.57 g, 25.1 mmol) were dissolved in tetrahydrofuran (33 mL) and stirred at 0°C, and a 1.84 M sodium bis(trimethylsilyl)amide tetrahydrofuran solution (29 mL, 55.3 mmol) was then slowly added thereto, and a reaction solution thus obtained was stirred at room temperature for 30 minutes. After confirming the disappearance of the raw materials, 2,6-diisopropylaniline (4.90 g, 27.6 mmol) was added to the reaction solution, and a 1.84 M sodium bis(trimethylsilyl)amide tetrahydrofuran solution (29 mL, 55.3 mmol) was added slowly thereto, and the mixed solution thus obtained was then stirred at room temperature for 2 hours. Water (25 mL) was slowly added to the reaction solution, saturated saline (50 mL) and ethyl acetate (50 mL) were added, and the mixed solution was stirred at room temperature for 30 minutes. A water layer was removed with a separating funnel, and an organic layer thus obtained was concentrated under reduced pressure. The crude product thus obtained was purified by silica gel chromatography (eluent (volume ratio): hexane/ethyl acetate = 95:5 to hexane/ethyl acetate = 85:15) to obtain a compound (A-1) (14.00 g, yield 99%).

(Synthesis of Compound (A-2))

The compound (A-1) (14.00 g, 25.0 mmol) and p-toluenesulfonic acid monohydrate (14.28 g, 75.1 mmol) were dissolved in acetic anhydride (25 mL), and a reaction solution thus obtained was stirred at 130°C for 4 hours. After being left to cool, the reaction solution was added to an aqueous solution composed of a 50% aqueous sodium hydroxide

solution (70 mL) and ice (210 g), and a mixture thus obtained was stirred at room temperature for 30 minutes to precipitate a solid. The precipitated solid was collected by filtration and washed with water, and thereafter, vacuum dried. A crude product thus obtained was dissolved in tetrahydrofuran, and methanol was added thereto to precipitate a solid. The solid was collected by filtration and washed with methanol. The solid thus obtained was vacuum dried to obtain a compound (A-2) (12.3 g, yield 84%).

(Synthesis of Compound (A-3))

The compound (A-2) (10.00 g, 17.1 mmol) and a Vilsmeier reagent (6.58 g, 51.4 mmol) were dissolved in acetonitrile (100 mL), and a reaction solution thus obtained was stirred at 60°C for 2 hours. After being left to cool, the reaction solution was added to an aqueous solution composed of 1M sodium hydroxide (150 mL) and ice (150 g), and a mixture thus obtained was stirred at room temperature for 30 minutes to precipitate a solid. The precipitated solid was collected by filtration and washed with water, and thereafter, vacuum dried. The crude product thus obtained was dissolved in dichloromethane, and methanol was added thereto to precipitate a solid. The solid was collected by filtration and washed with methanol. The solid thus obtained was vacuum dried to obtain a compound (A-3) (9.10 g, yield 87%).

(Synthesis of Compound (A-4))

The compound (A-3) (6.11 g, 10.0 mmol), 5-formyl-2-thiophene boronic acid (4.70 g, 30.0 mmol), sodium carbonate (3.80 g, 36.0 mmol), and tetrahydrofuran/water (15/1 (v/v), 100 mL) were mixed, and degassed under reduced pressure while stirring at room temperature. After degassing, tetrakis(triphenylphosphine)palladium (0) (0.23 g, 0.20 mmol) was added thereto, and heated and refluxed, and then stirred under nitrogen for 14 hours. After being left to cool, an insoluble matter thus obtained was removed by Celite filtration, and then concentrated under reduced pressure. A crude product thus obtained was purified by silica gel chromatography (eluent (volume ratio): dichloromethane/ethyl acetate = 100:0 to dichloromethane/ethyl acetate = 85:15) to obtain a compound (A-4) (3.53 g, yield 70%).

<Synthesis of Compound (D-1)>

The compound (A-4) (1.61 g, 2.5 mmol), benzindandione (1.28 g, 6.5 mmol), piperidine (3.22 mL), and tetrahydrofuran (16.1 mL) are mixed and stirred at 50°C for 2 hours. After being left to cool, methanol was added to precipitate a solid. The solid was collected by filtration and washed with methanol. A crude product thus obtained was purified by silica gel chromatography (eluent (volume ratio): dichloromethane/ethyl acetate = 100:0 to dichloromethane/ethyl acetate = 85:15) to obtain a compound (D-1) (1.60 g, yield 64%).

<Synthesis of Compounds (D-2) to (D-16)>

Compounds (D-2) to (D-16) described later were synthesized with reference to the synthesis method of the above-described compound (D-1).

<Synthesis of Comparative Compounds (R-1) to (R-3)>

A comparative compound (R-1) described later was synthesized with reference to JP2009-167348A. In addition, a comparative compound (R-2) described later was synthesized with reference to JP2004-211096A. Furthermore, a comparative compound (R-3) described later was synthesized with reference to the synthesis method of the above-described compound (D-1).

Structures of the compounds (D-1) to (D-16) and comparative compounds (R-1) to (R-3) will be specifically described below.

In a case where the structures of the compounds (D-1) to (D-16) described below include a C=C double bond as a partial structure, it is intended that both cis and trans isomers that can be distinguished based on a C=C double bond, which are described above, are included.

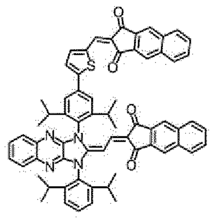
D-1
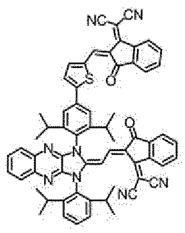
D-2
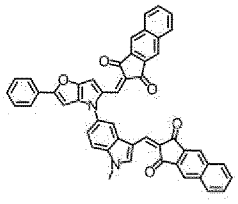
D-3
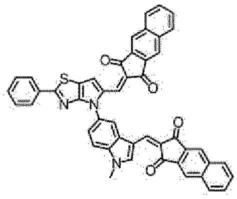
D-4

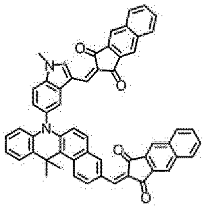
D-5
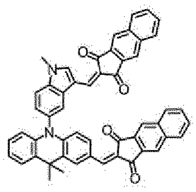
D-6
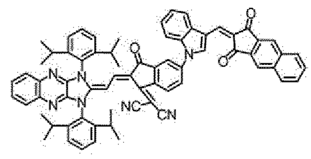
D-7
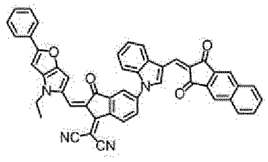
D-8

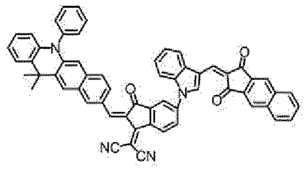
D-9
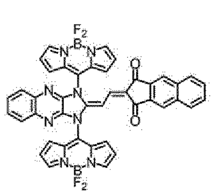
D-10
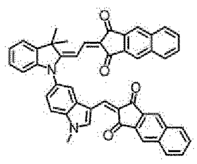
D-11
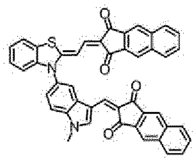
D-12

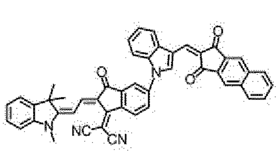
D-13
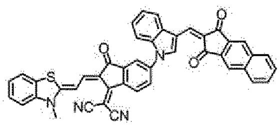
D-14
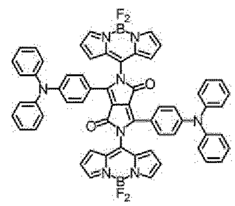
D-15
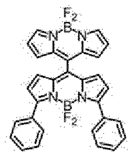
D-16

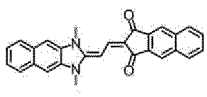
R-1
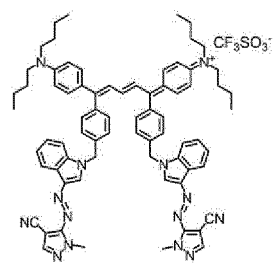
R-2
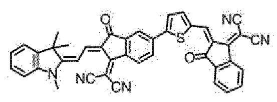
R-3

[Production of Photoelectric Conversion Element]

A photoelectric conversion element was produced by the following procedure.

[Production (vapor deposition Formation) of Photoelectric Conversion Element in Examples 1 to 24 and Comparative Examples 1 to 4]

A photoelectric conversion element having the form of Fig. 1 was produced using the obtained compound. Here, the photoelectric conversion element consists of a lower electrode 11, an electron blocking film 16A, a photoelectric conversion film 12, and an upper electrode 15.

Specifically, an amorphous ITO was formed into a film on a glass substrate by a sputtering method to form the lower electrode 11 (thickness: 30 nm). Furthermore, a compound (EB-1) described later was formed into a film on the lower electrode 11 by a vacuum thermal vapor deposition method to form the electron blocking film 16A (thickness: 30 nm).

Furthermore, in a state where the temperature of the substrate was controlled to 25°C, the above-described compound (D-1), the n-type semiconductor material (fullerene ($C_{60}$)), and the p-type semiconductor material (any compound of compounds (P-1) to (P-4) described later) as desired were subjected to co-vapor deposition by a vacuum vapor deposition method to be 80 nm respectively, in terms of a single layer, on the electron blocking film 16A, thereby being formed into a film. As a result, a photoelectric conversion film 12 having a bulk hetero structure of 160 nm (240 nm in a case where the p-type semiconductor material was also used) was formed.

Furthermore, amorphous ITO was formed into a film on the photoelectric conversion film 12 by a sputtering method to form the upper electrode 15 (the transparent conductive film) (the thickness: 10 nm). After the SiO film was formed as the sealing layer on the upper electrode 15 by a vacuum evaporation method, an aluminum oxide ($Al_2O_3$) layer was formed thereon by an atomic layer chemical vapor deposition (ALCVD) method to produce a photoelectric conversion element.

In addition, photoelectric conversion elements were produced by the same method, except that the compound (D-1) was changed to each of the compounds (D-2) to (D-16) or the comparative compounds (R-1) to (R-3). The same descriptions applied to the compounds (D-2) to (D-16) and the comparative compounds (R-1) to (R-3).

[Production (Coating Formation) of Photoelectric Conversion Element of Example 25]

The photoelectric conversion element was produced by coating using the obtained compound.

The photoelectric conversion element was produced using the obtained compounds. Here, the photoelectric conversion element includes a lower electrode 11, an positive hole blocking film 16B, a photoelectric conversion film 12, an electron blocking film 16A, and an upper electrode 15.

Specifically, an amorphous ITO was formed into a film on a glass substrate by a sputtering method to form the lower electrode 11 (thickness: 30 nm). Furthermore, zinc oxide was spin-coated on the lower electrode and dried at 120°C for 10 minutes to form a positive hole blocking layer. Next, an o-dichlorobenzene solution (concentration: 2.5% by mass) obtained by dissolving the compound (D-1) described above, an n-type semiconductor material ($PC_{60}BM$), and a p-type semiconductor material (P3HT) was spin-coated on the zinc oxide layer to form a photoelectric conversion film having a bulk hetero structure (thickness: about 100 nm). Furthermore, a compound (EB-1) described later was formed into a film by vapor deposition on the photoelectric conversion film to form the electron blocking film 16A (thickness: 10 nm). Furthermore, amorphous ITO was formed into a film on the electron blocking film 16A by a sputtering method to form the upper electrode 15 (the transparent conductive film) (thickness: 10 nm). A SiO film was formed, as a sealing layer, on the upper electrode 15 by a vacuum vapor deposition method, and thereafter, an aluminum oxide ($Al_2O_3$) layer is formed on the SiO film by an ALCVD method to produce a photoelectric conversion element.

[Various Materials]

Various materials used for producing the above-described photoelectric conversion element will be described.

<Material for Forming Electron Blocking Film>

As an electron blocking film forming material, the following compound (EB-1) was used.

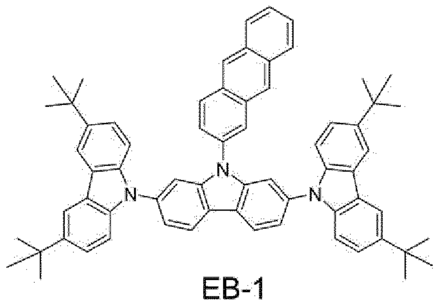

EB-1

<n-type Semiconductor Material>

As the n-type semiconductor material, fullerene ($C_{60}$) (used in Examples 1 to 24 and Comparative Examples 1 to 4) and $PC_{60}BM$ (phenyl-C61-butyric acid methyl ester, used in Example 25) were used.

<p-type Semiconductor Material>

As the p-type semiconductor material, the following compounds (P-1) to (P-4) (used in Examples 17 to 24 and Comparative Example 4) and P3HT (poly (3-hexylthiophen-2,5-diyl), used in Example 25) were used.

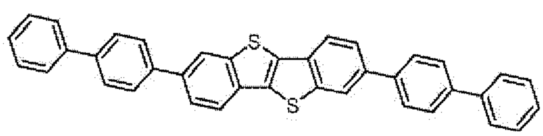

(P-1)

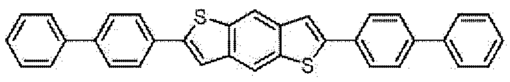

(P-2)

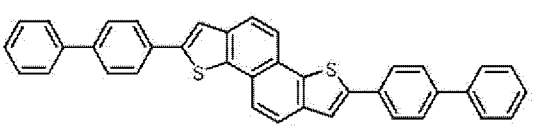

(P-3)

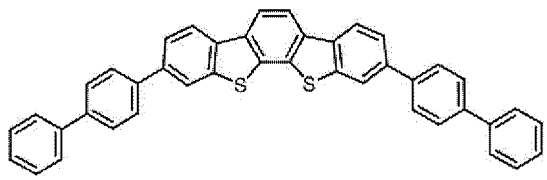

(P-4)

[Various Evaluations]

[Calculation of $\Delta E_A$ and $\Delta E_B$]

$\Delta E_A$ and $\Delta E_B$ of each coloring agent were determined by the method described above. The results are shown in Table 1.

[Calculation of Dihedral Angle a]

A dihedral angle $\alpha$ of each coloring agent was calculated by the method described above. The results are shown in Table 1.

[Evaluation]

[Evaluation of Photoelectric Conversion Efficiency (External Quantum Efficiency)]

The drive of each photoelectric conversion element thus obtained was confirmed. A voltage was applied to each photoelectric conversion element to have an electric field strength of $2.0 \times 10^5$ V/cm. Thereafter, light is emitted from the upper electrode (transparent conductive film) side to perform an incident photon to current conversion efficiency (IPCE) measurement, and the external quantum efficiency (external quantum efficiency before the continuous drive) at each of a wavelength of 450 nm, a wavelength of 580 nm, and a wavelength of 650 nm was extracted. It was confirmed

that all of the photoelectric conversion elements produced by using the compounds (D-1) to (D-16) had a photoelectric conversion efficiency of 50% or more at all wavelengths of 450 nm, 580 nm, and 650 nm, and the photoelectric conversion elements had the sufficient external quantum efficiency. The external quantum efficiency was measured using a constant energy quantum efficiency measuring device manufactured by Optel Co., Ltd.. The amount of light emitted was 50 $\mu W/cm^2$.

In addition, the photoelectric conversion efficiency of the photoelectric conversion element obtained in Comparative Example 1 was standardized at each of wavelengths of 450 nm, 580 nm, and 650 nm to 1 to obtain a relative value of the photoelectric conversion efficiency of each photoelectric conversion element, and the value thus obtained was evaluated according to the standard described below. In terms of practicality, "A", "B", "C", and "D" are preferable evaluation results, and "A", "B", and "C" are more preferable evaluation results.

The photoelectric conversion efficiency at a wavelength of 580 nm satisfied the evaluations of "A", "B", "C", or "D", except for Comparative Example 1.

<Evaluation Standard>

"A": 1.5 or more
"B": 1.4 or more and less than 1.5
"C": 1.2 or more and less than 1.4
"D": 1.1 or more and less than 1.2
"E": less than 1.1

The results are shown in Table 1.

In Table 1, the column of "580 nm" in the column of "External quantum efficiency" indicates a case where the evaluation result of the external quantum efficiency at a wavelength of 580 nm was "A", "B", "C", or "D" as "P" and indicates a case where the evaluation result was "E" as "N".

In Table 1, the column of "Methine coloring agent residue" in the column of "Structure of A" indicates whether or not the structure of "A" is the aromatic ring-containing methine coloring agent residue in a case where the coloring agent is applied to the compound represented by Formula (1). A case where "A" in the coloring agent corresponds to the aromatic ring-containing methine coloring agent residue is represented by "A", and a case where "A" in the coloring agent does not correspond to the aromatic ring-containing methine coloring agent residue is represented by "B".

In Table 1, the column of "Residues of Formulae (3) to (5)" in the column of "Structure of A" indicates whether or not the structure of "A" corresponds to any of residues derived from the compounds represented by Formulae (3) to (5) described above in a case where the coloring agent is applied to the compound represented by Formula (1). A case where "A" in the coloring agent does not correspond to any of the residues derived from the compounds represented by Formulae (3) to (5) described above is represented by "-".

In Table 1, the column of "Methine coloring agent residue or pyrromethene coloring agent residue" in the column of "Structure of B" indicates whether or not "B" corresponds to the aromatic ring-containing methine coloring agent residue or the pyrromethene coloring agent residue in a case where the coloring agent is applied to the compound represented by Formula (1). A case where "B" in the coloring agent corresponds to the aromatic ring-containing methine coloring agent residue or the pyrromethene coloring agent residue is represented by "A", and a case where "B" in the coloring agent does not correspond to the aromatic ring-containing methine coloring agent residue or the pyrromethene coloring agent residue is represented by "B".

In Table 1, the column of "Residue of Formula (6)" in the column of "Structure of B" indicates whether or not "B" corresponds to a residue derived from the compound represented by Formula (6) described above in a case where the coloring agent is applied to the compound represented by Formula (1). A case where "B" in the coloring agent corresponds to the residue derived from the compound represented by Formula (6) described above is represented by "A", and a case where "B" in the coloring agent does not correspond to the residue derived from the compound is represented by Formula (6) is represented by "B".

In Table 1, the column of "Bonding atoms of first monocyclic structure" indicates whether or not the atom directly bonded to B in the first monocyclic structure in "A" corresponds to a nitrogen atom in a case where the coloring agent is applied to the compound represented by Formula (1). A case where the atom directly bonded to B in the first monocyclic structure in the coloring agent corresponds to a nitrogen atom is represented by "A", and a case where the atom does not correspond to a nitrogen atom is represented by "B".

In Table 1, the column of "Number of p" represents the number of p (1 or 2) in a case where the coloring agent is applied to the compound represented by Formula (1).

[Table 1]

| Table 1 | Coloring agent | p-type semiconductor | n-type semiconductor | ΔE(ev) | | Dihedral angle α (°) | External quantum efficiency | | | Note | | | | | |
|---|---|---|---|---|---|---|---|---|---|---|---|---|---|---|---|
| | | | | | | | | | | Structure of A | | Structure of B | | Bonding atoms of first monocyclic structure | Number of p |
| | | | | $\Delta E_A$ (ev) | $\Delta E_B$ (ev) | | 450nm | 580nm | 650nm | Methine coloring agent residue | Residues of Formulae (3) to (5) | Methine coloring agent residue or pyrromethene coloring agent residue | Residue of Formula (6) | | |
| Example 1 | D-1 | - | C60 | 2.89 | 3.11 | 85 | B | P | B | A | (3) | A | A | A | 1 |
| Example 2 | D-2 | - | C60 | 2.74 | 2.83 | 85 | B | P | B | A | (3) | A | A | A | 1 |
| Example 3 | D-3 | - | C60 | 2.77 | 3.31 | 82 | B | P | B | A | (4) | A | A | A | 1 |
| Example 4 | D-4 | - | C60 | 3.11 | 3.31 | 87 | B | P | B | A | (4) | A | A | A | 1 |
| Example 5 | D-5 | - | C60 | 2.58 | 3.31 | 89 | B | P | B | A | (5) | A | A | A | 1 |
| Example 6 | D-6 | - | C60 | 2.87 | 3.31 | 89 | B | P | B | A | (5) | A | A | A | 1 |
| Example 7 | D-7 | - | C60 | 2.66 | 3.35 | 43 | C | P | B | A | (3) | A | A | B | 1 |
| Example 8 | D-8 | - | C60 | 2.55 | 3.35 | 41 | C | P | B | A | (4) | A | A | B | 1 |
| Example 9 | D-9 | - | C60 | 2.28 | 3.35 | 40 | C | P | B | A | (5) | A | A | B | 1 |
| Example 10 | D-10 | | C60 | 2.93 | 3.12 | 75 | C | P | B | A | (3) | A | B | A | 2 |
| Example 11 | D-11 | - | C60 | 2.31 | 3.31 | 82 | B | P | C | A | - | A | A | A | 1 |

(continued)

| Table 1 | Coloring agent | p-type semiconductor | n-type semiconductor | ΔE(ev) | | Dihedral angle α (°) | External quantum efficiency | | | Note | | | | | |
|---|---|---|---|---|---|---|---|---|---|---|---|---|---|---|---|
| | | | | | | | | | | Structure of A | | Structure of B | | Bonding atoms of first monocyclic structure | Number of p |
| | | | | $\Delta E_A$ (ev) | $\Delta E_B$ (ev) | | 450nm | 580nm | 650nm | Methine coloring agent residue | Residues of Formulae (3) to (5) | Methine coloring agent residue or pyrromethene coloring agent residue | Residue of Formula (6) | | |
| Example 12 | D-12 | - | C60 | 3.05 | 3.31 | 86 | B | P | C | A | - | A | A | A | 1 |
| Example 13 | D-13 | - | C60 | 2.82 | 3.35 | 41 | C | P | C | A | - | A | A | B | 1 |
| Example 14 | D-14 | - | C60 | 2.79 | 3.35 | 42 | C | P | C | A | - | A | A | B | 1 |
| Example 15 | D-15 | - | C60 | 2.42 | 3.12 | 53 | C | P | D | B | - | A | B | A | 2 |
| Example 16 | D-16 | - | C60 | 2.66 | 3.12 | 71 | D | P | D | B | - | A | B | B | 2 |

[Table 2]

| Table 1 continued | Coloring agent | p-type semiconductor | n-type semiconductor | AE(ev) | | Dihedral angle $\alpha$ (°) | External quantum efficiency | | | Note | | | | | |
|---|---|---|---|---|---|---|---|---|---|---|---|---|---|---|---|
| | | | | | | | | | | Structure of A | | Structure of B | | | |
| | | | | $\Delta E_A$ (ev) | $\Delta E_B$ (ev) | | 450nm | 580nm | 650nm | Methine coloring agent residue | Residues of Formulae (3) to (5) | Methine coloring agent residue or pyrromethene coloring agent residue | Residue of Formula (6) | Bonding atoms of first monocyclic structure | Number of p |
| Example 17 | D-1 | P-1 | C60 | 2.89 | 3.11 | 85 | A | P | A | A | (3) | A | A | A | 1 |
| Example 18 | D-2 | P-1 | C60 | 2.74 | 2.98 | 68 | A | P | A | A | (3) | A | A | A | 1 |
| Example 19 | D-1 | P-2 | C60 | 2.89 | 3.11 | 85 | A | P | A | A | (3) | A | A | A | I |
| Example 20 | D-2 | P-2 | C60 | 2.74 | 2.98 | 68 | A | P | A | A | (3) | A | A | A | 1 |
| Example 21 | D-1 | P-3 | C60 | 2.89 | 3.11 | 85 | A | P | A | A | (3) | A | A | A | 1 |
| Example 22 | D-2 | P-3 | C60 | 2.74 | 2.98 | 68 | A | P | A | A | (3) | A | A | A | 1 |
| Example 23 | D-1 | P-4 | C60 | 2.89 | 3.11 | 85 | A | P | A | A | (3) | A | A | A | 1 |
| Example 24 | D-2 | P-4 | C60 | 2.74 | 2.98 | 68 | A | P | A | A | (3) | A | A | A | 1 |
| Example 25 | D-1 | P3HT | PC60BM | 2.89 | 3.11 | 85 | A | P | B | A | (3) | A | A | A | 1 |
| Comparative Example 1 | R-1 | - | C60 | - | - | - | E | N | E | - | - | - | - | - | - |
| Comparative Example 2 | R-2 | - | C60 | - | - | - | cannot be vapor deposited | | | - | - | - | - | - | - |
| Comparative Example 3 | R3 | - | C60 | 2.82 | 3.21 | 22 | cannot be vapor deposited | | | - | - | - | - | - | - |
| Comparative Example 4 | R-1 | P-1 | C60 | - | - | - | E | P | D | - | - | - | - | - | - |

From the results in Table 1, it was confirmed that the photoelectric conversion film using the specific compound has exhibited excellent vapor deposition suitability. In addition, it was confirmed that the photoelectric conversion elements obtained in Examples exhibit the excellent external quantum efficiency to light at all wavelengths in the red wavelength range, the green wavelength range, and the blue wavelength range.

In comparisons of Examples 11 to 16, it was confirmed that the external quantum efficiency was more excellent in a case where "A" in the specific compound was the methine coloring agent residue and "B" represented the residue derived from the compound represented by Formula (6). Furthermore, it was confirmed that the external quantum efficiency was further excellent in a case where "A" in the specific compound was the methine coloring agent residue, "B" represented the residue derived from the compound represented by Formula (6), and the atom directly bonded to B in the first monocyclic structure was a nitrogen atom.

In addition, in comparisons of Examples 8 to 16, it was confirmed that the external quantum efficiency was further excellent in a case where "A" in the specific compound represented the residue derived from the compound represented by any one of Formulae (3), (4), or (5).

In addition, in comparisons of Examples 1 to 16, it was confirmed that the external quantum efficiency was further excellent in a case where "A" in the specific compound represented the residue derived from the compound represented by any one of Formulae (3), (4), or (5), "B" represented the residue derived from the compound represented by Formula (6), and the atom directly bonded to B in the first monocyclic structure was a nitrogen atom.

In addition, in comparisons of Examples 17 to 24 and Examples 1 and 2, it was confirmed that the external quantum efficiency was further excellent in a case where the photoelectric conversion film further contained the p-type semiconductor.

In addition, in the photoelectric conversion film (Example 25) produced by coating formation, it was confirmed that the excellent external quantum efficiency to light at all wavelengths in the red wavelength range, the green wavelength range, and the blue wavelength range was exhibited.

Comparative Examples 1 and 4 had vapor deposition suitability, and the photoelectric conversion elements could be produced by vapor deposition. However, the requirement of exhibiting the excellent external quantum efficiency to light at all wavelengths in the red wavelength range, the green wavelength range, and the blue wavelength range was not satisfied.

Comparative Examples 2 and 3 did not have vapor deposition suitability, and the photoelectric conversion elements could not be produced by vapor deposition.

Explanation of References

10a, 10b: photoelectric conversion element
11: conductive film (lower electrode)
12: photoelectric conversion film
15: transparent conductive film (upper electrode)
16A: electron blocking film
16B: positive hole blocking film

**Claims**

**1.** A photoelectric conversion element comprising, in the following order:

a conductive film;
a photoelectric conversion film; and
a transparent conductive film,
wherein the photoelectric conversion film contains a compound represented by Formula (1),

$$A\left[B\right]_p \qquad (1)$$

in Formula (1),
A has $\Delta E_A$ of 2.00 to 3.20 eV and represents a first aromatic ring-containing coloring agent residue having a

first monocyclic structure directly bonded to B,
B has $\Delta E_B$ of 2.80 to 4.00 eV and represents a second aromatic ring-containing coloring agent residue having a second monocyclic structure directly bonded to A,
p represents 1 or 2,
where, in Formula (1), a dihedral angle $\alpha$ formed of the first monocyclic structure and the second monocyclic structure defined below is 30° to 90°, the $\Delta E_A$ and the $\Delta E_B$ satisfy a relationship of $\Delta E_A < \Delta E_B$,
$\Delta E_A$: in a case where a structure optimization calculation is performed on a compound UA obtained by replacing B with a hydrogen atom in the compound represented by Formula (1), by density functional calculation B3LYP/6-31G (d) with quantum chemical calculation software Gaussian09 to obtain HOMO energy and LUMO energy, the obtained HOMO energy being represented by $E_{HOMO-A}$ (eV) and the obtained LUMO energy being represented by $E_{LUMO-A}$ (eV), $\Delta E_A$ represents a numeric value (eV) obtained by Formula (EA),

$$\text{Formula (EA)}: \Delta E_A = E_{LUMO-A} - E_{HOMO-A}$$

$\Delta E_B$: in a case where a structure optimization calculation is performed on a compound UB obtained by replacing A with a hydrogen atom in the compound represented by Formula (1), by density functional calculation B3LYP/6-31G (d) with quantum chemical calculation software Gaussian09 to obtain HOMO energy and LUMO energy, the obtained HOMO energy being represented by $E_{HOMO-B}$ (eV) and the obtained LUMO energy being represented by $E_{LUMO-B}$ (eV), $\Delta E_B$ represents a numeric value (eV) obtained by Formula (EB),

$$\text{Formula (EB)}: \Delta E_B = E_{LUMO-B} - E_{HOMO-B}$$

dihedral angle a: in a structure obtained by performing a structure optimization calculation on the compound represented by Formula (1), by density functional calculation B3LYP/6-31G (d) with quantum chemical calculation software Gaussian09, the dihedral angle $\alpha$ is a dihedral angle having a smallest absolute value among four kinds of dihedral angles (X1) to (X4), which are able to be formed with four adjacent atoms represented by a to f in a partial structure corresponding to Formula (1-A) in Formula (1), and

(X1) a-b-e-d

(X2) a-b-e-f

(X3) c-b-e-d

(X4) c-b-e-f

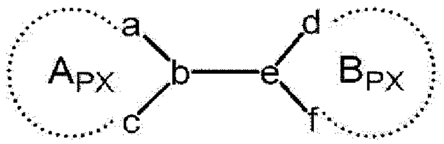

(1-A)

in Formula (1-A),
$A_{PX}$ corresponds to the first monocyclic structure contained in the first aromatic ring-containing coloring agent residue in Formula (1), and at least contains three atoms represented by a, b, and c as ring-constituting atoms,
$B_{PX}$ corresponds to the second monocyclic structure contained in the second aromatic ring-containing coloring agent residue in Formula (1), and at least contains three atoms represented by d, e, and f as ring-constituting atoms, and
in Formula (1-A), all of bonds represented by a-b, c-b, d-e, e-f, and b-e are covalent bonds.

2. The photoelectric conversion element according to claim 1,
wherein in Formula (1), A represents an aromatic ring-containing methine coloring agent residue.

3. The photoelectric conversion element according to claim 1 or 2,
wherein in Formula (1), an atom at a bonding position with B of the first monocyclic structure is a nitrogen atom.

**4.** The photoelectric conversion element according to any one of claims 1 to 3,

wherein in Formula (1), A represents a residue derived from a compound represented by Formula (3), which is formed by removing p hydrogen atom bonded to ring-constituting atoms forming a ring structure in the compound represented by Formula (3), a residue derived from a compound represented by Formula (4), which is formed by removing p hydrogen atom bonded to ring-constituting atoms forming a ring structure in the compound represented by Formula (4), or a residue derived from a compound represented by Formula (5), which is formed by removing p hydrogen atom bonded to ring-constituting atoms forming a ring structure in the compound represented by Formula (5),

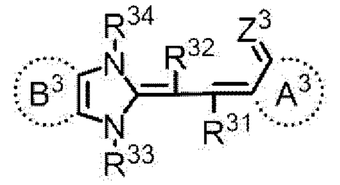
(3)

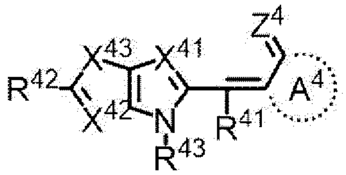
(4)

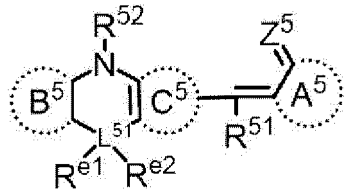
(5)

in Formula (3),
$A^3$ represents a ring which contains at least two carbon atoms and may have a substituent,
$Z^3$ represents an oxygen atom, a sulfur atom, $=NR^{Z31}$, or $=CR^{Z32}R^{Z33}$, $R^{Z31}$ represents a hydrogen atom or a substituent, $R^{Z32}$ and $R^{Z33}$ each independently represent a cyano group or $-COOR^{Z34}$, $R^{Z34}$ represents an alkyl group which may have a substituent, an aryl group which may have a substituent, or a heteroaryl group which may have a substituent,
$R^{31}$ and $R^{32}$ each independently represent a hydrogen atom or a substituent,
$R^{33}$ and $R^{34}$ represent an aryl group which may have a substituent, a heteroaryl group which may have a substituent, an alkyl group which may have a substituent, or a hydrogen atom,
$B^3$ represents an aromatic ring which contains at least two carbon atoms and may have a substituent,
in Formula (4),
$A^4$ represents a ring which contains at least two carbon atoms and may have a substituent,
$Z^4$ represents an oxygen atom, a sulfur atom, $=NR^{Z41}$, or $=CR^{Z42}R^{Z43}$, $R^{Z41}$ represents a hydrogen atom or a substituent, $R^{Z42}$ and $R^{Z43}$ each independently represent a cyano group or $-COOR^{Z44}$, $R^{Z44}$ represents an alkyl group which may have a substituent, an aryl group which may have a substituent, or a heteroaryl group which may have a substituent,
$R^{41}$ and $R^{42}$ each independently represent a hydrogen atom or a substituent,
$R^{43}$ represents an aryl group which may have a substituent, a heteroaryl group which may have a substituent, an alkyl group which may have a substituent, or a hydrogen atom,
$X^{41}$ and $X^{42}$ each independently represent a nitrogen atom or $CR^{X4}$, $R^{X4}$ represents a hydrogen atom or a substituent,
$X^{43}$ each independently represents an oxygen atom, a sulfur atom, or $NR^{X4}$, $R^{X4}$ represents a hydrogen atom or a substituent,
in Formula (5),
$A^5$ represents a ring which contains at least two carbon atoms and may have a substituent,
$Z^5$ represents an oxygen atom, a sulfur atom, $=NR^{Z51}$, or $=CR^{Z52}R^{Z53}$, $R^{Z51}$ represents a hydrogen atom or a

substituent, $R^{Z52}$ and $R^{Z53}$ each independently represent a cyano group or $-COOR^{Z54}$, $R^{Z54}$ represents an alkyl group which may have a substituent, an aryl group which may have a substituent, or a heteroaryl group which may have a substituent,
$R^{51}$ represents a hydrogen atom or a substituent,
$R^{52}$ represents an aryl group which may have a substituent, a heteroaryl group which may have a substituent, an alkyl group which may have a substituent, or a hydrogen atom,
$R^{e1}$ and $R^{e2}$ each independently represent a substituent, $R^{e1}$ and $R^{e2}$ may be bonded to each other to form a ring,
$L^{51}$ represents a carbon atom, a silicon atom, or a germanium atom,
$B^5$ represents an aromatic ring which contains at least two carbon atoms and may have a substituent, and
$C^5$ represents an aromatic ring which contains at least three carbon atoms and may have a substituent.

**5.** The photoelectric conversion element according to any one of claims 1 to 4, wherein in Formula (1), B represents an aromatic ring-containing methine coloring agent residue or a pyrromethene coloring agent residue.

**6.** The photoelectric conversion element according to any one of claims 1 to 5,

wherein in Formula (1), B represents a residue derived from a compound represented by Formula (6), which is formed by removing one hydrogen atom bonded to ring-constituting atoms forming a ring structure in the compound represented by Formula (6),

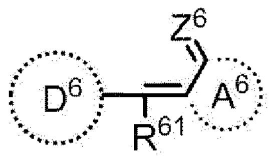

(6)

in Formula (6),
$A^6$ represents a ring which contains at least two carbon atoms and may have a substituent,
$Z^6$ represents an oxygen atom, a sulfur atom, $=NR^{Z61}$, or $=CR^{Z62}R^{Z63}$, $R^{Z61}$ represents a hydrogen atom or a substituent, $R^{Z62}$ and $R^{Z61}$ each independently represent a cyano group or $-COOR^{Z64}$, $R^{Z64}$ represents an alkyl group which may have a substituent, an aryl group which may have a substituent, or a heteroaryl group which may have a substituent,
$D^6$ represents a heteroaryl group which may have a substituent, and
$R^{61}$ represents a hydrogen atom or a substituent.

**7.** The photoelectric conversion element according to any one of claims 1 to 6, wherein the photoelectric conversion film further includes a n-type organic semiconductor, and has a bulk hetero structure formed in a state where the compound represented by Formula (1) and the n-type organic semiconductor are mixed with each other.

**8.** The photoelectric conversion element according to any one of claims 1 to 7, wherein the photoelectric conversion film further includes a p-type organic semiconductor.

**9.** The photoelectric conversion element according to any one of claims 1 to 8, further comprising one or more interlayers between the conductive film and the transparent conductive film, in addition to the photoelectric conversion film.

**10.** An imaging element comprising the photoelectric conversion element according to any one of claims 1 to 9.

**11.** An optical sensor comprising the photoelectric conversion element according to any one of claims 1 to 9.

**12.** A compound represented by Formula (1),

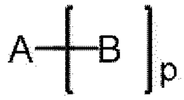

(1)

in Formula (1),

A has $\Delta E_A$ of 2.00 to 3.20 eV and represents a first aromatic ring-containing coloring agent residue having a first monocyclic structure directly bonded to B,
B has $\Delta E_B$ of 2.80 to 4.00 eV and represents a second aromatic ring-containing coloring agent residue having a second monocyclic structure directly bonded to A,
p represents 1 or 2,
where, in Formula (1), a dihedral angle $\alpha$ formed of the first monocyclic structure and the second monocyclic structure defined below is 30° to 90°, the $\Delta E_A$ and the $\Delta E_B$ satisfy a relationship of $\Delta E_A < \Delta E_B$,
$\Delta E_A$: in a case where a structure optimization calculation is performed on a compound UA obtained by replacing B with a hydrogen atom in the compound represented by Formula (1), by density functional calculation B3LYP/6-31G (d) with quantum chemical calculation software Gaussian09 to obtain HOMO energy and LUMO energy, the obtained HOMO energy being represented by $E_{HOMO-A}$ (eV) and the obtained LUMO energy being represented by $E_{LUMO-A}$ (eV), $\Delta E_A$ represents a numeric value (eV) obtained by Formula (EA),

$$\text{Formula (EA): } \Delta E_A = E_{LUMO-A} - E_{HOMO-A}$$

$\Delta E_B$: in a case where a structure optimization calculation is performed on a compound UB obtained by replacing A with a hydrogen atom in the compound represented by Formula (1), by density functional calculation B3LYP/6-31G (d) with quantum chemical calculation software Gaussian09 (manufactured by Gaussian, Inc.) to obtain HOMO energy and LUMO energy, the obtained HOMO energy being represented by $E_{HOMO-B}$ (eV) and the obtained LUMO energy being represented by $E_{LUMO-B}$ (eV), $\Delta E_B$ represents a numeric value (eV) obtained by Formula (EB),

$$\text{Formula (EB): } \Delta E_B = E_{LUMO-B} - E_{HOMO-B}$$

dihedral angle a: in a structure obtained by performing a structure optimization calculation on the compound represented by Formula (1), by density functional calculation B3LYP/6-31G (d) with quantum chemical calculation software Gaussian09, the dihedral angle $\alpha$ is a dihedral angle having a smallest absolute value among four kinds of dihedral angles (X1) to (X4), which are able to be formed with four adjacent atoms represented by a to f in a partial structure corresponding to Formula (1-A) in Formula (1), and

(X1) a-b-e-d

(X2) a-b-e-f

(X3) c-b-e-d

(X4) c-b-e-f

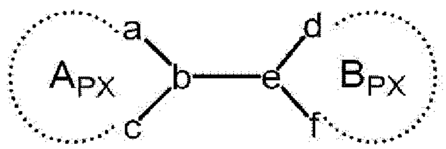

(1-A)

in Formula (1-A),
$A_{PX}$ corresponds to the first monocyclic structure contained in the first aromatic ring-containing coloring agent residue in Formula (1), and at least contains three atoms represented by a, b, and c as ring-constituting atoms,
$B_{PX}$ corresponds to the second monocyclic structure contained in the second aromatic ring-containing coloring agent residue in Formula (1), and at least contains three atoms represented by d, e, and f as ring-constituting atoms, and
in Formula (1-A), all of bonds represented by a-b, c-b, d-e, e-f, and b-e are covalent bonds.

**13.** The compound according to claim 12,

wherein in Formula (1), A represents an aromatic ring-containing methine coloring agent residue.

**14.** The compound according to claim 12 or 13,
wherein in Formula (1), an atom at a bonding position with B of the first monocyclic structure is a nitrogen atom.

**15.** The compound according to any one of claims 12 to 14,

wherein in Formula (1), A represents a residue derived from a compound represented by Formula (3), which is formed by removing p hydrogen atom bonded to ring-constituting atoms forming a ring structure in the compound represented by Formula (3), a residue derived from a compound represented by Formula (4), which is formed by removing p hydrogen atom bonded to ring-constituting atoms forming a ring structure in the compound represented by Formula (4), or a residue derived from a compound represented by Formula (5), which is formed by removing p hydrogen atom bonded to ring-constituting atoms forming a ring structure in the compound represented by Formula (5),

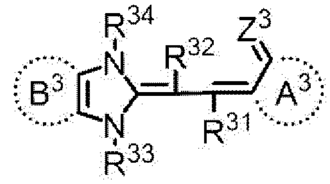

(3)

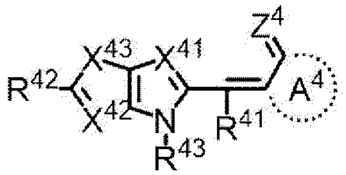

(4)

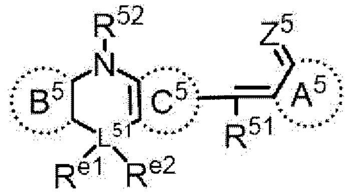

(5)

in Formula (3),
$A^3$ represents a ring which contains at least two carbon atoms and may have a substituent,
$Z^3$ represents an oxygen atom, a sulfur atom, $=NR^{Z31}$, or $=CR^{Z32}R^{Z33}$, $R^{Z31}$ represents a hydrogen atom or a substituent, $R^{Z32}$ and $R^{Z33}$ each independently represent a cyano group or $-COOR^{Z34}$, $R^{Z34}$ represents an alkyl group which may have a substituent, an aryl group which may have a substituent, or a heteroaryl group which may have a substituent,
$R^{31}$ and $R^{32}$ each independently represent a hydrogen atom or a substituent,
$R^{33}$ and $R^{34}$ represent an aryl group which may have a substituent, a heteroaryl group which may have a substituent, an alkyl group which may have a substituent, or a hydrogen atom,
$B^3$ represents an aromatic ring which contains at least two carbon atoms and may have a substituent,
in Formula (4),
$A^4$ represents a ring which contains at least two carbon atoms and may have a substituent,
$Z^4$ represents an oxygen atom, a sulfur atom, $=NR^{Z41}$, or $=CR^{Z42}R^{Z43}$, $R^{Z41}$ represents a hydrogen atom or a substituent, $R^{Z42}$ and $R^{Z43}$ each independently represent a cyano group or $-COOR^{Z44}$, $R^{Z44}$ represents an alkyl group which may have a substituent, an aryl group which may have a substituent, or a heteroaryl group which may have a substituent,
$R^{41}$ and $R^{42}$ each independently represent a hydrogen atom or a substituent,
$R^{43}$ represents an aryl group which may have a substituent, a heteroaryl group which may have a substituent, an alkyl group which may have a substituent, or a hydrogen atom,
$X^{41}$ and $X^{42}$ each independently represent a nitrogen atom or $CR^{X4}$, $R^{X4}$ represents a hydrogen atom or a substituent,

$X^{43}$ each independently represents an oxygen atom, a sulfur atom, or $NR^{X4}$, $R^{X4}$ represents a hydrogen atom or a substituent,
in Formula (5),
$A^5$ represents a ring which contains at least two carbon atoms and may have a substituent,
$Z^5$ represents an oxygen atom, a sulfur atom, $=NR^{Z51}$, or $=CR^{Z52}R^{Z53}$, $R^{Z51}$ represents a hydrogen atom or a substituent, $R^{Z52}$ and $R^{Z53}$ each independently represent a cyano group or $-COOR^{Z54}$, $R^{Z54}$ represents an alkyl group which may have a substituent, an aryl group which may have a substituent, or a heteroaryl group which may have a substituent,
$R^{51}$ represents a hydrogen atom or a substituent,
$R^{52}$ represents an aryl group which may have a substituent, a heteroaryl group which may have a substituent, an alkyl group which may have a substituent, or a hydrogen atom,
$R^{e1}$ and $R^{e2}$ each independently represent a substituent, $R^{e1}$ and $R^{e2}$ may be bonded to each other to form a ring,
$L^{51}$ represents a carbon atom, a silicon atom, or a germanium atom,
$B^5$ represents an aromatic ring which contains at least two carbon atoms and may have a substituent, and
$C^5$ represents an aromatic ring which contains at least three carbon atoms and may have a substituent.

**16.** The compound according to any one of claims 12 to 15,
wherein in Formula (1), B represents an aromatic ring-containing methine coloring agent residue or a pyrromethene coloring agent residue.

**17.** The compound according to any one of claims 12 to 16,

wherein in Formula (1), B represents a residue derived from a compound represented by Formula (6), which is formed by removing one hydrogen atom bonded to ring-constituting atoms forming a ring structure in the compound represented by Formula (6),

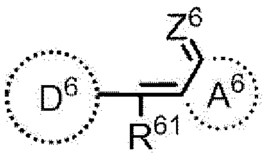

(6)

in Formula (6),
$A^6$ represents a ring which contains at least two carbon atoms and may have a substituent,
$Z^6$ represents an oxygen atom, a sulfur atom, $=NR^{Z61}$, or $=CR^{Z62}R^{Z63}$, $R^{Z61}$ represents a hydrogen atom or a substituent, $R^{Z62}$ and $R^{Z63}$ each independently represent a cyano group or $-COOR^{Z64}$, $R^{Z64}$ represents an alkyl group which may have a substituent, an aryl group which may have a substituent, or a heteroaryl group which may have a substituent,
$D^6$ represents a heteroaryl group which may have a substituent, and
$R^{61}$ represents a hydrogen atom or a substituent.

FIG. 1

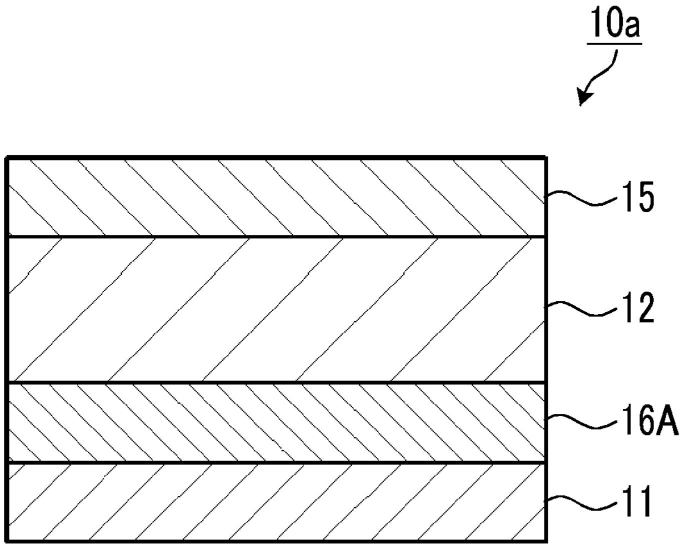


FIG. 2

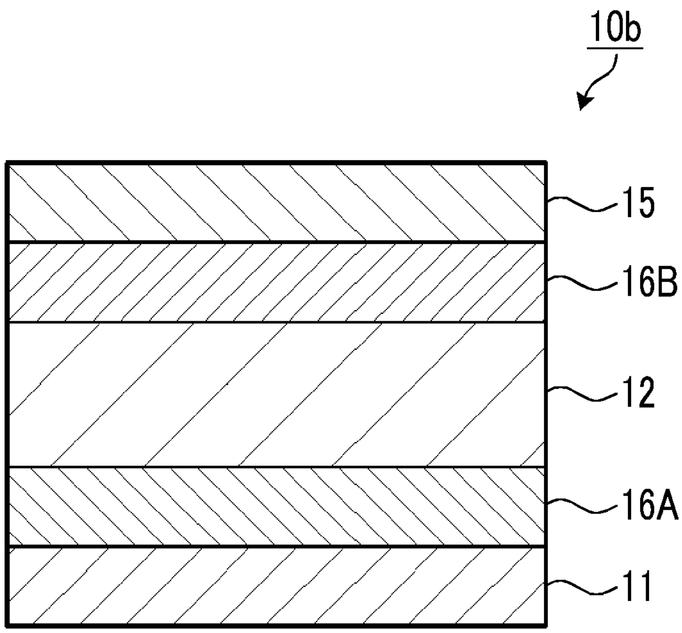

## INTERNATIONAL SEARCH REPORT

International application No.
**PCT/JP2021/027028**

### A. CLASSIFICATION OF SUBJECT MATTER

***C07D 401/04***(2006.01)i; ***C07D 401/10***(2006.01)i; ***C07D 417/04***(2006.01)i; ***C07D 491/048***(2006.01)i; ***C07D 513/04***(2006.01)i; ***C07F 5/02***(2006.01)i; ***H01L 51/42***(2006.01)i; ***C07D 209/12***(2006.01)i; ***C07D 487/04***(2006.01)i
FI: H01L31/08 T; C07D487/04 144; C07D513/04 325; C07D401/04; C07D401/10; C07D209/12; C07D417/04; C07F5/02 D; C07D491/048 CSP

According to International Patent Classification (IPC) or to both national classification and IPC

### B. FIELDS SEARCHED

Minimum documentation searched (classification system followed by classification symbols)

H01L51/42-51/48

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2018/016570 A1 (SONY CORP.) 25 January 2018 (2018-01-25) paragraphs [0032], [0039], [0045], fig. 1 | 1, 5, 7-11 |
| A | | 2-4, 6, 13-15, 17 |
| Y | ZHANG, Xian-Fu, "BisBODIPY as PCT-based halogen free photosensitizers for highly efficient excited triplet state and singlet oxygen formation: Tuning the efficiency by different linking positions", Dyes and Pigments, 2017, vol. 146, pp. 491-501 In particular, abstract; fig. 1-3 | 1, 5, 7-11 |
| X | | 12, 16 |
| A | | 2-4, 6, 13-15, 17 |
| Y | GOMEZ-INFANTE, A. J. et al., "Synthesis, Properties, and Functionalization of Nonsymmetric 8-MethylthioBODIPYs", European Journal of Organic Chemistry, 2016, vol. 2016, pp. 5009-5023 in particular, fig. 1 | 1, 5, 7-11 |
| A | | 2-4, 6, 13-15, 17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 October 2021** | **12 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/027028**

**C. DOCUMENTS CONSIDERED TO BE RELEVANT**

| Category* | Citation of document, with indication, where appropriate, of the relevant passages | Relevant to claim No. |
|---|---|---|
| Y<br><br><br>A | MUKHERJEE, S. et al., "Effect of alkyl substituents in BODIPYs: a comparative DFT computational investigation", RSC Advances, 2015, vol. 5, pp. 2706-2714<br>in particular, fig. 3, 5 | 1, 5, 7-11<br><br><br>2-4, 6, 13-15, 17 |
| Y<br><br><br>A | JP 2015-530729 A (THE REGENTS OF THE UNIVERSITY OF MICHIGAN) 15 October 2015 (2015-10-15)<br>paragraph [0112], fig. 6 | 1, 5, 7-11<br><br><br>2-4, 6, 13-15, 17 |
| A | WO 2020/013246 A1 (FUJIFILM CORPORATION) 16 January 2020 (2020-01-16) | 1-17 |
| A | WO 2019/009249 A1 (FUJIFILM CORPORATION) 10 January 2019 (2019-01-10) | 1-17 |
| A | WO 2019/054327 A1 (FUJIFILM CORPORATION) 21 March 2019 (2019-03-21) | 1-17 |
| A | WO 2019/189134 A1 (FUJIFILM CORPORATION) 03 October 2019 (2019-10-03) | 1-17 |

Form PCT/ISA/210 (second sheet) (January 2015)

**INTERNATIONAL SEARCH REPORT**
**Information on patent family members**

International application No.
**PCT/JP2021/027028**

| Patent document cited in search report | Publication date (day/month/year) | Patent family member(s) | Publication date (day/month/year) |
|---|---|---|---|
| WO 2018/016570 A1 | 25 January 2018 | US 2019/0319071 A1<br>paragraphs [0078], [0085], [0091], fig. 1<br>EP 3490003 A1<br>CN 109417085 A<br>KR 10-2019-0028661 A | |
| JP 2015-530729 A | 15 October 2015 | WO 2014/025435 A2<br>paragraph [0131], fig. 16<br>CA 2873468 A1<br>KR 10-2015-0020297 A<br>CN 105409020 A | |
| WO 2020/013246 A1 | 16 January 2020 | US 2021/0135128 A1 | |
| WO 2019/009249 A1 | 10 January 2019 | US 2020/0135951 A1<br>EP 3651222 A1<br>KR 10-2020-0010470 A | |
| WO 2019/054327 A1 | 21 March 2019 | US 2020/0212107 A1<br>KR 10-2020-0031685 A | |
| WO 2019/189134 A1 | 03 October 2019 | US 2020/0411569 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009167348 A
- JP 2004211096 A
- JP 2007234651 A
- JP 2006100767 A
- JP 2007123707 A
- JP 2014082483 A
- JP 2012077064 A
- WO 2018105269 A
- WO 2018186389 A
- WO 2018186397 A
- WO 2019009249 A
- WO 2019049946 A
- WO 2019054327 A
- WO 2019098161 A
- JP 2005303266 A
- JP 2011228614 A
- JP 2011176259 A
- JP 2011225544 A
- JP 2015153910 A
- JP 2012094660 A
- JP 2018014474 A
- WO 2016194630 A
- WO 2017159684 A
- JP 2017076766 A
- WO 2018207722 A
- JP 2019054228 A
- WO 2019058995 A
- WO 2019081416 A
- JP 2019080052 A
- WO 2019054125 A
- WO 2019093188 A
- JP 2011082508 A
- JP 2019208026 A
- JP 2018125850 A
- JP 2018125848 A